# EUROPEAN PATENT APPLICATION

(11) **EP 1 426 056 A1**
(43) Date of publication of application: **09.06.2004**
(21) Application number: 02798045.7
(22) Date of filing: 09.09.2002
(51) Int. Cl.: A61K 38/17, A61K 38/43, A61K 45/00, A61P 19/02, A61P 19/10, C12Q 1/68, G01N 33/15, G01N 33/50

(54) **PREVENTIVES & REMEDIES FOR BONE & JOINT DISEASES**

(30) Priority: 10.09.2001 JP 2001273914; 17.09.2001 JP 2001281472; 28.09.2001 JP 2001300417; 28.09.2001 JP 2001300289; 28.09.2001 JP 2001300347; 28.09.2001 JP 2001303390
(71) Applicant: Takeda Chemical Industries, Ltd., Osaka-shi, Osaka 541-0045 (JP)
(72) Inventor: HIKICHI, Yuichi, Shareru Tsukuba Matsushiro 1-504, Tsukuba-shi, Ibaraki 305-0035 (JP); INAZUKA, Masakazu, Takeda Matsushiro Residence 408, Tsukuba-shi, Ibaraki 305-0035 (JP); YOSHIMURA, Koji, Takeda Kasuga Haitsu 404, Tsukuba-shi, Ibaraki 305-0821 (JP)
(74) Representative: Lewin, John Harvey
(86) International application number: PCT/JP2002/009140
(87) International publication number: WO 2003/022300

(57) **Abstract**

The present invention provides compounds or salts thereof that regulate the activity of a protein comprising an amino acid sequence identical or substantially identical to the amino acid sequence represented by SEQ ID NO: 1, SEQ ID NO: 3, SEQ ID NO: 5, SEQ ID NO: 7, SEQ ID NO: 9, SEQ ID NO: 11 or SEQ ID NO: 13, and antisense polynucleotides comprising a nucleotide sequence, or a part thereof, complementary or substantially complementary to the nucleotide sequence of a DNA encoding a protein, or a partial peptide thereof, comprising an amino acid sequence identical or substantially identical to the amino acid sequence represented by SEQ ID NO: 1, SEQ ID NO: 3, SEQ ID NO: 5, SEQ ID NO: 7, SEQ ID NO: 9, SEQ ID NO: 11 or SEQ ID NO: 13. These compounds, salts and antisense poly nucleotides may be used as prophylactic and/or therapeutic agents for bone and/or arthropathy diseases such as arthritis.

## Description

### TECHNICAL FIELD

The present invention relates to prophylactic and/or therapeutic agents, diagnostic agents and the like for bone and/or arthropathy diseases.

### BACKGROUND ART

An arthro is the part where two bones adjacently move, and the bones in this part are covered with articular cartilage to allow them to move smoothly. Besides, synovial fluid produced in the synovial membrane can lightly move as a lubricant by working together with arthrodial cartilage in an arthro. Osteoarthosis is one of arthropathy diseases accompanied by chronic arthritis; this disease is caused by the generative of the cartilage and results in the breakdown of the cartilage and proliferative changes in the bone and cartilage. Deformation of articulation, such as osteophyte formation at the edges of bones, is also observed. It is told that osteoarthosis patients gradually increase with age and there are more than 80% of people of age 60 or older develop the osteoarthosis in knee, elbow, hip and the spine. The treatment of osteoarthosis is mainly symptomatic treatment to relieve pain and used non-steroidal, anti-inflammatory drugs or hyaluronic acid or steroids by directly injecting into articulations. If osteoarthosis is progressed, arthroscopic surgery is performed; if pain or deformation is severe, osteotomy or replacement with artificial arthropathys is applied to knee or hip articulations. However, since artificial articulations can last only for a certain period, it is considered desirable that patients under 55 should avoid this replacement surgery. Under circumstances, development of a treatment method that can inhibit degenerative changes in articulations is desired. Further, at present, osteoarthosis can only be diagnosed by using apparatuses such as for taking X-ray photos, and thus development of a more simple diagnosis method is also desired.

In order to analyze gene expression comprehensively, the microarray technique using immobilized cDNA or oligonucleotides has been developed. Technologies to find out changes in the expression of disease-specific genes have been spread broadly and their utility has been confirmed. For example, the GeneChip system of Affimetrix has been used increasingly in the diagnosis of diseases such as cancer and in the finding of target genes for drug development.

Attempts have been made to find out genes or proteins that are expressed specifically in arthroculatio of osteoarthosis patients and to create therapeutic or diagnostic agents for osteoarthosis from the resultant genes or proteins. For example, it is known that the expression of matrix metalloproteinase (MMP) such as MMP-13 is increased in arthroculatio in osteoarthosis; MMP inhibitor is now examined as an arthroculatio breakdown inhibitor.

Type II iodothyronine deiodinase (DIO2) is an enzyme that converts thyroxin (hereinafter, optionally abbreviated to "T4") to triiodothyronine (hereinafter, optionally abbreviated to "T3"). It is known that the expression of this enzyme is high in the thymus which is an organ producing thyroid and triiodothyronine (J. Clin. Invest. 98: 962-968, 1996). On the other hand, it is also reported that triiodothyronine is involved in cartilage differentiation and skeletal formation (J. Bone Miner. Res., 11: 105-113, 1956; Endocrinology 141: 3887-3897, 2000). It is also known that DIO2 can be produced from a recombinant DNA in which a DIO2 selenocysteine insert is linked downstream of the amino acid coding region for DIO2 (J. Biol. Chem. 273: 33374-33378, 1998). It is also reported that addition of T3 to a culture system of arthrodial cartilage cells induces late differentiation (hypertrophy) (J. Rheumatolo., 26: 395-401, 1999) and that arthrodial cartilage is terminally differentiated in human osteoarthosis, which is the cause of the breakdown of cartilage in osteoarthosis (Osteoarthosis Cartilage 8: 294-302, 2000). However, relations between substances such as DIO2, thyroxin and triiodothyronine, and arthropathy diseases such as osteoarthosis have not been elucidated yet.

*ANKH* has been identified as a human homologous gene to *ank* which is a causative gene for mouse progressive synarthrophysis (Science 289: 265-270, 2000), and eventually it was found that this gene is a causative gene for human parietal dysplasia (Nature Genetics 28: 37-41, 2001). Based on researches into the amino acid sequence and mutated cells of ANKH, it has been assumed that ANKH is a pyrophosphate transporter. Since pyrophosphate has calcification-inhibiting effect, it is considered that ANKH regulates the calcification of tissues such as arthropathys by regulating the concentration of pyrophosphate (Science 289: 265-270, 2000; Nature genetics 28: 37-41, 2001). However, there has been no report concerning relation between ANKH and osteoarthosis.

*SHOX2* (short stature homeobox 2) is a gene belonging to the homeobox gene family and encoding a transcriptional control factor. This gene is believed to be involved in morphogenesis. *SHOX2* has two transcripts designated *SHOX2a* (short stature homeobox 2; isoform a) and *SHOX2b* (short stature homeobox 2; isoform b), respectively, due to alternative splicing. *SHXO2a* and *SHOX2b* have a homeodomain and a 14-amino acid motif that is characteristic for a homeodomain protein expressed in the skull in common. In view of the location of *SHOX2* in the chromosome and the expression pattern of its mouse homologous gene at developmental stages, it is considered that *SHOX2* is a candidate for the causative gene for Cornelia de Lang syndrome which presents symptoms such as malformation in the skull, limbs, etc. and delay in bone maturation (Proc. Natl. Acad. Sci.USA 95: 2406-2411, 1998). However, there has been no report on relation between *SHOX2* and bone and/or arthropathy diseases including osteoarthosis. Homeobox genes encode transcription factors which bind to a specific DNA sequence to thereby control the expression of a target gene. Further, it is considered that homeobox genes are involved in the differentiation and proliferation of cells. For example, it is known that *Hoxc-8* (one of homeobox genes) regulates the differentiation of cartilage cells (Y.G. Yueh et al., Proc. Natl. Acad. Sci. USA 95: 9956-61, 1998) and the differentiation of osteoblast cells (X. Yang et al., J. Biol. Chem. 275:1065-72,2000).

*TASK-4* (optionally called *"TALk-2")* gene has been already cloned. It is reported that this gene belongs to the potassium ion channel family and is expressed in the pancreas, etc. (FEBS Lett., 492(1-2): 84-89, 2001; Biochem. Biophys. Res. Commun., 282(1): 249-256, 2001). On the other hand, it has been suggested that ion channels are involved in the proliferation of cartilage cells (J. Orthop. Sci. 6(2): 15-159, 2001). However, there has been no report concerning relation between these genes and osteoarthosis. It is unknown which ion channel is involved in the proliferation of cartilage cells, and there has been no report on their relation with osteoarthosis.

It is reported that *EphA3* gene belongs to the receptor type tyrosine kinase family and is expressed in embryonic brain, etc. (Proc. Natl. Acad. Sci. USA 89(5): 1611-1615, 1992). The Eph (erythropoietin-producing human hepatocellular carcinoma cell line) receptor gene family is currently composed of 14 members (Cell 90: 403-404, 1997). As Eph receptors, there have been reported EphA1 (Science 238: 1717-1720, 1987), EphA2 (Mol. Cell. Biol., 10: 6316-6324, 1990), EphA3 (Oncogene, 10: 897-905, 1995), EphA4 (Oncogene, 10: 897-905, 1995), EphA5 (Oncogene, 10: 897-905, 1995), EphA6 (Oncogene, 8: 3277-3288, 1993), EphA7 (Oncogene, 10: 897-905, 1995), EphA8 (Oncogene, 6: 1057-1061, 1991), EphB1 (Cell, 90: 403-404, 1997), EphB2 (Cell, 90: 403-404, 1997), EphB3 (Cell, 90: 403-404, 1997), EphB4 (J. Biol. Chem., 269: 14211-14218, 1994), EphB5 (J. Cell. Biol., 135: 781-795, 1996), EphB6 (Oncogene, 13: 777-786, 1996) and the like. However, there has been no report concerning relation between the Eph family including *EphA3* and osteoarthosis.

Although it is reported that MMP-16 (matrix metalloproteinase 16) (MT3-MMP) is expressed in the synovial membrane, etc. of rheumatoid arthritis patients (Arthritis Rheum., 43: 1226-1232, 2000; Lab. Invest., 80: 677-687, 2000) and is associated with cancer (WO 97/04080), its involvement in the breakdown of cartilage has not been indicated directly.

### DISCLOSURE OF THE INVENTION

It is demanded that safe and excellent prophylactic and/or therapeutic agents for bone and/or arthropathy diseases.

As a result of extensive and intensive researches toward the solution of the above problem, the present inventors have found a gene whose expression is remarkably increased in cartilage in osteoarthosis and made further researches based on this finding. Thus, the present invention has been achieved.

The present invention provides:
(1) A prophylactic and/or therapeutic agent for bone and/or arthropathy diseases, comprising a compound or a salt thereof that regulates the activity of a protein comprising an amino acid sequence identical or substantially identical to the amino acid sequence represented by SEQ ID NO: 1, SEQ ID NO: 3, SEQ ID NO: 5, SEQ ID NO: 7, SEQ ID NO: 9, SEQ ID NO: 11 or SEQ ID NO: 13, a partial peptide of the protein, or a salt of the protein or the partial peptide;
(2) The prophylactic and/or therapeutic agent of (1) above, comprising a compound or a salt thereof that inhibits the activity of a protein comprising an amino acid sequence identical or substantially identical to the amino acid sequence represented by SEQ ID NO: 1, SEQ ID NO: 3, SEQ ID NO: 5, SEQ ID NO: 7, SEQ ID NO: 9, SEQ ID NO: 11 or SEQ ID NO: 13, a partial peptide of the protein, or a salt of the protein or the partial peptide;
(3) The prophylactic and/or therapeutic agent of (2) above, which is a compound or a salt thereof that inhibits the activity of a protein consisting of the amino acid sequence represented by SEQ ID NO: 1, a partial peptide of the protein, or a salt of the protein or the partial peptide;
(4) A prophylactic and/or therapeutic agent for bone and/or arthropathy diseases, comprising a compound or a salt thereof that regulates the expression of the gene of a protein comprising an amino acid sequence identical or substantially identical to the amino acid sequence represented by SEQ ID NO: 1, SEQ ID NO: 3, SEQ ID NO: 5, SEQ ID NO: 7, SEQ ID NO: 9, SEQ ID NO: 11 or SEQ ID NO: 13, a partial peptide of the protein, or a salt of the protein or the partial peptide;
(5) A prophylactic and/or therapeutic agent for bone and/or arthropathy diseases, comprising a compound or a salt thereof that inhibits the expression of the gene of a protein comprising an amino acid sequence identical or substantially identical to the amino acid sequence represented by SEQ ID NO: 1, SEQ ID NO: 3, SEQ ID NO: 5, SEQ ID NO: 7, SEQ ID NO: 9, SEQ ID NO: 11 or SEQ ID NO: 13, a partial peptide of the protein, or a salt of the protein or the partial peptide;
(6) The prophylactic and/or therapeutic agent of (5) above, which is a compound or a salt thereof that regulates the expression of the gene of a protein consisting of the amino acid sequence represented by SEQ ID NO: 1, a partial peptide of the protein, or a salt of the protein or the partial peptide;
(7) A prophylactic and/or therapeutic agent for bone and/or arthropathy diseases, comprising an antisense polynucleotide comprising a nucleotide sequence, or a part thereof, complementary or substantially complementary to the nucleotide sequence of a DNA encoding a protein, or a partial peptide thereof, comprising an amino acid sequence identical or substantially identical to the amino acid sequence represented by SEQ ID NO: 1, SEQ ID NO: 3, SEQ ID NO: 5, SEQ ID NO: 7, SEQ ID NO: 9, SEQ ID NO: 11 or SEQ ID NO: 13;
(8) A prophylactic and/or therapeutic agent for bone and/or arthropathy diseases, comprising an antibody against a protein comprising an amino acid sequence identical or substantially identical to the amino acid sequence represented by SEQ ID NO: 1, SEQ ID NO: 3, SEQ ID NO: 5, SEQ ID NO: 7, SEQ ID NO: 9, SEQ ID NO: 11 or SEQ ID NO: 13, a partial peptide of the protein, or a salt of the protein or the partial peptide;
(9) The prophylactic and/or therapeutic agent of (1), (4), (7) or (8) above, wherein the bone and/or arthropathy disease is chondrodystrophy, osteodystrophy, osteoporosis, osteoarthosis, rheumatoid arthritis, arthritis, synovitis, metabolic arthropathy or arthropathy disorder caused by sports;
(10) A diagnostic agent for bone and/or arthropathy diseases, comprising an antibody against a protein comprising an amino acid sequence identical or substantially identical to the amino acid sequence represented by SEQ ID NO: 1, SEQ ID NO: 3, SEQ ID NO: 5, SEQ ID NO: 7, SEQ ID NO: 9, SEQ ID NO: 11 or SEQ ID NO: 13, a partial peptide of the protein, or a salt of the protein or the partial peptide;
(11) A diagnostic agent for bone and/or arthropathy diseases, comprising a DNA encoding a protein, or a partial peptide thereof, comprising an amino acid sequence identical or substantially identical to the amino acid sequence represented by SEQ ID NO: 1, SEQ ID NO: 3, SEQ ID NO: 5, SEQ ID NO: 7, SEQ ID NO: 9, SEQ ID NO: 11 or SEQ ID NO: 13;
(12) The diagnostic agent of (10) or (11) above, wherein the bone and/or arthropathy disease is chondrodystrophy, osteodystrophy, osteoporosis, osteoarthosis, rheumatoid arthritis, arthritis, synovitis, metabolic arthropathy or arthropathy disorder caused by sports;
(13) A method of screening for a prophylactic and/or therapeutic agent for bone and/or arthropathy diseases, comprising using a protein comprising an amino acid sequence identical or substantially identical to the amino acid sequence represented by SEQ ID NO: 1, SEQ ID NO: 3, SEQ ID NO: 5, SEQ ID NO: 7, SEQ ID NO: 9, SEQ ID NO: 11 or SEQ ID NO: 13, a partial peptide of the protein, or a salt of the protein or the partial peptide;
(14) The screening method of (13) above, wherein a protein consisting of the amino acid sequence represented by SEQ ID NO: 1, a partial peptide of the protein, or a salt of the protein or the partial peptide is used;
(15) A kit for screening for a prophylactic and/or therapeutic agent for bone and/or arthropathy diseases, containing a protein comprising an amino acid sequence identical or substantially identical to the amino acid sequence represented by SEQ ID NO: 1, SEQ ID NO: 3, SEQ ID NO: 5, SEQ ID NO: 7, SEQ ID NO: 9, SEQ ID NO: 11 or SEQ ID NO: 13, a partial peptide of the protein, or a salt of the protein or the partial peptide;
(16) A prophylactic and/or therapeutic agent for bone and/or arthropathy diseases, obtainable by using the screening method of (13) above or the screening kit of (15) above;
(17) A method of screening for a prophylactic and/or therapeutic agent for bone and/or arthropathy diseases, comprising using a DNA encoding a protein, or a partial peptide thereof, comprising an amino acid sequence identical or substantially identical to the amino acid sequence represented by SEQ ID NO: 1, SEQ ID NO: 3, SEQ ID NO: 5, SEQ ID NO: 7, SEQ ID NO: 9, SEQ ID NO: 11 or SEQ ID NO: 13;
(18) The screening method of (17) above, wherein a DNA encoding a protein, or a partial peptide thereof, consisting of the amino acid sequence represented by SEQ ID NO: 1 is used;
(19) A kit for screening for a prophylactic and/or therapeutic agent for bone and/or arthropathy diseases, containing a DNA encoding a protein, or a partial peptide thereof, comprising an amino acid sequence identical or substantially identical to the amino acid sequence represented by SEQ ID NO: 1, SEQ ID NO: 3, SEQ ID NO: 5, SEQ ID NO: 7, SEQ ID NO: 9, SEQ ID NO: 11 or SEQ ID NO: 13;
(20) A prophylactic and/or therapeutic agent for bone and/or arthropathy diseases, obtainable by using the screening method of (17) above or the screening kit of (19) above;
(21) A method of preventing and/or treating bone and/or arthropathy diseases, comprising administering to a mammal an effective amount of a compound or a salt thereof that regulates the activity of a protein comprising an amino acid sequence identical or substantially identical to the amino acid sequence represented by SEQ ID NO: 1, SEQ ID NO: 3, SEQ ID NO: 5, SEQ ID NO: 7, SEQ ID NO: 9, SEQ ID NO: 11 or SEQ ID NO: 13, a partial peptide of the protein, or a salt of the protein or the partial peptide;
(22) A method of preventing and/or treating bone and/or arthropathy diseases, comprising administering to a mammal an effective amount of a compound or a salt thereof that regulates the expression of the gene of a protein comprising an amino acid sequence identical or substantially identical to the amino acid sequence represented by SEQ ID NO: 1, SEQ ID NO: 3, SEQ ID NO: 5, SEQ ID NO: 7, SEQ ID NO: 9, SEQ ID NO: 11 or SEQ ID NO: 13, a partial peptide of the protein, or a salt of the protein or the partial peptide;
(23) Use of a compound or a salt thereof that regulates the activity of a protein comprising an amino acid sequence identical or substantially identical to the amino acid sequence represented by SEQ ID NO: 1, SEQ ID NO: 3, SEQ ID NO: 5, SEQ ID NO: 7, SEQ ID NO: 9, SEQ ID NO: 11 or SEQ ID NO: 13, a partial peptide of the protein, or a salt of the protein or the partial peptide, for manufacturing a prophylactic and/or therapeutic agent for bone and/or arthropathy diseases;
(24) Use of a compound or a salt thereof that regulates the expression of the gene of a protein comprising an amino acid sequence identical or substantially identical to the amino acid sequence represented by SEQ ID NO: 1, SEQ ID NO: 3, SEQ ID NO: 5, SEQ ID NO: 7, SEQ ID NO: 9, SEQ ID NO: 11 or SEQ ID NO: 13, a partial peptide of the protein, or a salt of the protein or the partial peptide, for manufacturing a prophylactic, and/or therapeutic agent for bone and/or arthropathy diseases;
(25) A prophylactic and/or therapeutic agent for bone and/or arthropathy diseases, comprising a compound or a salt thereof that has a regulating effect on thyroxin 5'-deiodinase activity, pyrophosphate transport activity, potassium ion transport activity, or the signal transduction activity or ligand-binding activity of Eph receptor, and
(26) A prophylactic and/or therapeutic agent for bone and/or arthropathy diseases, comprising a compound or a salt thereof that has a regulating effect on the expression of thyroxin 5'-deiodinase, pyrophosphate transporter, potassium ion transporter or Eph receptor.

### BRIEF DESCRIPTION OF THE DRAWINGS

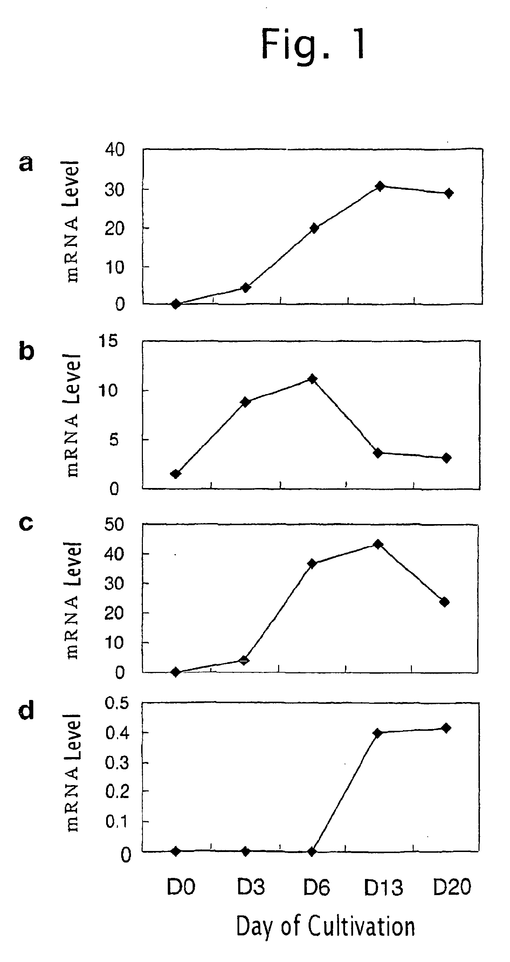

Fig. 1 shows the time course of changes in the expression of DIO2, SOX9, type II collagen and osteocalcin. Fig. 1a shows the time course of the expression of DIO2 mRNA; Fig. 1b shows the time course of the expression of SOX9 mRNA; Fig. 1c shows the time course of the expression of type II collagen; and Fig. 1d shows the time course of the expression of osteocalcin mRNA.

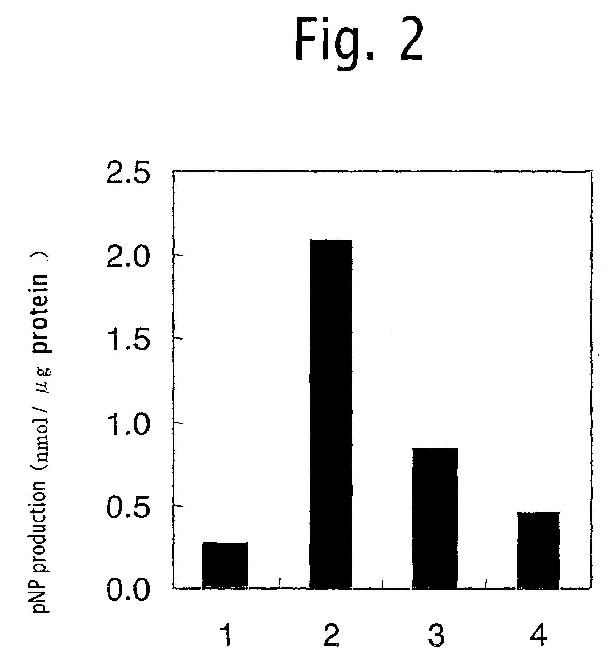

Fig. 2 shows the results of induction of alkaline phosphatase activity in normal human knee arthrodial cartilage cells by T3. In this Figure, No. 1 in the horizontal axis shows the result when the culture broth was composed of DMEM, ITS, BSA, ascorbic acid phosphate magnesium salt n-hydrate and β -glycerophosphate. No. 2 shows the result when the culture broth was composed of DMEM, ITS, BSA, ascorbic acid phosphate magnesium salt n-hydrate, β-glycerophosphate and 50 nM T3. No. 3 shows the result when the culture broth was composed of DMEM, ITS, BSA, ascorbic acid phosphate magnesium salt n-hydrate, β-glycerophosphate and 0.5 nM T3. No. 4 shows the result when the culture broth was composed of DMEM, ITS, BSA, ascorbic acid phosphate magnesium salt n-hydrate, β-glycerophosphate and 0.05 nM T3. The longitudinal axis represents the alkaline phosphatase activity in cell surface protein extract.

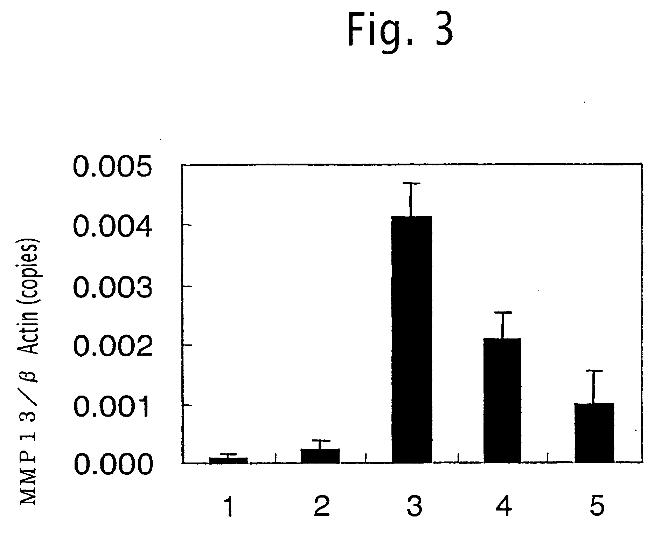

Fig. 3 shows the results of induction of *MMP13* gene expression in normal human knee arthrodial cartilage cells by T3. In this Figure, No. 1 in the horizontal axis shows the result when the culture broth was composed of DMEM, ITS and BSA. No. 2 shows the result when the culture broth was composed of DMEM, ITS, BSA, ascorbic acid phosphate magnesium salt n-hydrate and β-glycerophosphate. No. 3 shows the result when the culture broth was composed of DMEM, ITS, BSA, ascorbic acid phosphate magnesium salt n-hydrate, β-glycerophosphate and 10 nM T3. No. 4 shows the result when the culture broth was composed of DMEM, ITS, BSA, ascorbic acid phosphate magnesium salt n-hydrate, β-glycerophosphate and 1 nM T3. No. 5 shows the result when the culture broth was composed of DMEM, ITS, BSA, ascorbic acid phosphate magnesium salt n-hydrate, β-glycerophosphate and 0.1 nM T3. The longitudinal axis shows the number of MMP13 cDNA copies normalized by β actin.

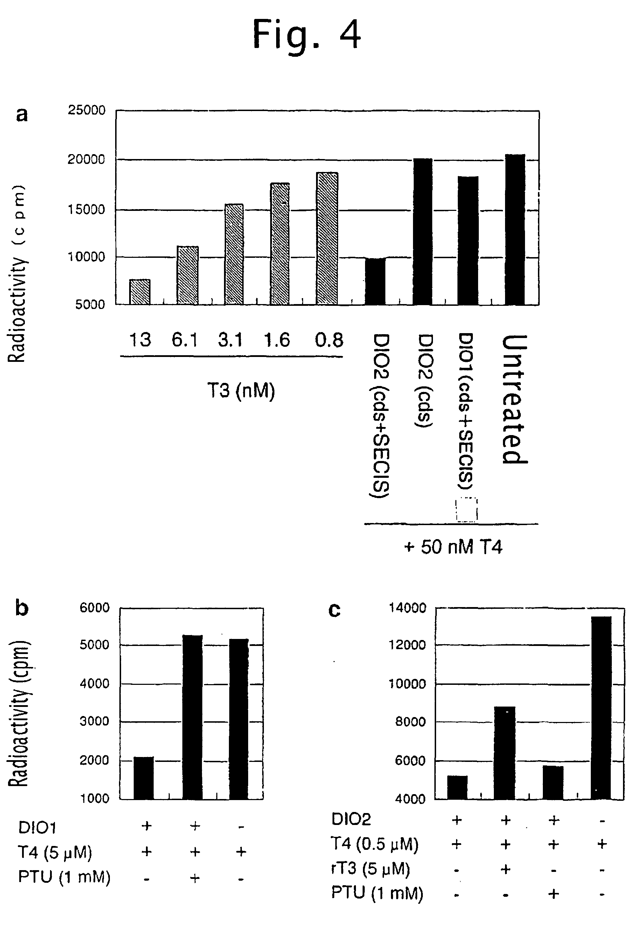

Fig. 4 shows the DIO1 and DIO2 activities measured by T3 RIA method.
   Fig. 4a) Columns provided with slant lines represent the results of measurement of T3 reagent at concentrations indicated in the horizontal axis by T3 RIA method. Filled columns show the results of measurement by T3 RIA method of T3 produced when disrupted COS-7 cells into which the indicated genes had been introduced or not introduced (untreated) were mixed with T4 reagent. The longitudinal axis shows radioactivity, the raw data obtained by T3 RIA method.
   Fig. 4b) This Figure shows the results of measurement by T3 RIA method of T3 reagent produced when DIO1-transferred, disrupted cells, T4 reagent and PTU reagent are mixed in combinations indicated in the horizontal axis. The longitudinal axis shows radioactivity, the raw data obtained by T3 RIA method.
   Fig. 4c) This Figure shows the results of measurement by T3 RIA method of T3 reagent produced when DIO1-transferred, disrupted cells, T4 reagent, rT3 reagent and PTU reagent are mixed in combinations indicated in the horizontal axis. The longitudinal axis shows radioactivity, the raw data obtained by T3 RIA method.

### BEST MODES FOR CARRYING OUT THE INVENTION

A protein comprising an amino acid sequence identical or substantially identical to the amino acid sequence represented by SEQ ID NO: 1 is optionally referred to herein as the "protein A of the invention" or the "protein A used in the invention".

A protein comprising an amino acid sequence identical or substantially identical to the amino acid sequence represented by SEQ ID NO: 3 is optionally referred to herein as the "protein B of the invention" or the "protein B used in the invention".

A protein comprising an amino acid sequence identical or substantially identical to the amino acid sequence represented by SEQ ID NO: 5 or SEQ ID NO: 7 is optionally referred to herein as the "protein C of the invention" or the "protein C used in the invention".

A protein comprising an amino acid sequence identical or substantially identical to the amino acid sequence represented by SEQ ID NO: 9 is optionally referred to herein as the "protein D of the invention" or the "protein D used in the invention".

A protein comprising an amino acid sequence identical or substantially identical to the amino acid sequence represented by SEQ ID NO: 11 is optionally referred to herein as the "protein E of the invention" or the "protein E used in the invention".

A protein comprising an amino acid sequence identical or substantially identical to the amino acid sequence represented by SEQ ID NO: 13 is optionally referred to herein as the "protein F of the invention" or the "protein F used in the invention".

A protein comprising an amino acid sequence identical or substantially identical to the amino acid sequence represented by SEQ ID NO: 1, SEQ ID NO: 3, SEQ ID NO: 5, SEQ ID NO: 7, SEQ ID NO: 9, SEQ ID NO: 11 or SEQ ID NO: 13 is optionally referred to herein as the "protein of the invention" or the "protein used in the invention".

The protein used in the invention may be a protein derived from cells of any kind (e.g. hepatocytes, splenocytes, nerve cells, glia cells, pancreatic *β* cells, bone marrow cells, mesangial cells, Langerhan's cells, epidermal cells, epithelial cells, goblet cells, endothelial cells, smooth muscle cells, fibroblasts, fibrous cells, muscle cells, fat cells, immune cells (e.g. macrophages, T cells, B cells, natural killer cells, mast cells, neutrophils, basophils, eosinophils, monocytes), megakaryocytes, synovial cells, chondrocytes, osteocytes, osteoblasts, osteoclasts, mammary cells, hepatocytes or interstitial cells, or progenitor cells, stem cells or cancer cells of these cells, etc.) of human or other warm-blooded animals (e.g. guinea pig, rat, mouse, chicken, rabbit, pig, sheep, bovine, monkey, etc.) or any tissue in which such cells are present, such as brain, various parts of brain (e.g. olfactory bulb, amygdaloid nucleus, cerebral basal nucleus, hippocampus, thalamus, hypothalamus, cerebral cortex, medulla oblongata, cerebellum), spinal cord, pituitary gland, stomach, pancreas, kidney, liver, gonad, thyroid, gall-bladder, bone marrow, adrenal gland, skin, muscle, lung, gastrointestinal tracts (e.g. large intestine, small intestine), blood vessels, heart, thymus, spleen, submandibular gland, peripheral blood, prostate, testis, ovary, placenta, uterus, bone, arthroculotis, skeletal muscle, etc. The protein used in the invention may also be a synthetic protein.

Examples of amino acid sequences substantially identical to the amino acid sequence represented by SEQ ID NO: 1 include amino acid sequences having about 50% or more, preferably about 60% or more, more preferably about 70% or more, still more preferably about 80% or more, especially preferably about 90% or more, most preferably about 95% or more homology to the amino acid sequence represented by SEQ ID NO: 1.

Examples of proteins comprising an amino acid sequence substantially identical to the amino acid sequence represented by SEQ ID NO: 1 include those proteins which comprise the above-described amino acid sequence substantially identical to the amino acid sequence represented by SEQ ID NO: 1 and have an activity substantially the same in quality as the activity of a protein comprising the amino acid sequence represented by SEQ ID NO: 1.

Examples of the "activity substantially the same in quality" include thyroxin 5'-deiodinase activity (e.g. DIO1 activity, DIO2 activity, etc.). The term "substantially the same in quality" means that they are equal in nature (e.g. physiologically, pharmacologically, etc.). Therefore, it is preferable that thyroxin 5'-deiodinase activities should be equivalent (e.g. about 0.01- to 100-fold, preferably about 0.1- to 10-fold, more preferably 0.5- to 2-fold). However, quantitative factors, such as the degrees of these activities, the molecular weights of proteins, etc. may be different.

The measurement of thyroxin 5'-deiodinase activity may be carried out by known methods. This activity may be measured according to the procedures, or modifications thereof, described in, for example, J. Clin. Invest. 98: 405-417, 1996; Endocrinology 142: 2961-2967, 2001; New Biochemical Experiment Series No. 9, the Japanese Biochemical Society (Ed.), 1992. For example, thyroxin 5'-deiodinase activity may be measured as follows: a plasmid carrying a cDNA encoding the protein A of the invention (e.g. DIO2) is introduced into COS-7 cells; the transformant COS-7 cells expressing the protein A of the invention (e.g. DIO2) are sonicated and then (1) reacted with a substrate T4 or reverse T3 (hereinafter, optionally abbreviated to "rT3"), followed by detection of the product by radioimmunoassay; or (2) reacted with a substrate T4 or rT3 labeled with ¹²⁵I, followed by detection of the liberated ¹²⁵I.

Examples of amino acid sequences substantially identical to the amino acid sequence represented by SEQ ID NO: 3 include amino acid sequences having about 50% or more, preferably about 60% or more, more preferably about 70% or more, still more preferably about 80% or more, especially preferably about 90% or more, most preferably about 95% or more homology to the amino acid sequence represented by SEQ ID NO: 3.

Examples of proteins comprising an amino acid sequence substantially identical to the amino acid sequence represented by SEQ ID NO: 3 include those proteins which comprise the above-described amino acid sequence substantially identical to the amino acid sequence represented by SEQ ID NO: 3 and have an activity substantially the same in quality as the activity of a protein comprising the amino acid sequence represented by SEQ ID NO: 3.

Examples of the "activity substantially the same in quality" include pyrophosphate transport activity. The term "substantially the same in quality" means that they are equal in nature (e.g. physiologically, pharmacologically, etc.). Therefore, it is preferable that pyrophosphate transport activities should be equivalent (e.g. about 0.01- to 100-fold, preferably about 0.1- to 10-fold, more preferably 0.5- to 2-fold). However, quantitative factors, such as the degrees of these activities, the molecular weights of proteins, etc. may be different.

The measurement of pyrophosphate transport activity may be carried out by known methods, e.g. the method described in Proc. Natl. Acad. Sci. USA 92: 10364-10368, 1995 or modifications thereof. For example, pyrophosphate transport activity may be measured as follows: a plasmid carrying a cDNA encoding the protein B of the invention (e.g. ANKH) is introduced into mouse ATDC5 cells (Cell Differentiation and Development 30: 109-116, 1990) to allow high expression of the protein B of the invention (e.g. ANKH); the culture broth of this transformant is recovered and reacted with a mixed solution of inorganic pyrophosphatase, maltose, maltose phosphorylase, glucose oxidase, peroxidase and 10-acetyl-3,7-dihydroxyphenoxazine; then, the pyrophosphate in the culture broth is measured as a fluorescent product resorfin to thereby determine the pyrophosphate transport activity.

Examples of amino acid sequences substantially identical to the amino acid sequence represented by SEQ ID NO: 5 or SEQ ID NO: 7 include amino acid sequences having about 50% or more, preferably about 60% or more, more preferably about 70% or more, still more preferably about 80% or more, especially preferably about 90% or more, most preferably about 95% or more homology to the amino acid sequence represented by SEQ ID NO: 5 or SEQ ID NO: 7.

Examples of proteins comprising an amino acid sequence substantially identical to the amino acid sequence represented by SEQ ID NO: 5 or SEQ ID NO: 7 include those proteins which comprise the above-described amino acid sequence substantially identical to the amino acid sequence represented by SEQ ID NO: 5 or SEQ ID NO: 7 and have an activity substantially the same in quality as the activity of a protein comprising the amino acid sequence represented by SEQ ID NO: 5 or SEQ ID NO: 7.

Examples of the activity substantially the same in quality" include cell differentiation-regulating activity (e.g. cartilage cell differentiation-regulating activity, osteoblast differentiation-regulating activity, etc.). The term "substantially the same in quality" means that they are equal in nature (e.g. physiologically, pharmacologically, etc.). Therefore, it is preferable that cell differentiation-regulating activities should be equivalent (e.g. about 0.01- to 100-fold, preferably about 0.1- to 10-fold, more preferably 0.5- to 2-fold). However, quantitative factors, such as the degrees of these activities, the molecular weights of proteins, etc. may be different.

The measurement of cell differentiation-regulating activity may be carried out by known methods, e.g. those methods described in Journal of Bone and Mineral Research 11: 105-113, 1996; The Journal of Cell, Biology 154: 659-666, 2001, or modifications thereof.

Specifically, cell differentiation-regulating activity may be measured as follows: a plasmid carrying a cDNA encoding the protein C of the invention (e.g. SHOX2) is introduced into mouse ATDC5 cells (Cell Differentiation and Development 30: 109-116, 1990) to create a cell strain that expresses the protein C of the invention (e.g. SHOX2) stably; the culture broth of this transformant is recovered and reacted with a mixed solution of inorganic pyrophosphatase, maltose, maltose phosphorylase, glucose oxidase, peroxidase and 10-acetyl-3,7-dihydroxyphenoxazine; then, the protein C (e.g. SHOX2)-expressing mouse ATDC5 cells are cultured in the presence of various growth factors (e.g. BMP-2, BMP-4, insulin, TGF-β, FGF-1, FGF-2, etc.; among all, BMP-2, BMP-4 and insulin are preferable) to thereby induce the differentiation of these cells into cartilage. After the induction, cell differentiation-regulating activity is measured by any of the following procedures: (1) the expression of X type collagen is detected by RT-PCR; (2) alkaline phosphatase activity is measured using TRACP & ALP double-stain kit (Takara Shuzo) or the like; (3) calcium deposited in the cell layer is detected by alizarin red staining or the like; or (4) the cell layer is labeled with ⁴⁵Ca²⁺ and the deposited calcium is determined. If necessary, an inducible promoter is used for the expression of the protein C of the invention (e.g. SHOX2). Alternatively, gene transfer may be performed after the early differentiation of cartilage.

Specifically, a gene encoding the protein C of the invention (e.g. SHOX2) is introduced into, for example, MC3T3-E1 cells (J. Cell Biol., 96: 191-198, 1983), and then (1) the expression of a cell differentiation marker gene (e.g. type I collagen, osteopontin, osteocalcin, etc.) is detected by RT-PCT; (2) alkaline phosphatase activity is measured using TRACP & ALP double-stain kit (Takara Shuzo) or the like; (3) calcium deposited in the cell layer is detected by alizarin red staining or the like; or (4) the cell layer is labeled with ⁴⁵Ca²⁺ and the deposited calcium is determined. Thus, cell differentiation-regulating activity can be measured. If necessary, an inducible promoter is used for the expression of the protein C of the invention (e.g. SHOX2). Alternatively, gene transfer may be performed after the early differentiation

The measurement of cell differentiation-regulating activity may be carried out by known methods, e.g. the method described in J. Biol. Chem. 275: 1065-72, 2000 or a modification thereof. Specifically, a reporter plasmid in which the expression control region of a cell differentiation marker gene (e.g. osteopontin, type I collagen, type II collagen, type X collagen, etc.) is linked to luciferase gene and an expression plasmid for the protein C of the invention (e.g. SHOX2) are introduced into mouse 3H10T1/2 cells. Then, luciferase activity is measured to thereby determine cell differentiation-regulating activity.

Examples of amino acid sequences substantially identical to the amino acid sequence represented by SEQ ID NO: 9 include amino acid sequences having about 50% or more, preferably about 60% or more, more preferably about 70% or more, still more preferably about 80% or more, especially preferably about 90% or more, most preferably about 95% or more homology to the amino acid sequence represented by SEQ ID NO: 9.

Examples of proteins comprising an amino acid sequence substantially identical to the amino acid sequence represented by SEQ ID NO: 9 include those proteins which comprise the above-described amino acid sequence substantially identical to the amino acid sequence represented by SEQ ID NO: 9 and have an activity substantially the same in quality as the activity of a protein comprising the amino acid sequence represented by SEQ ID NO: 9.

Examples of the "activity substantially the same in quality" include potassium ion transport activity. The term "substantially the same in quality" means that they are equal in nature (e.g. physiologically, pharmacologically, etc.). Therefore, it is preferable that pyrophosphate transport activities should be equivalent (e.g. about 0.01- to 100-fold, preferably about 0.1- to 10-fold, more preferably 0.5- to 2-fold). However, quantitative factors, such as the degrees of these activities, the molecular weights of proteins, etc. may be different.

The measurement of potassium ion transport activity may be carried out by known methods, e.g. the method described in Biochem. Biophys. Res. Commun. 282: 249-256, 2001 or a modification thereof. For example, a plasmid carrying a cDNA encoding the protein D of the invention (e.g. TASK-4) is introduced into mouse ATDC5 cells (Cell Differentiation and Development 30: 109-116, 1990) to allow high expression of the protein D of the invention (e.g. TASK-4). Using the resultant transformant, potassium ion transport activity may be measured by the patch-clump technique or the like.

Examples of amino acid sequences substantially identical to the amino acid sequence represented by SEQ ID NO: 11 include amino acid sequences having about 50% or more, preferably about 60% or more, more preferably about 70% or more, still more preferably about 80% or more, especially preferably about 90% or more, most preferably about 95% or more homology to the amino acid sequence represented by SEQ ID NO: 11.

Examples of proteins comprising an amino acid sequence substantially identical to the amino acid sequence represented by SEQ ID NO: 11 include those proteins which comprise the above-described amino acid sequence substantially identical to the amino acid sequence represented by SEQ ID NO: 11 and have an activity substantially the same in quality as the activity of a protein comprising the amino acid sequence represented by SEQ ID NO: 11.

Examples of the "activity substantially the same in quality" include signal transduction activity and ligand-binding activity. The term "substantially the same in quality" means that such activities are equal in nature (e.g. physiologically, pharmacologically, etc.). Therefore, it is preferable that signal transduction activities or ligand-binding activities should be equivalent (e.g. about 0.01- to 100-fold, preferably about 0.1- to 10-fold, more preferably 0.5- to 2-fold). However, quantitative factors, such as the degrees of these activities, the molecular weights of proteins, etc. may be different.

The measurement of signal transduction activity may be carried out based on known methods. For example, using (1) Ephrin/Fc chimeric protein (R&D system) or (2) cultured cells expressing a ligand for the protein E of the invention (e.g. EphA3), stimulatory activity against the protein E (e.g. EphA3)-expressing cells (e.g. activity to promote or inhibit the autophosphorylation of EphA3, intracellular Ca²⁺ liberation, inositol phosphate production, changes in cell membrane potentials, the phosphorylation of intracellular proteins, c-fos activation, the lowering of pH, etc.) is measured by known methods. Specifically, a plasmid carrying a cDNA encoding the protein E of the invention (e.g.EphA3) is introduced into mouse ATDC5 cells (Cell Differentiation and Development 30: 109-116, 1990) to allow high expression of the protein E of the invention (e.g. EphA3). The resultant transformant is (1) cultured in the presence of Ephrin/Fc chimeric protein (R&D system) or (2) co-cultured with cells expressing a ligand for the protein E of the invention (e.g. EphA3). Then, signal transduction activity mediated by the protein E of the invention (e.g. EphA3) expressed on the cell membrane (e.g. activity to promote or inhibit the autophosphorylation of EphA3, intracellular Ca²⁺ liberation, inositol phosphate production, changes in cell membrane potentials, the phosphorylation of intracellular proteins, c-fos activation, the lowering of pH, etc.) is measured by known methods.

Ligand-binding activity may be measured based on known methods. Briefly, (1) a labeled ligand for the protein E of the invention (e.g. EphA3) is contacted with the protein E, followed by measuring the amount of binding of the labeled ligand to the protein; (2) a labeled ligand for the protein E of the invention (e.g. EphA3) is contacted with the protein E-containing cells or the membrane fraction of the cells, followed by measuring the amount of binding of the labeled ligand to the cells or the membrane fraction; or (3) a labeled ligand for the protein E of the invention (e.g. EphA3) is contacted with the protein E which has been expressed on cell membranes by culturing a transformant comprising a DNA encoding the protein E of the invention, followed by measuring the amount of binding of the labeled ligand to the cells or the membrane fraction thereof.

Specific examples of the ligand for the protein E of the invention (e.g. EphA3) include proteins belonging to the ephrin A family (e.g. ephrin-A2, ephrin-A5, etc.) (Wilkinson D.G., Nat. Rev. Neurosci. 2: 155-64, 2001).

Examples of amino acid sequences substantially identical to the amino acid sequence represented by SEQ ID NO: 13 include amino acid sequences having about 50% or more, preferably about 60% or more, more preferably about 70% or more, still more preferably about 80% or more, especially preferably about 90% or more, most preferably about 95% or more homology to the amino acid sequence represented by SEQ ID NO: 13.

Examples of proteins comprising an amino acid sequence substantially identical to the amino acid sequence represented by SEQ ID NO: 13 include those proteins which comprise the above-described amino acid sequence substantially identical to the amino acid sequence represented by SEQ ID NO: 13 and have an activity substantially the same in quality as the activity of a protein comprising the amino acid sequence represented by SEQ ID NO: 13.

Examples of the "activity substantially the same in quality" include matrix metalloproteinase activity. The term "substantially the same in quality" means that such activities are equal in nature (e.g. physiologically, pharmacologically, etc.). Therefore, it is preferable that matrix metalloproteinase activities should be equivalent (e.g. about 0.01- to 100-fold, preferably about 0.1- to 10-fold, more preferably 0.5- to 2-fold). However, quantitative factors, such as the degrees of these activities, the molecular weights of proteins, etc. may be different.

The measurement of matrix metalloproteinase activity may be carried out by known methods, e.g. the method described in J. Biol. Chem. 271: 17119-17123, 1996 or a modification thereof. Specifically, a recombinant protein of the protein F of the invention (e.g. MMP-16) which lacks the membrane-binding region and the intracellular region is created according to the method, or a modification thereof, described in Eur. J. Biochem. 262: 907-914, 1999. Then, the protein is retained at 37°C in a buffer containing a substarate (20 mM sodium tetraborate, pH 9.0, 10 mM CaCl₂, 100 mM NaCl, 0.02% NaN₃, 0.025% Brij 35 (CN Biosciences)) and then fluorescence intensity is measured to determine the activity according to the method, or a modification thereof, described in J. Biol. Chem. 271: 17119-17123, 1996. The substrate may be any substrate as long as the protein F of the invention (e.g. MMP-16) cuts it. For example, (7 - methoxycoumarin - 4 - yl ) acetyl - Pro - Leu-Gly-Leu-[3-(2,4-Dinitrophenyl)-L-2,3-diaminopropionyl]-Ala-Arg-NH₂ may be used.

Alternatively, the matrix metalloproteinase activity may be measured according to the PER method (Electrophoresis 16: 43, 1995).

Further, the activation of MMP-2 (which is a substrate for MMP-16 and is activated by MMP-16) may be detected by zymography according to known methods such as described in J. Biol. Chem. 270: 23013-23020, 1995. It is also possible to measure the matrix metalloproteinase activity by analyzing the substrate protein degraded by MMP-16 using SDS-PAGE according to the method, or a modification thereof, described in Eur. J. Biochem. 262: 907-914, 1999.

The protein used in the invention include the so-called muteins, such as proteins comprising (i) the amino acid sequence of SEQ ID NO: 1, SEQ ID NO: 3, SEQ ID NO: 5, SEQ ID NO: 7, SEQ ID NO: 9, SEQ ID NO: 11 or SEQ ID NO: 13 wherein 1 or 2 or more amino acids (preferably about 1-30, more preferably about 1-10, and still more preferably several (1-5) amino acids) are deleted therefrom; (ii) the amino acid sequence of SEQ ID NO: 1, SEQ ID NO: 3, SEQ ID NO: 5, SEQ ID NO: 7, SEQ ID NO: 9, SEQ ID NO: 11 or SEQ ID NO: 13 wherein 1 or 2 or more amino acids (preferably about 1-30, more preferably about 1-10, and still more preferably several (1-5) amino acids) are added thereto; (iii) the amino acid sequence of SEQ ID NO: 1, SEQ ID NO: 3, SEQ ID NO: 5, SEQ ID NO: 7, SEQ ID NO: 9, SEQ ID NO: 11 or SEQ ID NO: 13 wherein 1 or 2 or more amino acids (preferably about 1-30, more preferably about 1-10, and still more preferably several (1-5) amino acids) are inserted thereinto; (iv) the amino acid sequence of SEQ ID NO: 1, SEQ ID NO: 3, SEQ ID NO: 5, SEQ ID NO: 7, SEQ ID NO: 9, SEQ ID NO: 11 or SEQ ID NO: 13 wherein 1 or 2 or more amino acids (preferably about 1-30, more preferably about 1-10, and still more preferably, several (1-5) amino acids) are replaced with other amino acids; or (v) an amino acid sequence which is a combination of these sequences.

When the amino acid sequence has such an insertion, deletion or substitution as described above, the site of the insertion, deletion or substitution is not particularly restricted.

The proteins in the present, specification are expressed in accordance with the conventions for description of peptides, that is, the N-terminus (amino terminus) at the left end and the C-terminus (carboxyl terminus) at the right end. The C-terminus of the protein used in the invention may be either a carboxyl group (-COOH), a carboxylate (-COO), an amide (-CONH₂) or an ester (-COOR).

Examples of R of the above ester group include C₁₋₆ alkyl groups (e.g. methyl, ethyl, n-propyl, isopropyl or n-butyl), C₃₋₈ cycloalkyl groups (e.g. cyclopentyl or cyclohexyl), C₆₋₁₂ aryl groups (e.g. phenyl or α-naphthyl), C₇₋₁₄ aralkyl groups such as phenyl-C₁₋₂ alkyl groups (e.g. benzyl or phenethyl) and α -naphthyl-C₁₋₂ alkyl groups (e.g. α - naphthylmethyl), and pivaloyloxymethyl esters.

When the protein used in the invention has a carboxyl group (or carboxylate) at any position other than its C-terminus, the carboxyl group may be amidated or esterified; such a protein is also included in the protein used in the invention. The ester in this case may be, for example, any of the esters mentioned above for the C-terminal ester.

Furthermore, the protein used in the invention includes those proteins in which the N-terminal amino acid residue (e.g. Met) is protected by a protective group (e.g. C₁₋₆ acyl group such as C₁₋₆ alkanoyl group (e.g. formyl group or acetyl group)); those proteins in which the N-terminal Glu generated through *in vivo* cleavage is pyroglutaminated; those proteins in which a substituent on a side chain of an amino acid (e.g. -OH, -SH, amino group, imidazole group, indole group, or guannidino group) is protected by an appropriate protective group (e.g. C₁₋₆ acyl group such as C₁₋₆ alkanoyl group (e.g. formyl group or acetyl group)); and conjugated proteins such as the so-called glycoproteins to which sugar chains are linked.

Specific examples of the protein A used in the invention include a protein comprising the amino acid sequence represented by SEQ ID NO: 1 (DIO2).

Specific examples of the protein B used in the invention include a protein comprising the amino acid sequence represented by SEQ ID NO: 3 (ANKH).

Specific examples of the protein C used in the invention include a protein comprising the amino acid sequence represented by SEQ ID NO: 5 (SHOX2a) and a protein comprising the amino acid sequence represented by SEQ ID NO: 7 (SHOX2b).

Specific examples of the protein D used in the invention include a protein comprising the amino acid sequence represented by SEQ ID NO: 9 (TASK-4).

Specific examples of the protein E used in the invention include a protein comprising the amino acid sequence represented by SEQ ID NO: 11 (EphA3).

Specific examples of the protein F used in the invention include a protein comprising the amino acid sequence represented by SEQ ID NO: 13 (MMP 16).

The partial peptide of the protein used the invention may be any partial peptide as long as it is a partial peptide of the protein used in the invention and, preferably, has a nature similar to that of the protein used in the invention. For example, a partial peptide may be used which has an amino acid sequence of at least 20 or more, preferably 50 or more, more preferably 70 or more, still more preferably 100 or more, most preferably 200 or more amino acids of the amino acid sequence constituting the protein used in the invention. Specifically, a peptide having an amino acid sequence spanning from position 48 to position 62 of the amino acid sequence represented by SEQ ID NO: 1; a peptide having an amino acid sequence spanning from position 105 to position 130 of the amino acid sequence represented by SEQ ID NO: 3; a peptide having an amino acid sequence spanning from position 105 to position 130 of the amino acid sequence represented by SEQ ID NO: 9; a peptide having an amino acid sequence spanning from position 105 to position 130 of the amino acid sequence represented by SEQ ID NO: 11; and the like may be used.

For example, peptides having an amino acid sequence of at least 20 or more, preferably 50 or more, more preferably 70 or more, still more preferably 100 or more, most preferably 200 or more amino acids of the amino acid sequence constituting the protein used in the invention may be used.

Further, the partial peptide used in the invention may comprise the above-described amino acid sequence where 1 or 2 or more amino acids (preferably about 1-10 amino acids, more preferably several (1-5) amino acids) are deleted; 1 or 2 or more amino acids (preferably about 1-20 amino acids, more preferably about 1-10 amino acids, still more preferably several (1-5) amino acids) are added; 1 or 2 or more amino acids (preferably about 1-20 amino acids, more preferably about 1-10 amino acids, still more preferably several (1-5) amino acids) are inserted; or 1 or 2 or more amino acids (preferably about 1-10 amino acids, more preferably several amino acids, still more preferably about 1-5 amino acids) are substituted with other amino acids.

The C-terminus of the partial peptide used in the invention may be either a carboxyl group (-COOH), a carboxylate (-COO), an amide (-CONH₂) or an ester (-COOR).

Further, like the protein used in the invention described above, the partial peptide used in the present invention also includes those peptides in which the N-terminal amino acid residue (e.g. Met) is protected by a protective group; those peptides in which the N-terminal Glu generated through *in vivo* cleavage is pyrogluminated; those peptides in which a substituent on a side chain of an amino acid is protected by an appropriate protective group; and conjugated peptides such as the so-called glycopeptides to which sugar chains are linked.

The partial peptide used in the invention may also be used as an antigen for antibody preparation.

As salts of the protein or the partial peptide used in the invention, salts formed with physiologically acceptable acids (e.g. organic or inorganic acids) or bases (e.g. alkali metals) are used. Especially preferable are physiologically acceptable acid addition salts. Examples of such salts include salts formed with inorganic acids (e.g. hydrochloric acid, phosphoric acid, hydrobromic acid or sulfuric acid) and salts formed with organic acids (e.g. acetic acid, formic acid, propionic acid, fumaric acid, maleic acid, succinic acid, tartaric acid, citric acid, malic acid, oxalic acid, benzoic acid, methanesulfonic acid or benzenesulfonic acid).

The protein or partial peptide used in the invention or salts thereof can be produced from the afore-mentioned cells or tissues of human or other warm-blooded animals by known purification methods for proteins. Alternatively, they can also be produced by culturing a transformant comprising a DNA encoding the protein. They can also be produced in accordance with the procedures for peptide synthesis which are described later.

When the protein or salts thereof or the partial peptide or salts thereof are produced from tissues or cells of human or other mammals, the relevant tissue or cell is homogenized and then the protein, etc. of interest is extracted with acids, etc. The protein, etc. of interest can be purified and isolated from the resultant extract by a combination of chromatography, such as reversed phase chromatography, ion exchange chromatography and so on.

For the synthesis of the protein or partial peptide used in the invention, or salts thereof, or amides thereof, any of the commercial resins available for protein synthesis may be used. Examples of such resins include chloromethyl resin, hydroxymethyl resin, benzhydrylamine resin, aminomethyl resin, 4-benzyloxybenzyl alcohol resin, 4 - methylbenzhydrylamine resin, PAM resin, 4-hydroxymethylmethylphenylacetamidomethyl resin, polyacrylamide resin, 4-(2',4'-dimethoxyphenylhydroxymethyl) phenoxy resin, and 4-(2',4'-dimethoxyphenyl-Fmoc-aminoethyl) phenoxy resin. Using such a resin, amino acids protected at their α-amino groups and side chain functional groups are condensed on the resin according to the amino acid sequence of the protein of interest by conventional condensation methods. At the final stage of the reaction, all protective groups are removed simultaneously with the cleavage of the protein from the resin. Then, in a highly diluted solution, intramolecular disulfide bond formation reaction is carried out to obtain the protein of interest, partial peptides thereof or the protein or partial peptide thereof in an amide form.

With respect to the condensation of the above-described protected amino acids, various activators useful for protein synthesis may be utilized. Among all, carbodiimide reagents are especially preferred. Examples of carbodiimide reagents include DCC, N,N'-diisopropylcarbodiimide, and N-ethyl-N'-(3-dimethylaminoprolyl)carbodiimide. For activation by these reagents, protected amino acids and a recemization inhibitor (e.g. HOBt or HOOBt) may be directly added to the resin, or protected amino acids may be activated in advance in the form of symmetric acid anhydride, HOBt ester or HOOBt ester and then added to the resin.

The solvent used for the above-mentioned activation of protected amino acids or the condensation thereof with a resin may be appropriately selected from those solvents known to be useful for protein condensation reactions. Examples of useful solvents include acid amides (e.g. N,N-dimethylformamide, N,N-dimethylacetamide or N - methylpyrrolidone ), halogenated hydrocarbons (e.g. methylene chloride, or chloroform), alcohols (e.g. trifluoroethanol), sulfoxides (e.g. dimethyl sulfoxide), ethers (e.g. pyridine, dioxane, tetrahydrofuran), nitriles (e.g. acetonitrile or propionitrile), esters (e.g. methyl acetate or ethyl acetate), and suitable mixtures of these solvents. The reaction temperature may be appropriately selected from the range known to be useful for protein bond-forming reactions; usually, the temperature is selected from the range from about -20 °C to about 50°C. The activated amino acid derivative is usually used in 1.5- to 4-fold excess. When the condensation is found insufficient as a result of test using the ninhydrin reaction, sufficient condensation can be achieved by repeating reactions without removing protective groups. When sufficient condensation cannot be achieved even by repeating reactions, unreacted amino acids may be acetylated with acetic anhydride or acetylimidazole so that they do not affect subsequent reactions.

Examples of useful protective groups for the amino group of raw materials include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulfenyl, diphenylphosphinothioyl, and Fmoc.

The carboxyl group can be protected, for example, in the form of an alkyl ester (e.g. straight-chain, branched, or cyclic alkyl esters such as methyl, ethyl, propyl, butyl, t-butyl, cyclopentyl, cyclohexyl, cycloheptyl, cyclooctyl, 2-adamantyl, and so on), aralkyl ester (e.g. benzyl, 4-nitrobenzyl, 4-methoxybenzyl, 4-chlorobenzyl, benzhydryl, and so on), phenacyl ester, benzyloxycarbonylhydrazide, t-butoxycarbonylhydrazide or tritylhydrazide.

The hydroxyl group of serine can be protected, for example, by esterification or etherification. Examples of suitable groups for this esterification include lower (C₁₋₆) alkanoyl groups such as acetyl, aroyl groups such as benzoyl, and carbonic acid-derived groups such as benzyloxycarbonyl and ethyloxycarbonyl. Examples of groups suitable for the etherification include benzyl, tetrahydropyranyl and t-butyl.

Examples of protective groups for the phenolic hydroxyl group of tyrosine include Bzl, Cl₂-Bzl, 2-nitrobenzyl, BrZ, and t-butyl.

Examples of protective groups for the imidazole ring of histidine include Tos, 4-methoxy-2,3,6-trimethylbenzenesulfonyl, DNP, benzyloxymethyl, Bum, Boc, Trt and Fmoc.

Examples of raw materials with activated carboxyl groups include the corresponding acid anhydrides, azides and active esters (esters of alcohols such as pentachlorophenol, 2,4,5-trichlorophenol, 2,4-dinitrophenol, cyanomethyl alcohol, p-nitrophenol, HONB, N-hydroxysuccinimide, N-hydroxyphthalimide and HOBt). Examples of raw materials with activated amino groups include the corresponding phosphoric acid amides.

Methods for removing (eliminating) protective groups include, for example, catalytic reduction in a hydrogen stream in the presence of a catalyst such as Pd black or Pd-carbon, acid treatment with anhydrous hydrogen fluoride, methanesulfonic acid, trifluoromethanesulfonic acid, trifluoroacetic acid or mixtures thereof, treatment with a base such as diiso-propylethylamine, triethylamine, piperidine, piperazine or the like, and reduction with sodium in liquid ammonia. The elimination reaction by the above-mentioned acid treatment is generally conducted at temperatures of about -20°C to about 40°C. In the acid treatment, it is effective to add a cation scavenger such as anisole, phenol, thioanisole, m-cresol, p-cresol, dimethylsulfide, 1,4-butanedithiol or 1,2-ethanedithiol. The 2,4-dinitrophenyl group used as the protective group for the imidazole ring of histidine is removed by thiophenol treatment. The formyl group used as the protective group for the indole ring of tryptophan may be removed by the above-mentioned deprotection by the acid treatment in the presence of 1,2-ethanedithiol, 1,4-butanedithiol or the like, or by alkali treatment using dilute sodium hydroxide, dilute ammonia or the like.

The protection of functional groups in raw materials that should not be involved in the reaction, protective groups therefor, the removal of these protective groups and the activation of functional groups involved in the reaction can be appropriately selected from groups or methods known in the art.

An alternative method for obtaining amides of the protein or the partial peptide of the invention comprises, for example, protecting the α-carboxyl group of the C-terminal amino acid by amidation, extending the peptide (protein) chain to a desired length on the side of the amino group, preparing a protein or a partial peptide with its N-terminal α-amino group selectively deprotected, preparing another protein or another partial peptide with its C-terminal carboxyl group selectively deprotected, and condensing these two proteins or partial peptides in a mixed solvent such as described above. Details of this condensation reaction are the same as described above. After purification of the protected protein or peptide thus obtained by condensation, all the protective groups are removed by the method described above to thereby to provide a crude protein or peptide of interest. This crude protein or peptide is purified by various known purification techniques and lyophilized to provide the desired protein or peptide in an amide form.

As a method for obtaining esters of the protein or partial peptide used in the invention, for example, the α-carboxyl group of the C-terminal amino acid is condensed with a desired alcohol to prepare the corresponding amino acid ester, and then this ester is subjected to the same procedures as described above in the preparation of amides to thereby obtain the desired protein or peptide in an ester form.

The partial peptide used in the invention or salts thereof can be produced by known methods for peptide synthesis. Alternatively, the partial peptide or salts thereof may be produced by cutting the protein of the invention with an appropriate peptidase. The method for peptide synthesis may be solid-phase synthesis or liquid-phase synthesis. Briefly, a peptide of interest can be produced by condensing a partial peptide or amino acids capable of constituting the partial peptide of the invention with the remaining part thereof and, if the product has protective groups, removing the protective groups. Examples of condensation methods and methods for removal of protective groups known in the art include those described in the following references (i) to (v).
(i) M. Bodanszky & M.A. Ondetti, Peptide Synthesis, Interscience Publishers, New York, 1966
(ii) Schroeder & Luebke, The Peptide, Academic Press, New York, 1965
(iii) Nobuo Izumiya et al., Fundamentals and Experiments in Peptide Synthesis, Maruzen, 1975
(iv) Haruaki Yajima and Shumpei Sakakibara, Biochemical Experiment Series 1, Protein Chemistry IV, 205,1977, and
(v) Haruald Yajima (ed.), Development of Drugs (Continued), Vol. 14, Peptide Synthesis, Hirokawa Shoten

After the reaction, the partial peptide can be isolated and purified by a combination of conventional purification techniques such as solvent extraction, distillation, column chromatography, liquid chromatography, and recrystallization. When the partial peptide thus obtained is a free peptide, it can be converted to a suitable salt by known methods or modifications thereof. On the contrary, when the partial peptide is obtained in a salt form, it can be converted to a free peptide or another salt by known methods or modifications thereof.

The DNA encoding the protein used in the invention may be any DNA as long as it comprises the above-described nucleotide sequence encoding the protein used in the invention. Preferably, the DNA is a DNA. The DNA may be genomic DNA, genomic DNA library, cDNA derived from the above-mentioned cells or tissues, cDNA library derived from the above-mentioned cells or tissues, or synthetic DNA.

Vectors used for library construction may be any vectors such as bacteriophage, plasmid, cosmid, phagemid, and so on. Alternatively, total RNA or mRNA fraction may be prepared from the above-mentioned cells or tissues, followed by direct amplification by reverse transcriptase polymerase chain reaction (hereinafter, abbreviated to "RT-PCR").

Specific examples of the DNA encoding the protein A used in the invention include any DNA comprising the nucleotide sequence represented by SEQ ID NO: 2, or any DNA which comprises a nucleotide sequence hybridizing to the nucleotide sequence represented by SEQ ID NO: 2 under high stringency conditions and encodes a protein having a nature substantially the same in quality to the nature of a protein comprising an amino acid sequence represented by SEQ ID NO: 1.

Specific examples of the DNA encoding the protein B used in the invention include any DNA comprising the nucleotide sequence represented by SEQ ID NO: 4, or any DNA which comprises a nucleotide sequence hybridizing to the nucleotide sequence represented by SEQ ID NO: 4 under high stringency conditions and encodes a protein having a nature substantially the same in quality to the nature of a protein comprising an amino acid sequence represented by SEQ ID NO: 3.

Specific examples of the DNA encoding the protein C used in the invention include any DNA comprising the nucleotide sequence represented by SEQ ID NO: 6 or SEQ ID NO: 8, or any DNA which comprises a nucleotide sequence hybridizing to the nucleotide sequence represented by SEQ ID NO: 6 or SEQ ID NO: 8 under high stringency conditions and encodes a protein having a nature substantially the same in quality to the nature of a protein comprising an amino acid sequence represented by SEQ ID NO: 5 or SEQ ID NO: 8.

Specific examples of the DNA encoding the protein D used in the invention include any DNA comprising the nucleotide sequence represented by SEQ ID NO: 10, or any DNA which comprises a nucleotide sequence hybridizing to the nucleotide sequence represented by SEQ ID NO: 10 under high stringency conditions and encodes a protein having a nature substantially the same in quality to the nature of a protein comprising an amino acid sequence represented by SEQ ID NO: 9.

Specific examples of the DNA encoding the protein E used in the invention include any DNA comprising the nucleotide sequence represented by SEQ ID NO: 12, or any DNA which comprises a nucleotide sequence hybridizing to the nucleotide sequence represented by SEQ ID NO: 12 under high stringency conditions and encodes a protein having a nature substantially the same in quality to the nature of a protein comprising an amino acid sequence represented by SEQ ID NO: 11.

Specific examples of the DNA encoding the protein F used in the invention include any DNA comprising the nucleotide sequence represented by SEQ ID NO: 14, or any DNA which comprises a nucleotide sequence hybridizing to the nucleotide sequence represented by SEQ ID NO: 14 under high stringency conditions and encodes a protein having a nature substantially the same in quality to the nature of a protein comprising an amino acid sequence represented by SEQ ID NO: 13.

As DNAs capable of hybridizing to the nucleotide sequence represented by SEQ ID NO: 2, SEQ ID NO: 4, SEQ ID NO: 6, SEQ ID NO: 8, SEQ ID NO: 10, SEQ ID NO: 12 or SEQ ID NO: 14 under high stringency conditions, DNAs comprising a nucleotide sequence having about 50% or more, preferably about 60% or more, more preferably about 70% or more, still more preferably about 80% or more, especially preferably about 90% or more, most preferably about 95% or more homology to the nucleotide sequence represented by SEQ ID NO: 2, SEQ ID NO: 4, SEQ ID NO: 6, SEQ ID NO: 8, SEQ ID NO: 10, SEQ ID NO: 12 or SEQ ID NO: 14 may be used, for example.

Hybridization can be carried out according to known methods or modifications thereof, e.g. those methods described in "Molecular Cloning," 2nd Ed. (J. Sambrook et al., Cold Spring Harbor Lab. Press, 1989). When commercial libraries are used, hybridization can be carried out in accordance with the methods described in the instructions attached thereto; more preferably, hybridization is carried out under high stringency conditions.

"High stringency conditions" refers to, for example, conditions where sodium concentration is about 19-40 mM, preferably about 19-20 mM, and temperature is about 50-70 °C, preferably about 60-65°C. In particular, conditions where sodium concentration is about 19 mM and temperature is about 65°C are most preferable.

More specifically, as a DNA encoding a protein comprising the amino acid sequence represented by SEQ ID NO: 1, a DNA comprising the nucleotide sequence represented by SEQ ID NO: 2 may be used, for example. As a DNA encoding a protein comprising the amino acid sequence represented by SEQ ID NO: 3, a DNA comprising the nucleotide sequence represented by SEQ ID NO: 4 may be used, for example. As a DNA encoding a protein comprising the amino acid sequence represented by SEQ ID NO: 5, a DNA comprising the nucleotide sequence represented by SEQ ID NO: 6 may be used, for example. As a DNA encoding a protein comprising the amino acid sequence represented by SEQ ID NO: 7, a DNA comprising the nucleotide sequence represented by SEQ ID NO: 8 may be used, for example. As a DNA encoding a protein comprising the amino acid sequence represented by SEQ ID NO: 9, a DNA comprising the nucleotide sequence represented by SEQ ID NO: 10 may be used, for example. As a DNA encoding a protein comprising the amino acid sequence represented by SEQ ID NO: 11, a DNA comprising the nucleotide sequence represented by SEQ ID NO: 12 may be used, for example. As a DNA encoding a protein comprising the amino acid sequence represented by SEQ ID NO: 13, a DNA comprising the nucleotide sequence represented by SEQ ID NO: 14 may be used, for example.

The DNA encoding the partial peptide used the invention may be any DNA as long as it comprises the above-described nucleotide sequence encoding the partial peptide used in the invention. The DNA may be genomic DNA, genomic DNA library, cDNA derived from the above-mentioned cells or tissues, cDNA library derived from the above-mentioned cells or tissues, or synthetic DNA.

Specific examples of the DNA encoding the partial peptide used in the invention include DNAs having a part of a DNA comprising the nucleotide sequence represented by SEQ ID NO: 2, SEQ ID NO: 4, SEQ ID NO: 6, SEQ ID NO: 8, SEQ ID NO: 10, SEQ ID NO: 12 or SEQ ID NO: 14; or DNAs having a part of a DNA which comprises a nucleotide sequence hybridizing to the nucleotide sequence represented by SEQ ID NO: 2, SEQ ID NO: 4, SEQ ID NO: 6, SEQ ID NO: 8, SEQ ID NO: 10, SEQ ID NO: 12 or SEQ ID NO: 14 under high stringency conditions and encodes a protein having a nature substantially the same in quality to the nature of the protein of the invention.

The DNA capable of hybridizing to the nucleotide sequence represented by SEQ ID NO: 2, SEQ ID NO: 4, SEQ ID NO: 6, SEQ ID NO: 8, SEQ ID NO: 10, SEQ ID NO: 12 or SEQ ID NO: 14 is as defined above.

Methods of hybridization and high stringency conditions are as described above.

The cloning of a DNA encoding the full length of the protein or the partial peptide used in the invention (hereinafter, in the explanation of the cloning and expression of DNAs encoding these, optionally the protein and the partial peptide may be briefly referred to as the protein of the invention) can be performed either by PCR amplification from genomic DNA or cDNA using synthetic DNA primers each having a partial nucleotide sequence of the protein of the invention, or by a method where a DNA fragment of interest is selected by hybridization of DNA incorporated into an appropriate vector to a labeled DNA probe, the DNA probe being a DNA fragment or a synthetic DNA encoding a part or full length of the protein of the invention. The hybridization can be carried out, for example, according to the method described in "Molecular Cloning", 2nd Edition (J. Sambrook et al., Cold Spring Harbor Lab. Press, 1989). When commercial libraries are used, the hybridization can be carried out according to the instructions attached thereto.

Substitution of the nucleotide sequence of a DNA can be performed by known methods such as ODA-LA PCR, the gapped duplex method, the Kunkel method or modifications thereof, using PCR, known kits such as Mutant^{TM}-Super Express Km (Takara), Mutan™-K (Takara), etc.

The cloned DNA encoding the protein of the invention may be used as it is or after digestion with restriction enzymes or addition of linkers, depending on purposes. The DNA may have ATG at its 5' end as a translation initiation codon and TAA, TGA, or TAG at its 3' end as a translation termination codon. The translation initiation and termination codons may also be added by using appropriate synthetic DNA adapters.

Expression vectors for the protein of the invention can be prepared by, for example, (a) cutting out a DNA fragment of interest from a DNA encoding the protein of the invention and (b) ligating the DNA fragment to an appropriate expression vector downstream of its promoter.

Examples of vectors useful in the invention include plasmids derived from *Escherichia coli* (e.g. pBR322, pBR325, pUC12, and pUC13); plasmids derived from *Bacillus subtilis* (e.g. pUB110, pTP5 and pC194); plasmids derived from yeast (e.g. pSH19 and pSH15); bacteriophages such as λ -phage; animal viruses such as retrovirus, vaccinia virus, baculovirus; and other vectors such as pA1-11, pXT1, pRc/CMV, pRc/RSV, pcDNAI/Neo and so on.

Any promoter may be used in the invention as long as it is appropriate for the host that will be used for expressing a gene of interest. When the host is an animal cell, examples of promoters useful in the invention include SR α promoter, SV40 promoter, LTR promoter, CMV promoter and HSV-TK promoter.

Among these promoters, CMV (cytomegalovirus) promoter, SR α promoter or the like is preferably used. When the host is an *Escherichia* bacterium, trp promoter, lac promoter, recA promoter, λ P_{L} promoter, lpp promoter, T7 promoter or the like is preferably used. When the host is a *Bacillus* bacterium, SPO1 promoter, SPO2 promoter, penP promoter or the like is preferably used. When the host is a yeast, PHO5 promoter, PGK promoter, GAP promoter, ADH promoter, or the like is preferably used. When the host is an insect cell, polyhedrin promoter, P10 promoter or the like is preferably used.

The expression vectors may, if desired, further comprise enhancers, splicing signals, polyadenylation signals, selective markers, SV40 replication origin (hereinafter, optionally abbreviated to "SV40 ori") and the like. Examples of selective markers useful in the invention include dihydrofolate reductase (hereinafter, optionally abbreviated to "dhfr") gene [methotorexate (MTX) resistance], ampicillin resistance gene (hereinafter, optionally abbreviated to "Amp^{r}"), neomycin resistance gene [hereinafter, optionally abbreviated to "Neo^{r}": G418 resistance] and the like. When dhfr gene-deficient Chinese hamster cells are used in combination with dhfr gene as a selective marker, a gene of interest may be selected even in a thymidine-free medium.

Furthermore, a signal sequence appropriate for the host may be added, if necessary, to the N-terminal of the protein of the invention. When the host is an *Escherichia* bacterium, the utilizable signal sequences may include PhoA signal sequence, OmpA signal sequence or the like may be added. When the host is a *Bacillus* bacterium, α-amylase signal sequence, subtilisin signal sequence, or the like may be added. When the host is yeast, MF α signal sequence, SUC2 signal sequence or the like may be added. When the host is an animal cell, insulin signal sequence, α-interferon signal sequence, antibody molecule signal sequence, or the like may be used.

Using the thus constructed vector comprising a DNA encoding the protein of the invention, transformants can be prepared.

Examples of hosts useful for this purpose include bacteria belonging to the genus *Escherichia,* bacteria belonging to the genus *Bacillus,* yeasts, insect cells, insects, and animal cells.

Specific examples of bacteria belonging to the genus *Escherichia* useful in the invention include *E*. *coli* K12 DH1 [Proc. Natl. Acad. Sci. USA, Vol. 60, 160 (1968)], JM103 [Nucleic Acids Research, Vol. 9, 309 (1981)], JA221 [Journal of Molecular Biology, Vol. 120, 517 (1978)]), HB101 [Journal of Molecular Biology, Vol, 41, 459 (1969)] and C600 [Genetics, Vol. 39,440 (1954)].

Specific examples of bacteria belonging to the genus *Bacillus* useful in the invention include *B. subtilis* MI114 [Gene, Vol. 24, 255 (1983)] and 207-21 [Journal of Biochemistry, Vol. 95, 87 (1984)].

Specific examples of yeasts useful in the invention include *Saccharomyces cerevisiae* AH22, AH22R⁻, NA87-11A, DKD-5D and 20B-12, *Schizosaccharomyces pombe* NCYC 1913 and NCYC2036, and *Pichia pastoris* KM71.

Specific examples of insect cells useful in the invention include, when the virus used is AcNPV, a cell line derived from larvae of *Spodoprera frugiperda* (Sf cells), MG1 cells derived from the midgut of *Trichoplusia ni,* High Five™ cells derived from eggs of *Trichoplusia ni, Mamestra brassicae*-derived cells and *Estigmena acrea*-derived cells. When the virus used is BmNPV, insect cells such as a silkworm-derived cell line (Bombyx mori N cells; BmN cells) may be used. Specific examples of Sf cells useful in the invention include Sf9 cells (ATCC CRL 1711) and Sf21 cells [both disclosed in Vaughn J. L. et al., In Vivo, 13, 213-217 (1977)].

Specific examples of insects useful in the invention include larvae of silkworm (Maeda et al., Nature, 315, 592 (1985)).

Specific examples of animal cells useful in the invention include simian cell COS-7, Vero cells, Chinese hamster cell CHO (hereinafter, abbreviated to "CHO cells"), sdhfr gene-deficient Chinese hamster cell CHO (hereinafter, abbreviated to "CHO(dhfr) cells"), mouse L cells, mouse AtT-20 cells, mouse myeloma cells, rat GH3 cells, and human FL cells.

Transformation of bacteria belonging to the genus *Escherichia* can be performed in accordance with methods disclosed, for example, in Proc. Natl. Acad. Sci. USA, Vol. 69, 2110 (1972) and Gene, Vol. 17, 107 (1982).

Transformation of bacteria belonging to the genus *Bacillus* can be performed in accordance with methods disclosed, for example, in Molecular & General Genetics, Vol. 168, 111 (1979).

Transformation of yeasts can be performed in accordance with methods disclosed, for example, in Methods in Enzymology, 194, 182-187(1991) and Proc. Natl. Acad. Sci. USA, Vo. 75, 1929 (1978).

Transformation of insect cells or insects can be performed in accordance with methods disclosed, for example, in Bio/Technology, 6, 47-55 (1988).

Transformation of animal cells can be performed by methods disclosed, for example, in Cell Engineering, Separate Vol. 8, New Cell Engineering Experiment Protocol, 263-267 (1995) (Shujunsha Co.) and Virology, Vol. 52, 456 (1973).

Thus, transformants transformed with the expression vector comprising a DNA encoding the protein can be obtained.

As a medium to culture transformants obtained from *Escherichia* or *Bacillus* bacteria as hosts, a liquid medium is appropriate. The medium may contain carbon sources, nitrogen sources, minerals, and so on which are necessary for the growth of the transformant. As carbon sources, glucose, dextrin, soluble starch, sucrose or the like may be enumerated. As nitrogen sources, organic or inorganic substances such as ammonium salts, nitrates, corn steep liquor, peptone, casein, meat extract, bean cake, potato extract, or the like may be enumerated. As minerals, calcium chloride, sodium dihydrogen phosphate, magnesium chloride, or the like may be enumerated. Further, yeast, vitamins, growth-promoting factors, etc. may also be added to the medium. Preferable pH of the medium is about 5-8.

As a medium to culture *Escherichia* bacteria, M9 medium containing glucose and casamino acid [Miller, Journal of Experiments in Molecular Genetics, 431-433, Cold Spring Harbor Laboratory, New York, (1972)] is preferable, for example. If necessary, drugs such as 3 β-indolyl acrylic acid can be added to the medium to improve the efficiency of the promoter.

When the host is an *Escherichia* bacterium, the transformant is cultured usually at about 15-43°C for about 3-24 hours. If necessary, aeration and stirring may be applied.

When the host is a *Bacillus* bacterium, the transformant is cultured usually at about 30-40°C for about 6-24 hours. If necessary, aeration and stirring may also be applied.

As a medium to culture transformants obtained from yeasts as hosts, a medium such as Burkholder minimum medium [Bostian, K.L. et al., Proc. Natl. Acad. Sci. USA, Vol. 77, 4505 (1980)] or SD medium containing 0.5% casamino acid [Bitter, G.A. et al., Proc. Natl. Acad. Sci. USA, Vol. 81, 5330 (1984)] may be used, for example. It is preferable that the pH of the medium be adjusted to about 5-8. The transformant is cultured usually at about 20-35°C for about 24-72 hours. If necessary, aeration and stirring may be applied.

As a medium to culture transformants obtained from insect cells or insects as hosts, Grace's Insect Medium [Grace, T.C.C., Nature, 195, 788 (1962)] supplemented with additives such as inactivated 10% bovine serum may be used, for example. It is preferable that the pH of the medium be adjusted to about 6.2-6.4. The transformant is cultured usually at about 27°C for about 3-5 days. If necessary, aeration and stirring may be applied.

As a medium to culture transformants obtained from animal cells as hosts, examples of useful media include MEM medium [Science, Vol. 122, 501 (1952)], DMEM medium [Virology, Vol. 8, 396 (1959)], RPMI 1640 medium [Journal of the American Medical Association, Vol. 199, 519 (1967)] and 199 medium [Proceedings of the Society of the Biological Medicine, Vol. 73, 1 (1950)] each containing about 5-20% fetal calf serum. Preferable pH of the medium is from about 6 to about 8. The transformant is cultured usually at about 30-40°C for about 15-60 hours. If necessary, aeration and stirring may be applied.

Thus, it is possible to allow the transformant to produce the protein of the invention in its cells, cell membranes, or outside the cells.

Separation and purification of the protein of the invention from the resultant culture can be carried out, for example, according to the methods described below.

For extraction of the protein of the invention from cultured microorganisms or cells, the microorganism cells are harvested by known methods after the cultivation, suspended in a suitable buffer, and disrupted by sonication or by lysozyme and/or freezing and thawing, etc. Then, a crude extract of the protein extract is obtained by centrifugation or filtration. The buffer may contain a protein denaturing agent such as urea or guanidine hydrochloride, or a surfactant such as Triton X-100™. If the protein is secreted into the culture broth, the supernatant is separated from the microorganisms or cells after completion of the cultivation and collected by known methods.

Purification of the protein of the invention contained in the thus obtained culture supernatant or extract can be performed by an appropriate combination of known methods for separation and purification. These known methods include methods utilizing solubility (such as salting out or sedimentation with solvents), methods mainly utilizing difference in molecular weight (such as dialysis, ultrafiltration, gel filtration and SDS-polyacrylamide gel electrophoresis), methods utilizing difference in electric charge (such as ion-exchange chromatography), methods utilizing specific affinity (such as affinity chromatography), methods utilizing difference in the hydrophobicity (such as reversed-phase high-performance liquid chromatography), and methods utilizing difference in isoelectric point (such as isoelectric electrophoresis).

When the thus obtained protein of the invention is a free protein, it can be converted into the above-described salt by known methods or modifications thereof. On the contrary, when the protein of interest is obtained in a salt form, the salt can be converted into a free form or another salt according to known methods or modifications thereof.

The protein produced by the transformant can be arbitrarily modified or a polypeptide can be removed therefrom by using an appropriate protein modification enzyme before or after the purification. Examples of such protein modification enzymes include trypsin, chymotrypsin, arginyl endopeptidase, protein kinase and glycosidase.

The presence of the thus produced protein of the invention can be measured by enzyme immunoassays using specific antibodies, Western blotting, and so on.

The antibody against the protein, partial peptides thereof, or salts of the protein or the partial peptide used in the invention may be either a polyclonal antibody or monoclonal antibody as long as it is capable of recognizing the protein, partial peptides thereof, or salts of the protein or the partial peptide used in the invention.

The antibody against the protein, partial peptides thereof, or salts of the protein or the partial peptide used in the invention (hereinafter, in the description of antibodies, these are briefly referred to as the protein of the invention) can be prepared in accordance with known procedures for preparing antibodies or antisera using the protein of the invention as an antigen.

### [Preparation of Monoclonal Antibodies]

### (a) Preparation of Monoclonal Antibody-Producing Cells

The protein of the invention is administered to warm-blooded animals either alone or together with a carrier or diluent to a site capable of producing antibodies upon the administration. In order to enhance the ability to produce antibodies, complete Freund's adjuvants or incomplete Freund's adjuvants may also be administered. The administration is usually carried out once in every two to six weeks and two to ten times in the total. Examples of warm-blooded animals useful in the invention include monkey, rabbit, dog, guinea pig, mouse, rat, sheep, goat and chicken. Among them, mouse or rat is used preferably.

In the preparation of monoclonal antibody-producing cells, individuals with detectable antibody titers are selected from warm-blooded animals (e.g. mice) immunized with antigen. Then, the spleen or lymph nodes are collected from them two to five days after the final immunization, and antibody-producing cells contained therein are fused with myeloma cells of a homologous or heterologous animal to thereby obtain monoclonal antibody-producing hybridomas. Measurement of antibody titers in antisera may be carried out, for example, by reacting a labeled protein (which will be described later) with the antiserum, followed by measuring the activity of the labeling agent bound to the antibody. The cell fusion may be carried out by a known method, for example, the method of Koehler and Milstein (Nature, 256, 495, (1975)). Examples of useful fusion promoters include polyethylene glycol (PEG), Sendai virus, etc. Preferably, PEG is used.

Examples of myeloma cells useful in the invention include myeloma cells of warm-blooded animals such as NS-1, P3U1, SP2/0, AP-1, etc. Preferably, P3U1 is used. A preferable ratio of the number of antibody-producing cells used (spleen cells) to the number of myeloma cells is from about 1:1 to about 20:1. When PEG (preferably, PEG 1000 to PEG 6000) is added at a concentration of about 10-80% and the resultant cell mixture is incubated at 20-40°C (preferably, at 30-37°C) for about 1-10 minutes, an efficient cell fusion can be achieved.

Various methods may be used for screening for monoclonal antibody-producing hybridomas. For example, hybridoma culture supernatant is added to a solid phase (e.g. microplate) on which the protein antigen has been adsorbed either directly or with a carrier. Then, a radioactively or enzymatically labeled anti-immunoglobulin antibody (anti-mouse immunoglobulin antibody is used when mouse cells are used in the cell fusion) or protein A is added thereto to detect monoclonal antibodies bound to the solid phase. Alternatively, a method may be used in which hybridoma culture supernatant is added to a solid phase on which an anti-immunoglobulin antibody or protein A has been adsorbed; then, a radioactively or enzymatically labeled protein is added thereto to thereby detect monoclonal antibodies bound to the solid phase.

Selection of monoclonal antibodies may be carried out by known methods or methods based on them. Usually, selection can be carried out in a medium for culturing animal cells supplemented with HAT (hypoxanthine, aminopterin and thymidine). As a medium for selection and culturing, any medium may be used as long as hybridomas are capable of growing therein. Examples of useful media include RPMI 1640 medium containing about 1-20% (preferably about 10-20%) of fetal calf serum, GIT medium (Wako Pure Chemical Industries, Ltd.) containing about 1-20% of fetal calf serum and a serum-free medium for hybridoma cultivation (SFM-101; Nissui Pharmaceutical Co.). The cultivation temperature is usually about 20-40°C, preferably about 37°C. The cultivation period is usually from five days to three weeks, preferably one to two weeks. The cultivation may be carried out usually under 5% carbon dioxide. The antibody titer of hybridoma culture supernatant may be measured in the same manner as in the above-mentioned measurement of the antibody titers in antisera.

### (b) Purification of the Monoclonal Antibodies

Separation and purification of monoclonal antibodies may be carried out by conventional methods, such as methods for separating/purifying immunoglobulin [e.g. salting-out, alcohol precipitation, isoelectric precipitation, electrophoresis, adsorption/desorption using ion exchangers (e.g. DEAE), ultracentrifugation, gel filtration, specific purification methods in which only an antibody is collected by means of an antigen-binding solid phase or active adsorbent such as protein A or protein G, followed by dissociation of the bond].

### [Preparation of Polyclonal Antibodies]

The polyclonal antibody of the invention can be produced by known methods or methods based on them. For example, an immunogen (protein antigen) *per se* or a complex of the immunogen and a carrier protein is prepared. Then, using the immunogen or the complex, warm-blooded animals are immunized in the same manner as described for the production of monoclonal antibodies. Fractions containing the antibody against the protein of the invention are harvested from the immunized animals, followed by separation and purification of the antibody.

With respect to the immunogen-carrier protein conjugate for use in the immunization of warm-blooded animals, the kind of carrier protein and the mixing ratio of the carrier and the hapten are not particularly restricted as long as antibodies are produced efficiently against the hapten cross-linked to the carrier. For example, bovine serum albumin, bovine thyroglobulin, hemocyanin, or the like is coupled to the hapten at a weight ratio of about 0.1-20:1, preferably about 1-5:1.

A variety of condensing agents can be used for the coupling between the hapten and the carrier. For example, glutaraldehyde, carbodiimide, maleimide, or active ester reagents containing a thiol or dithiopyridyl group may be used.

The condensation product is administered to a warm-blooded animal either alone or together with a carrier or diluent at a site capable of producing antibodies upon the administration. In order to enhance the antibody production ability, complete Freund's adjuvant or incomplete Freund's adjuvant may also be administered. Administration is carried out generally once in about every 2-6 weeks and about 3-10 times in the total.

Polyclonal antibodies can be recovered from the blood, abdominal dropsy or other body fluid, preferably from the blood, of the warm-blooded animal immunized as described above.

Polyclonal antibody titers in antisera can be determined in the same manner as described above for the determination of monoclonal antibody titers in antisera. The separation and purification of polyclonal antibodies can be carried by the same methods for separation and purification of immunoglobulin as those described for the separation and purification of monoclonal antibodies.

With respect to the antisense polynucleotide having a nucleotide sequence complementary to or substantially complementary to the DNA encoding the protein or the partial peptide used in the invention (hereinafter, these DNAs may be briefly referred to as the "DNA of the invention"), any antisense polynucleotide may be used as long as it has a nucleotide sequence complementary to or substantially complementary to the nucleotide sequence of the DNA of the invention and has an effect capable of inhibiting the expression of this DNA. Antisense DNA is preferably.

A nucleotide sequence substantially complementary to the DNA of the invention refers to, for example, a nucleotide sequence having about 70% or more, preferably about 80% or more, more preferably about 90% or more, most preferably about 95% or more homology to the full-length or a partial nucleotide sequence of the complementary nucleotide sequence of the DNA of the invention (i.e., the complementary strand of the DNA of the invention). Particularly preferable is (1) an antisense polynucleotide having about 70% or more, preferably about 80% or more, more preferably about 90% or more, most preferably about 95% or more homology to a part of the complementary strand of the DNA of the invention encoding an N-terminal region of the protein of the invention (e.g. nucleotide sequence encoding a region neighboring the initiation codon) when the antisense polynucleotide aims at inhibition of translation, or (2) an antisense polynucleotide having about 70% or more, preferably about 80% or more, more preferably about 90% or more, most preferably about 95% or more homology to the full-length of the complementary strand of the DNA of the invention including introns when the antisense polynucleotide aims at degradation of the RNA by RNaseH.

Specifically, antisense polynucleotides having a nucleotide sequence, or a part thereof, complementary to or substantially complementary to the nucleotide sequence of a DNA comprising the nucleotide sequence represented by SEQ ID NO: 2, SEQ ID NO: 4, SEQ ID NO: 6, SEQ ID NO: 8, SEQ ID NO: 10, SEQ ID NO: 12 or SEQ ID NO: 14 may be enumerated. Preferablbly, antisense polynucleotides having a nucleotide sequence, or a part thereof, complementary to the nucleotide sequence of a DNA comprising the nucleotide sequence represented by SEQ ID NO: 2, SEQ ID NO: 4, SEQ ID NO: 6, SEQ ID NO: 8, SEQ ID NO: 10, SEQ ID NO: 12 or SEQ ID NO: 14 (more preferably, antisense polynucleotides having a nucleotide sequence, or a part thereof, complementary to the nucleotide sequence of a DNA comprising the nucleotide sequence represented by SEQ ID NO: 2, SEQ ID NO: 4, SEQ ID NO: 6, SEQ ID NO: 8, SEQ ID NO: 10, SEQ ID NO: 12 or SEQ ID NO: 14) may be enumerated.

Usually, the antisense polynucleotide consists of about 10-40 nucleotides, preferably about 15-30 nucleotides.

In order to prevent the degradation by hydrolase such as nuclease, the phosphate group of individual nucleotides constituting the antisense polynucleotide may be substituted with chemically modified phosphate groups such as phosphorothioate, methylphosphonate, phosphorodithionate, or the like. Further, the sugar (deoxyribose) of individual nucleotides may be substituted with a chemically modified structure such as 2'-O-methylated; or the base (pyrimidine or purine) may be chemically modified. Any antisense polynucleotide may be used as long as it is capable of hybridizing to a DNA comprising the nucleotide sequence represented by SEQ ID NO: 2, SEQ ID NO: 4, SEQ ID NO: 6, SEQ ID NO: 8, SEQ ID NO: 10, SEQ ID NO: 12 or SEQ ID NO: 14. These antisense polynucleotides can be synthesized with known DNA synthesizers.

According to the present invention, antisense polynucleotides (nucleic acids) capable of inhibiting the replication or expression of the gene encoding the protein of the invention can be designed and synthesized based on information on the nucleotide sequence of cloned or sequenced DNA encoding the protein. Such an antisense polynucleotide (nucleic acid) is capable of hybridizing to the RNA of the protein gene of the invention to thereby inhibit the synthesis or function of the RNA, or capable of modulating/controlling the expression of the protein gene of the invention through interaction with those RNAs related to the protein of the invention. Polynucleotides complementary to selected sequences of RNAs related to the protein of the invention and polynucleotides specifically hybridizable to RNAs related to the protein of the invention are useful in regulating/controlling *in vivo* and *in vitro* the expression of the protein gene of the invention and also useful in treating or diagnosing diseases.

The relationship between the target nucleic acid and a polynucleotide complementary to at least a portion of the target region (i.e., a polynucleotide hybridizable to the target) can be said "antisense". Antisense polynucleotides may be polynucleotides containing 2-deoxy-D-ribose, polynucleotides containing D-ribose, any other type of polynucleotide which is an N-glycoside of purine or pyrimidine base, or other polymers with a non-nucleotide backbone (e.g., commercially available protein nucleic acids and synthetic sequence-specific nucleic acid polymers), or other polymers with special linkages (provided that these polymers contain nucleotides in a configuration which allows for base pairing and base stacking such as found in DNA and RNA). These antisense polynucleotides may be double-stranded DNAs, single-stranded DNAs, double-stranded RNAs, single-stranded RNAs and DNA:RNA hybrids. Further, they may be unmodified polynucleotides (or unmodified oligonucleotides). Further, they may have known types of modifications, for example, labels which are known to those skilled in the art, or "caps"; or they may be methylated; or they may have substitution of one or more of the naturally occurring nucleotides with analogues; or they may have intramolecular nucleotide modifications such as, for example, those with uncharged linkages (e.g., methyl phosphonate, phosphotriester, phosphoramidate, carbamate, etc.) and with charged linkages or sulfur-containing linkages (e.g., phosphorothioate, phosphorodithioate, etc.), those containing pendant moieties, such as, for example, proteins (including nucleases, nuclease inhibitors, toxins, antibodies, signal peptides, poly-L-lysine, etc.) and saccharides (e.g., monosaccharide, etc.), those with intercalate compounds (e.g., acridine, psoralen, etc.), those containing chelate compounds (e.g., metals, radioactive metals, boron, oxidative metals, etc.), those containing alkylators, or those with modified linkages (e.g., α anomeric nucleic acids, etc.). The terms "nucleoside", "nucleotide" and "nucleic acid" used herein will include those which contain not only the known purine and pyrimidine bases, but also other heterocyclic bases that have been modified. Such modifications include methylated purines and pyrimidines, acylated purines and pyrimidines, or other heterocycles. Modified nucleosides or nucleotides will also include modifications on the sugar moiety, e.g., wherein one or more of the hydroxyl groups are replaced with halogen, aliphatic groups, or functional groups such as ethers, amines or the like.

The antisense polynucleotide (nucleic acid) of the invention is RNA, DNA or a modified nucleic acid (RNA or DNA). Examples of modified nucleic acid include, but are not limited to, degradation-resistant sulfurized and thiophosphate derivatives of nucleic acids, and poly- or oligo-nucleoside amides. The antisense polynucleotide of the invention may be designed under the following strategies: to increase the intracellular stability of the nucleic acid; to increase the cellular permeability of the nucleic acid; to increase the affinity of the nucleic acid for the target sense strand; and to decrease the toxicity (if any) of the nucleic acid.

Such modifications are known to those skilled in the art, as described, for example, in J. Kawakami et al., Pharm Tech Japan, Vol. 8, p. 247, 1992; Vol. 8, p. 395, 1992; S. T. Crooke et al. ed., Antisense Research and Applications, CRC Press, 1993.

The antisense nucleic acid of the invention may contain altered or modified sugars, bases or linkages, may be supplied in specialized forms such as liposomes, microspheres, etc. or may be applied by gene therapy, or may be supplied as addition forms. Examples of such addition include polycationic moieties such as polylysine that act as charge neutralizers for the phosphate backbone, or hydrophobic moieties such as lipids (e.g., phospholipids, cholesterols, etc.) that enhance interaction with cell membranes or increase uptake of the nucleic acid. Preferred lipids that may be added are cholesterols or derivatives thereof (e.g., cholesteryl chloroformate, cholic acid, etc.). These moieties may be attached to the 3' or 5' end of the nucleic acids, and may be attached through a base, sugar, or intracellular nucleoside linkage. Other groups include capping groups specifically placed at the 3' or 5' end of the nucleic acid to prevent degradation by nuclease such as exonuclease, RNase, etc. Such capping groups include, but are not limited to, hydroxyl protecting groups known to those skilled in the art, including glycols such as polyethylene glycols, tetraethylene glycol and the like.

The inhibitory activity of the antisense nucleic acid can be examined using the transformant of the invention, the *in vitro* and *in vivo* gene expression system of the invention, or the *in vitro* and *in vivo* translation system of the protein of the invention. The nucleic acid can be applied to cells according to various methods known in the art.

Hereinbelow, uses of the protein, partial peptides thereof or salts of the protein or partial peptides of the invention (hereinafter, optionally they are briefly referred to as "the protein of the invention"); uses of DNAs encoding the protein or the partial peptide of the invention (hereinafter, optionally briefly referred to as "the DNA of the invention"); uses of antibodies to the protein, partial peptides thereof or salts of the protein or partial peptides of the invention (hereinafter, optionally referred to as the "antibody of the invention"); and uses of antisense polynucleotides to the DNA of the invention (hereinafter, optionally referred to as the "antisense polynucleotide of the invention") will be described.

Since the expression of the protein of the invention increases in cartilage in osteoarthosis, the protein may be used as a disease marker. Specifically, it is useful as a marker for early diagnosis in cartilage in osteoarthosis, for judging severity of symptoms, and for predicting the progress of the disease. Therefore, medicines comprising an antisense polynucleotide to the gene encoding the protein of the invention, a compound or a salt thereof that inhibits the activity of the protein of the invention, or an antibody against the protein of the invention may be used as prophylactic and/or therapeutic agents for bone and/or arthropathy diseases such as chondrodystrophy, osteodystrophy, osteoporosis, osteoarthosis, rheumatoid arthritis, arthritis, synovitis, metabolic arthropathy or arthropathy disorder caused by sports (e.g. tennis elbow).

### (1) Screening for Drug Candidate Compounds for Diseases

Since the expression of the protein of the invention increases in cartilage in osteoarthosis, a compound or a salt thereof that regulates the activity of the protein of the invention (preferably, a compound or a salt thereof that inhibits the activity of the protein of the invention may be used as a prophylactic and/or therapeutic agent for, e.g., bone and/or arthropathy diseases such as chondrodystrophy, osteodystrophy, osteoporosis, osteoarthosis, rheumatoid arthritis, arthritis, synovitis, metabolic arthropathy or arthropathy disorder caused by sports (e.g. tennis elbow).

Therefore, the protein of the invention is useful as a reagent for screening for compounds or salts thereof that regulate the activity of the protein of the invention.

### (A) Method of Screening for Compounds or Salts thereof that Regulate the Activity of the Protein A of the Invention (e.g. 5'-Deiodinase Activity) Using the Protein A of the Invention

Specifically, for example, a method of screening for a compound or a salt thereof that regulates (promotes or inhibits) the activity of the protein A of the invention characterized by the following procedures may be used: briefly, in the presence or absence of a test compound, (1) T4 or rT3 is added to the protein A of the invention, followed by measurement and comparison of the individual products or (2) ¹²⁵I-labeled T4 or rT3 is added to the protein A of the invention, followed by detection and comparison of the liberated ¹²⁵I in each case.

Further, a method of screening for a compound or a salt thereof that regulates (promotes or inhibits) the activity of the protein A of the invention characterized by the following procedures may be used: briefly, (1) the amounts of the product produced are measured and compared between case (i) where cells capable of expressing the protein A of the invention are cultured in contact with T4 or rT3 and case (ii) where cells capable of expressing the protein A of the invention are cultured in contact with T4 or rT3 and a test compound; or (2) the liberated ¹²⁵I is detected and compared between case (i) where cells capable of expressing the protein A of the invention are cultured in contact with ¹²⁵I-labeled T4 or ¹²⁵I-labeled rT3 and case (ii) where cells capable of expressing the protein A of the invention are cultured in contact with ¹²⁵I-labeled T4 or ¹²⁵I-labeled rT3 and a test compound.

T4 or rT3 may be mixed with a test compound and then added to cells capable of producing the protein A of the invention; or T4 or rT3 may be added to cells capable of producing the protein A of the invention and then a test compound may be added thereto. ¹²⁵I-labeled T4 or ¹²⁵I-labeled rT3 may be handled in the same manner.

As the cell capable of producing the protein A of the invention, the afore-mentioned host (transformant) transformed with a vector comprising a DNA encoding the protein of the invention may be used, for example. As the host, animal cells such as CHO cells may be used preferably. In the above-described screening, a transformant which has been cultured by the afore-mentioned method so that the protein A of the invention is expressed within its cells is used preferably. The method of culturing the cells capable of expressing the protein A of the invention is the same as the afore-mentioned method for culturing the transformant of the invention.

The test compound may be, for example, a peptide, protein, non-peptidic compound, synthetic compound, fermentation product, cell extract, plant extract, or animal tissue extract.

For example, a test compound which increases the enzyme activity in case (ii) above by about 20% or more, preferably by about 30% or more, more preferably by about 50% or more compared to the enzyme activity in case (i) above may be selected as a compound that promotes the activity of the protein A of the invention; and a test compound which decreases the enzyme activity in case (ii) above by about 20% or more, preferably by about 30% or more, more preferably by about 50% or more compared to the enzyme activity in case (i) above may be selected as a compound that inhibits the activity of the protein A of the invention.

### (B) Method of Screening for Compounds or Salts thereof that Regulate the Activity of the Protein B of the Invention (e.g. Pyrophosphate Transport Activity) Using the Protein B of the Invention

Specifically, for example, a method of screening for a compound or a salt thereof that regulates (promotes or inhibits) the activity of the protein B of the invention may be used which is characterized by comparing (i) pyrophosphate transport in cells capable of producing the protein B of the invention and (ii) pyrophosphate transport in a mixture of cells capable of producing the protein B of the invention and a test compound.

In the above-described screening method, pyrophosphate transport activities in cases (i) and (ii) are measured by known methods.

Specifically, in the absence or presence of a test compound, cells capable of producing the protein B of the invention are cultured. The culture broth is recovered and reacted with a mixture of inorganic pyrophosphatase, maltose, maltose phosphorylase, glucose oxidase, peroxidase and 10-acetyl-3,7-dihydroxyphenoxazine (e.g. Piper Pyrophosphate Assay Kit; Funakoshi) to thereby measure and compare the pyrophosphate in the culture broth as a fluorescent product resorfin; thus, pyrophosphate transport activities can be measured. Alternatively, cells are recovered after the cultivation, treated appropriately, and then reacted with the above-described mixture to thereby measure and compare the pyrophosphate in the solution as a fluorescent product resorfin; thus, pyrophosphate transport activities can be measured.

As the cell capable of producing the protein B of the invention, the afore-mentioned host (transformant) transformed with a vector comprising a DNA encoding the protein of the invention may be used, for example. As the host, animal cells such as mouse ATDC5 cells or CHO cells may be used preferably. In the above-described screening, a transformant which has been cultured by the afore-mentioned method so that the protein B of the invention is expressed on its cell membranes is used preferably. The method of culturing the cells capable of expressing the protein B of the invention is the same as the afore-mentioned method for culturing the transformant of the invention.

The test compound may be, for example, a peptide, protein, non-peptidic compound, synthetic compound, fermentation product, cell extract, plant extract, or animal tissue extract.

For example, a test compound which increases the pyrophosphate transport activity in case (ii) above by about 20% or more, preferably by about 30% or more, more preferably by about 50% or more compared to the pyrophosphate transport activity in case (i) above may be selected as a compound that promotes the activity of the protein B of the invention; and a test compound which decreases the pyrophosphate transport activity in case (ii) above by about 20% or more, preferably by about 30% or more, more preferably by about 50% or more compared to the pyrophosphate transport activity in case (i) above may be selected as a compound that inhibits the activity of the protein B of the invention.

### (C) Method of Screening for Compounds or Salts thereof that Regulate the Activity of the Protein C of the Invention (e.g. Cell Differentiation-Regulating Activity such as Cartilage Cell Differentiation-Regulating Activity, Osteoblast Differentiation-Regulating Activity) Using the Protein C of the Invention

Specifically, for example, a method of screening for a compound or a salt thereof that regulates (promotes or inhibits) the activity of the protein C of the invention may be used which is characterized by comparing (i) cell differentiation-regulating activity in cells capable of producing the protein C of the invention and (ii) cell differentiation-regulating activity in a mixture of cells capable of producing the protein C of the invention and a test compound.

In the above-described screening method, cell differentiation-regulating activities in cases (i) and (ii) are measured by known methods.

As known methods, the methods described, for example, in Journal of Bone and Mineral Research 11:105-113, 1996; The Journal of Cell. Biology 154: 659-666, 2001 or modifications thereof may be used.

Specifically, a plasmid carrying a cDNA encoding the protein C of the invention (e.g. SHOX2) is introduced into mouse ATDC5 cells to prepare a cell strain which expresses the protein C of the invention (e.g. SHOX2) stably. The resultant mouse ATDC5 cells expressing the protein C of the invention (e.g. SHOX2) are cultured with or without a growth factor (e.g. BMP-2, BMP-4, insulin, TGF-β, FGF-1, FGF-2, etc.; among all, BMP-2, BMP-4 and insulin are preferable) in the absence or presence of a test compound to thereby induce the differentiation of these cells into cartilage. After the induction, cell differentiation-regulating activity is measured by any of the following procedures and compared: (1) the expression of X type collagen is detected by RT-PCR; (2) alkaline phosphatase activity is measured using TRACP & ALP double-stain kit (Takara Shuzo) or the like; (3) calcium deposited in the cell layer is detected by alizarin red staining or the like; or (4) the cell layer is labeled with ⁴⁵Ca²⁺ and the deposited calcium is determined. If necessary, an inducible promoter is used for the expression of the protein C of the invention (e.g. SHOX2). Alternatively, gene transfer may be performed after the early differentiation of cartilage. It is desirable to measure also the cell differentiation-regulating activity of the parent strain ATDC5 cells.

Briefly, a gene for the protein C of the invention (e.g. SHOX2) is transferred into, for example, MC3T3-E1 cells, which are then cultured in the absence or presence of a test compound. Subsequently, (1) the expression of a cell differentiation marker gene (e.g. type I collagen, osteopontin, osteocalcin, etc.) is detected by RT-PCT; (2) alkaline phosphatase activity is measured using TRACP & ALP double-stain kit (Takara Shuzo) or the like; (3) calcium deposited in the cell layer is detected by alizarin red staining or the like; or (4) the cell layer is labeled with ⁴⁵Ca²⁺ and the deposited calcium is determined. Thus, cell differentiation-regulating activity is measured and compared. If necessary, an inducible promoter is used for the expression of the protein C of the invention (e.g. SHOX2). It is desirable to measure also the cell differentiation-regulating activity of the parent strain MC3T3-E1 cells.

The measurement of cell differentiation-regulating activity may be carried out by known methods, e.g. the method described in J. Biol. Chem. 275: 1065-72, 2000 or a modification thereof. Specifically, a reporter plasmid in which the expression control region of a cell differentiation marker gene (e.g. osteopontin, type I collagen, type II collagen, type X collagen, etc.) is linked to luciferase gene and an expression plasmid for the protein C of the invention (e.g. SHOX2) are introduced into mouse 3H10T1/2 cells. The cells are cultured in the absence or presence of a test compound. Then, luciferase activity is measured to thereby determine and compare cell differentiation-regulating activity.

As the cell capable of producing the protein C of the invention, the afore-mentioned host (transformant) transformed with a vector comprising a DNA encoding the protein of the invention may be used, for example. As the host, animal cells such as mouse ATDC5 cells, MC3T3-E1 cells, C3H10T1/2 cells or CHO cells may be used preferably. In the above-described screening, a transformant which has been cultured by the afore-mentioned method so that the protein C of the invention is expressed within its cells is used preferably. The method of culturing the cells capable of expressing the protein C of the invention is the same as the afore-mentioned method for culturing the transformant of the invention.

The test compound may be, for example, a peptide, protein, non-peptidic compound, synthetic compound, fermentation product, cell extract, plant extract, or animal tissue extract.

For example, a test compound which increases the cell differentiation-regulating activity in case (ii) above by about 20% or more, preferably by about 30% or more, more preferably by about 50% or more compared to the cell differentiation-regulating activity in case (i) above may be selected as a compound that promotes the activity of the protein C of the invention; and a test compound which decreases the cell differentiation-regulating activity in case (ii) above by about 20% or more, preferably by about 30% or more, more preferably by about 50% or more compared to the cell differentiation-regulating activity in case (i) above may be selected as a compound that inhibits the activity of the protein C of the invention.

### (D) Method of Screening for Compounds or Salts thereof that Regulate the Activity of the Protein D of the Invention (e.g. Potassium Ion Transport Activity) Using the Protein D of the Invention

Specifically, for example, a method of screening for a compound or a salt thereof that regulates (promotes or inhibits) the activity of the protein D of the invention may be used which is characterized by comparing (i) potassium ion transport in cells capable of producing the protein D of the invention and (ii) potassium ion transport in a mixture of cells capable of producing the protein D of the invention and a test compound.

In the above-described screening method, potassium ion transport activities in cases (i) and (ii) are measured by known methods.

Specifically, potassium ion transport in cases (i) and (ii) is measured by the patch-clump technique or the like and compared as an indicator for potassium ion transport activity.

As the cell capable of producing the protein D of the invention, the afore-mentioned host (transformant) transformed with a vector comprising a DNA encoding the protein of the invention may be used, for example. As the host, animal cells such as mouse ATDC5 cells or CHO cells may be used preferably. In the above-described screening, a transformant which has been cultured by the afore-mentioned method so that the protein D of the invention is expressed on its cell membranes is used preferably. The method of culturing the cells capable of expressing the protein D of the invention is the same as the afore-mentioned method for culturing the transformant of the invention.

The test compound may be, for example, a peptide, protein, non-peptidic compound, synthetic compound, fermentation product, cell extract, plant extract, or animal tissue extract.

For example, a test compound which increases the potassium ion transport activity in case (ii) above by about 20% or more, preferably by about 30% or more, more preferably by about 50% or more compared to the potassium ion transport activity in case (i) above may be selected as a compound that promotes the activity of the protein D of the invention; and a test compound which decreases the potassium ion transport activity in case (ii) above by about 20% or more, preferably by about 30% or more, more preferably by about 50% or more compared to the potassium ion transport activity in case (i) above may be selected as a compound that inhibits the activity of the protein D of the invention.

### (E) Method of Screening for Compounds or Salts thereof that Regulate the Activity of the Protein E of the Invention (e.g. Signal Transduction Activity or Ligand-Binding Activity) Using the Protein E of the Invention

Specifically, for example, a method of screening for a compound or a salt thereof that regulates (promotes or inhibits) the activity of the protein E of the invention may be used which is characterized by comparing (i) signal transduction activity or ligand-binding activity in cells capable of producing the protein E of the invention and (ii) signal transduction activity or ligand-binding activity in a mixture of cells capable of producing the protein E of the invention and a test compound.

In the above-described screening method, signal transduction activities or ligand-binding activities in cases (i) and (ii) are measured by known methods.

Specifically, in the absence or presence of a test compound, cells expressing the protein E of the invention (e.g. EphA3) (such as ATDC5 cells that are engineered to express EphA3 highly) are (1) cultured in the presence of Ephrin/Fc chimeric protein (R&D systems) or (2) co-cultured with cells expressing a ligand for the protein E of the invention (e.g. EphA3). Then, signal transduction activity mediated by the EphA3 expressed on the cell membrane (e.g. activity to promote or inhibit autophosphorylation of EphA3, intracellular Ca²⁺ liberation, inositol phosphate production, changes in cell membrane potentials, the phosphorylation of intracellular proteins, c-fos activation, the lowering of pH, etc.) is measured by known methods.

Alternatively, in the absence or presence of a test compound, (1) a labeled ligand for the protein E of the invention (e.g. EphA3) is contacted with the protein E of the invention, and then the amount of binding of the labeled ligand to the protein is measured and compared; (2) a labeled ligand for the protein E of the invention (e.g. EphA3) is contacted with cells containing the protein E of the invention or the membrane fraction of the cells, and then the amount of binding of the labeled ligand to the cells or the cell membrane fraction is measured and compared; or (3) a labeled ligand for the protein E of the invention (e.g. EphA3) is contacted with the protein E of the invention which was expressed on cell membranes by culturing a transformant comprising a DNA encoding the protein E of the invention, and then the amount of binding of the labeled ligand to the cells or the cell membrane fraction is measured and compared.

Specific examples of the ligand for the protein E of the invention (e.g. EphA3) include proteins belonging to the ephrin A family (such as ephrin A2, ephrin A5, etc.).

As the cell capable of producing the protein E of the invention, the afore-mentioned host (transformant) transformed with a vector comprising a DNA encoding the protein of the invention may be used, for example. As the host, animal cells such as mouse ATDC5 cells or CHO cells may be used preferably. In the above-described screening, a transformant which has been cultured by the afore-mentioned method so that the protein E of the invention is expressed on its cell membranes is used preferably. The method of culturing the cells capable of expressing the protein E of the invention is the same as the afore-mentioned method for culturing the transformant of the invention.

The test compound may be, for example, a peptide, protein, non-peptidic compound, synthetic compound, fermentation product, cell extract, plant extract, or animal tissue extract.

For example, a test compound which increases the signal transduction activity or ligand-binding activity in case (ii) above by about 20% or more, preferably by about 30% or more, more preferably by about 50% or more compared to the corresponding activity in case (i) above may be selected as a compound that promotes the activity of the protein E of the invention; and a test compound which decreases the signal transduction activity or ligand-binding activity in case (ii) above by about 20% or more, preferably by about 30% or more, more preferably by about 50% or more compared to the corresponding activity in case (i) above may be selected as a compound that inhibits the activity of the protein E of the invention.

### (F) Method of Screening for Compounds or Salts thereof that Regulate the Activity of the Protein F of the Invention (e.g. Matrix Metalloproteinase Activity) Using the Protein F of the Invention

Specifically, a method of screening for a compound or a salt thereof that regulates (promotes or inhibits) the activity of the protein F of the invention is used which is characterized by adding a substrate to the protein F of the invention in the absence or presence of a test compound and then measuring and comparing the proteinase activity.

The measurement of matrix metalloproteinase activity may be carried out by known methods, e.g. the method described in J. Biol. Chem. 271: 17119-17123, 1996 or modifications thereof. Specifically, a recombinant protein of the protein F of the invention (e.g. MMP-16) which lacks the membrane-binding region and the intracellular region is created according to the method, or a modification thereof, described in Eur. J. Biochem. 262: 907-914, 1999. Then, the protein is retained at 37°C in a buffer containing a substarate (20 mM sodium tetraborate, pH 9.0, 10 mM CaCl₂, 100 mM NaCl, 0.02% NaN₃, 0.025% Brij 35 (CN Biosciences)) and then fluorescence intensity is measured to determine the proteinase activity according to the method, or a modification thereof, described in J. Biol. Chem. 271: 17119-17123,1996.

Further, proteinase activities in case (i) where cells capable of expressing the protein F of the invention are cultured in contact with a substrate and in case (ii) where cells capable of expressing the protein F of the invention are cultured in contact the a substrate and a test compound are measured, respectively, and compared.

Specific examples of the substrate useful in the invention include casein, azocasein, type III collagen, cartilage proteoglycan, gelatin, fibronectin, vitronectin, laminin-1, α 1-proteinase inhibitor, α 2-macroglobulin, FITC-labeled casein, collagen, azocollagen, radiolabeled casein (e.g. with ¹⁴C, ³H, etc.), FITC-labeled collagen, radiolabeled collagen, and oligopeptides having (7-methoxycoumarin-4-yl)acetyl group at the N-terminal and N³-(2,4-Dinitrophenyl)-2,3-diaminopropionyl group several residues downstream thereof [e.g. (7-methoxycoumarin-4-yl)acetyl-Pro-Leu-Gly-Leu-[3-(2,4-Dinitrophenyl)-L-2,3-diaminopropionyl]-Ala-Arg-NH₂, etc.].

As the cell capable of producing the protein F of the invention, the afore-mentioned host (transformant) transformed with a vector comprising a DNA encoding the protein of the invention may be used, for example. As the host, animal cells such as CHO cells may be used preferably. In the above-described screening, a transformant which has been cultured by the afore-mentioned method so that the protein F of the invention is expressed on its cell membranes is used preferably. The method of culturing the cells capable of expressing the protein F of the invention is the same as the afore-mentioned method for culturing the transformant of the invention.

The test compound may be, for example, a peptide, protein, non-peptidic compound, synthetic compound, fermentation product, cell extract, plant extract, or animal tissue extract.

For example, a test compound which increases the enzyme activity in case (ii) above by about 20% or more, preferably by about 30% or more, more preferably by about 50% or more compared to the enzyme activity in case (i) above may be selected as a compound that promotes the activity of the protein E of the invention; and a test compound which decreases the enzyme activity in case (ii) above by about 20% or more, preferably by about 30% or more, more preferably by about 50% or more compared to the enzyme activity in case (i) above may be selected as a compound that inhibits the activity of the protein E of the invention.

Compounds that promote the activity of the protein of the invention are useful as safe and low-toxicity drugs to enhance the effect or activity of the protein of the invention.

Compounds that inhibit the activity of the protein of the invention are useful as safe and low-toxicity drugs to inhibit the effect or activity of the protein of the invention.

Compounds or salts thereof obtainable by using the screening method or the screening kit of the invention are compounds selected from, for example, peptides, proteins, non-peptidic compounds, synthetic compounds, fermentation products, cell extracts, plant extracts, animal tissue extracts or plasma. As salts for such compounds, the same salts described in the salts of the protein used in the invention mentioned above may be used.

Further, the expression of the gene encoding the protein of the invention (the protein gene of the invention) also increases in cartilage in osteoarthosis. Therefore, compounds or salts thereof that regulate the expression of the gene encoding the protein of the invention (preferably, compounds or salts thereof that inhibit the expression of the gene encoding the protein of the invention) may be used as prophylactic and/or therapeutic agents for bone and/or arthropathy diseases such as chondrodystrophy, osteodystrophy, osteoporosis, osteoarthosis, rheumatoid arthritis, arthritis, synovitis, metabolic arthropathy or arthropathy disorder caused by sports (e.g. tennis elbow).

Therefore, the DNA of the invention is useful as a reagent for screening for compounds or salts thereof that regulate the expression of the gene encoding the protein of the invention.

As the screening method, a method may be given which is characterized by comparing case (iii) where cells capable of producing the protein of the invention are cultured and case (iv) where cells capable of producing the protein of the invention are cultured in the presence of a test compound.

In the above-described method, the amounts of gene expression (i.e. the amounts of the protein of the invention or the amounts of the mRNA encoding the protein) in cases (iii) and (iv) are measured and compared.

Specific examples of test compounds and cells capable of producing the protein of the invention are the same as describe above.

Measurement of the amount of the protein may be performed by known methods, such as a method in which the protein of the invention in cell extract, etc. is measured by Western analysis, ELISA or the like using an antibody that recognizes the protein of the invention.

Measurement of the amount of mRNA may be performed by known methods, such as Northern hybridization using as a probe a nucleic acid molecule comprising the nucleotide sequence, or a part thereof, represented by SEQ ID NO: 2, SEQ ID NO: 4, SEQ ID NO: 6, SEQ ID NO: 8, SEQ ID NO: 10, SEQ ID NO: 12 or SEQ ID NO: 14; or PCR or a modification thereof using as primers nucleic acid molecules comprising the nucleotide sequence, or a part thereof, represented by SEQ ID NO: 2, SEQ ID NO: 4, SEQ ID NO: 6, SEQ ID NO: 8, SEQ ID NO: 10, SEQ ID NO: 12 or SEQ ID NO: 14.

For example, a test compound which increases the amount of gene expression in case (iv) above by about 20% or more, preferably by about 30% or more, more preferably by about 50% or more compared to the amount of gene expression in case (iii) above may be selected as a compound that promotes the expression of the gene encoding the protein of the invention; and a test compound which decreases the amount of gene expression in case (iv) above by about 20% or more, preferably by about 30% or more, more preferably by about 50% or more compared to the amount of gene expression in case (iii) above may be selected as a compound that inhibits the expression of the gene encoding the protein of the invention.

The screening kit of the invention contains the protein used in the invention or a partial peptide thereof, or a salt of the protein or the partial peptide; or cells capable of producing the protein used in the invention or a partial peptide thereof.

Compounds or salts thereof obtainable by using the screening method or the screening kit of the invention are compounds selected from the afore-mentioned test compounds, for example, peptides, proteins, non-peptidic compounds, synthetic compounds, fermentation products, cell extracts, plant extracts, animal tissue extracts or plasma; they are compounds or salts thereof that regulate the activity of the protein of the invention.

As salts for such compounds, the same salts as described in the salts of the protein of the invention mentioned above may be used.

Compounds or salts thereof that regulate (preferably, inhibit) the activity of the protein of the invention, and compounds or salts thereof that regulate (preferably, inhibit) the expression of the gene encoding the protein of the invention are useful as prophylactic and/or therapeutic agents for bone and/or arthropathy diseases such as chondrodystrophy, osteodystrophy, osteoporosis, osteoarthosis, rheumatoid arthritis, arthritis, synovitis, metabolic arthropathy or arthropathy disorder caused by sports (e.g. tennis elbow).

When the compounds or salts thereof obtainable by using the screening method or the screening kit of the invention are used as the above-described prophylactic and/or therapeutic agents, the compounds or salts thereof may be formulated into preparations according to conventional means.

For example, compositions for oral administration include solid or liquid preparations, i.e. tablets (sugar-coated or film-coated, if necessary), pills, granules, powders, capsules (including soft capsules), syrups, emulsions, suspensions or the like. These compositions are prepared by known methods and contain carriers, diluents or excipients conventionally used in the field of medicine manufacturing. For example, lactose, starch, sucrose, magnesium stearate and the like are used as carriers or excipients for tablets.

Compositions for parenteral administration include, for example, injections and suppositories. Injections include intravenous injections, subcutaneous injections, intradermal injections, muscle injections, instilment injections, intra-articular injections, etc. Such injections may be prepared by dissolving, suspending or emulsifying the above-described antibody or salt thereof in an aseptic, aqueous or oily liquid according to conventional procedures. Examples of aqueous liquids for injection include physiological saline and isotonic solutions containing glucose and other auxiliary agents. They may be used in combination with a suitable auxiliary solubilizer such as alcohol (e.g. ethanol), polyalcohol (e.g. propylene glycol, polyethylene glycol), nonionic surfactant [e.g. Polysorbate 80™, HCO-50 (polyoxyethylene (50 mol) adduct of hydrogenated castor oil)], etc.). Examples of oily liquids for injection include sesame oil and soybean oil. They may be used in combination with an auxiliary solubilizer such as benzyl benzoate, benzyl alcohol, etc. Usually, the prepared injections are filled in appropriate ampoules. Suppositories for administration into rectum may be prepared by mixing the above-described compound or salt thereof with a conventional suppository base.

It is convenient to formulate the above-described pharmaceutical compositions for oral or parenteral administration into unit dosage forms that would give an appropriate dose of the active ingredient. Examples of such unit dosage forms include tablets, pills, capsules, injections (ampoules), and suppositories. Usually, each unit of these dosage forms contains preferably about 5-500 mg of the above-described compound. In particular, each unit contains preferably about 5-100 mg in injections, and each unit in other dosage forms contains preferably about 10-250 mg.

The above-described pharmaceutical compositions may contain other active ingredients as long as they do not produce undesirable interaction with the above-described compound.

Since the thus obtained preparations are safe and of low toxicity, they can be administered to human and other warm-blooded mammals (e.g., mouse, rat, guinea pig, rabbit, sheep, pig, bovine, horse, bird, cat, dog, monkey, chimpanzee, etc.).

Although dose levels of the above-described compound or salt thereof may vary depending on its effect, the target disease, administration route and so on, when a compound or a salt thereof that inhibits the activity of the protein of the invention is administered orally for the purpose of treating osteoarthosis, the compound or salt thereof is generally administered to adult patients (60 kg in body weight) at a dose of about 0.1-100 mg/day, preferably about 1.0-50 mg/day, more preferably about 1.0-20 mg/day. With respect to parenteral administration, when a compound or a salt thereof that inhibits the activity of the protein of the invention is administered, for example, in the form of an injection for the purpose of treating osteoarthosis, it is convenient to intra-articularly inject the compound or salt thereof into adult patients (60 kg in body weight) at a dose of about 0.01-30 mg/day, preferably about 0.1-20 mg/day, and more preferably about 0.1-10 mg/day, though the dose per administration may vary depending on the disease to be treated, etc. For other animals, corresponding doses may be administered after appropriate conversion of the above-mentioned values per 60 kg.

### (2) Quantitative Determination of the Protein of the Invention, Partial Peptides thereof and Salts thereof

Since the antibody against the protein of the invention (hereinafter, optionally referred to as the "antibody of the invention") can specifically recognize the protein of the invention, the antibody may be used for quantitative determination of the protein of the invention contained in sample solutions, in particular, in quantitative determination by sandwich immunoassay.

The present invention provides:
(i) a method of quantitative determination of the protein of the invention in a sample solution, comprising competitively reacting the antibody of the invention with the sample solution and the protein of the invention labeled, and determining the ratio of the labeled protein of the invention bound to the antibody; and
(ii) a method of quantitative determination of the protein of the invention in a sample solution, comprising reacting the sample solution with the antibody of the invention insolubilized on a carrier and another antibody of the invention labeled, simultaneously or in succession, and determining the activity of the labeling agent on the insolubilized carrier.

In the quantitative determination method described in (ii) above, it is preferred that one antibody should be an antibody that recognizes an N-terminal region of the protein of the invention and the other antibody should be an antibody that reacts with a C-terminal region of the protein of the invention.

Further, a monoclonal antibody against the protein of the invention (hereinafter, optionally referred to as the "monoclonal antibody of the invention") may be used to quantitatively determine the protein of the invention or may be used for detection of the protein by tissue staining. For these purposes, either antibody molecules *per se* or the F(ab')₂, Fab' or Fab fragment thereof may be used.

Methods of quantitative determination of the protein of the invention using the antibody of the invention are not particularly limited. Any measuring method may be used in which the amount of antibody, antigen or antibody-antigen complex corresponding to the amount of the antigen in a sample solution (e.g. the amount of the protein of the invention) is detected by chemical or physical means, and then calculated from a standard curve prepared using a standard solution containing a known amount of the antigen. For example, nephrometry, competitive methods, immunometric methods and sandwich assay may be used conveniently and, in terms of sensitivity and specificity, the sandwich assay described later is particularly preferred.

Examples of labeling agents useful in measuring methods utilizing labeling substances include radioisotopes, enzymes, fluorescent substances, and luminescent substances. Examples of radioisotopes include [¹²⁵I], [¹³¹I], [³H] and [¹⁴C]. Preferred examples of enzymes are those which are stable and with high specific activity, e.g., β-galactosidase, β-glucosidase, alkali phosphatase, peroxidase and malate dehydrogenase. Examples of fluorescent substances include fluorescamine and fluorescein isothiocyanate. Examples of luminescent substances include luminol, luminol derivatives, luciferin, and lucigenin. Further, a biotin-avidin system may also be used for binding an antibody or antigen with a labeling agent.

Insolubilization of antigens or antibodies may be performed by physical adsorption or by chemical binding usually used for insolubilizing or immobilizing peptides or enzymes. Examples of carriers useful for this purpose include insoluble polysaccharides such as agarose, dextran and cellulose; synthetic resins such as polystyrene, polyacrylamide and silicone; and glass.

In the sandwich assay, a sample solution is reacted with an insolubilized monoclonal antibody of the invention (primary reaction); then, another monoclonal antibody of the invention that is labeled is reacted therewith (secondary reaction); and the activity of the labeling agent on the insolubilized carrier is measured to thereby quantitatively determine the amount of the protein of the invention in the sample solution. The primary reaction and the secondary reaction may be conducted in the reverse order, or they may be conducted simultaneously or with an interval. The type of the labeling agent and the method of insolubilization may be the same as those described herein earlier. In immunoassays using the sandwich technique, the antibody insolubilized on a solid phase or the antibody labeled is not necessarily a single antibody; a mixture of two or more antibodies may be used for the purposes of enhancing the sensitivity of measurement, etc.

In the method of measuring the protein of the invention by the sandwich assay of the invention, the monoclonal antibodies of the invention used in the primary and the secondary reactions are preferably those antibodies whose sites of binding to the protein of the invention are different from each other. For example, if the antibody used in the secondary reaction recognizes a C-terminal region of the protein of the invention, an antibody that recognizes a site other than the C-terminal region, e.g. an N-terminal region, is preferably used in the primary reaction.

The monoclonal antibody of the invention may be used in a measuring system other than the sandwich assay, such as competitive methods, immunometric methods and nephrometry.

In competitive methods, an antigen in a sample solution and a labeled antigen are reacted competitively with an antibody; then, unreacted labeled antigen (F) and labeled antigen bound to the antibody (B) are separated (i.e. B/F separation); and the amount of the label of B or F is measured to thereby quantitatively determine the amount of the antigen in the sample solution. With respect to this reaction method, there are a liquid phase method in which a soluble antibody is used, and the B/F separation is conducted with polyethylene glycol, and a second antibody against the above-mentioned antibody is used; and a solid phase method in which a solidified antibody is used as the first antibody or a soluble antibody is used as the first antibody while a solidified antibody is used as the second antibody.

In immunometric methods, an antigen in a sample solution and a solidified antigen are reacted competitively with a specific amount of a labeled antibody, followed by separation of the solid phase from the liquid phase; or an antigen in a sample solution is reacted with an excessive amount of a labeled antibody, and then a solidified antigen is added to bind unreacted labeled antibody against the solid phase, followed by separation of the solid phase from the liquid phase. Subsequently, the amount of label in one of the phases is measured to determine the amount of the antigen in the sample solution.

In nephrometry, the amount of insoluble precipitate generated as a result of antigen-antibody reaction in a gel or solution is measured. Even when the amount of the antigen in a sample solution is small and thus only a small amount of such precipitate is obtained, laser nephrometry utilizing the scattering of laser can be used conveniently.

In applying each of those immunological measuring methods to the measuring method of the present invention, no special conditions or operations are required. A measuring system for the protein of the present invention may be constructed using the conventional conditions and operational procedures in the relevant measuring method while taking into account usual technical consideration of those skilled in the art. For details of these commonly used technical means, a variety of reviews, reference books, etc. may be referred to.

For example, Hiroshi Irie (ed.): "Radioimmunoassay" (Kodansha, 1974); Hiroshi Irie (ed.): "Radioimmunoassay; Second Series" (Kodansha, 1979); Eiji Ishikawa et al. (ed.): "Enzyme Immunoassay" (Igaku Shoin, Japan, 1978); Eiji Ishikawa et al. (ed.): "Enzyme Immunoassay" (Second Edition) (Igaku Shoin, 1982); Eiji Ishikawa et al. (ed.): "Enzyme Immunoassay" (Third Edition) (Igaku Shoin, 1987); "Methods in Enzymology", Vol. 70 (Immunochemical Techniques (Part A)); ibid., Vol. 73 (Immunochemical Techniques (Part B)); ibid., Vol. 74 (Immunochemical Techniques (Part C)); ibid., Vol. 84 (Immunochemical Techniques (Part D: Selected Immunoassays)); ibid., Vol. 92 (Immunochemical Techniques (Part E: Monoclonal Antibodies and General Immunoassay Methods)); ibid., Vol. 121 (Immunochemical Techniques (Part I: Hybridoma Technology and Monoclonal Antibodies)) (Academic Press) and the like may be referred to.

By using the antibody of the invention as described above,' the protein of the invention can be quantitatively determined with high sensitivity.

Further, when abnormality is detected in the concentration of the protein of the invention in a subject by quantitatively determining the concentration of the protein of the invention using the antibody of the invention, it is possible to diagnose that the subject has a bone and/or arthropathy disease such as chondrodystrophy, osteodystrophy, osteoporosis, osteoarthosis, rheumatoid arthritis, arthritis, synovitis, metabolic arthropathy or arthropathy disorder caused by sports (e.g. tennis elbow); or that the subject is very likely to develop such a disease in the future.

Further, the antibody of the invention may be used for detecting the protein of the invention present in body fluids, tissues or other samples. The antibody of the invention may also be used in the preparation of antibody columns for use in the purification of the protein of the invention; in the detection of the protein of the invention in individual fractions generated in the course of purification; and in the analysis of the behavior of the protein of the invention in test cells.

### (3) Gene Diagnostics

The DNA of the invention can, when used as a probe for example, detect abnormalities in DNA or mRNA encoding the protein of the invention (gene abnormalities) in human or other warm-blooded animals (e.g. rat, mouse, guinea pig, rabbit, bird, sheep, pig, bovine, horse, cat, dog, monkey, chimpanzee, etc.). Thus, the DNA of the invention is useful as a gene diagnostic for diagnosing, e.g., damage, mutations or reduced expression of the above DNA or mRNA, or increase or excessive expression of the above DNA or mRNA.

Gene diagnosis using the DNA of the invention may be performed by known methods, such as Northern hybridization or PCR-SSCP method (Genomics, Vol. 5, 874-879 (1989); Proceedings of the National Academy of Sciences of the USA, 86: 2766-2770 (1989)).

When abnormality in expression has been detected by Northern hybridization or mutation in DNA has been found by PCR-SSCP method, for example, it is possible to diagnose that the relevant subject is very likely to have a bone and/or arthropathy disease such as chondrodystrophy, osteodystrophy, osteoporosis, osteoarthosis, rheumatoid arthritis, arthritis, synovitis, metabolic arthropathy or arthropathy disorder caused by sports (e.g. tennis elbow).

### (4) Medicines Comprising Antisense Polynucleotide

The antisense polynucleotide that complementarily binds to the DNA of the invention and thus inhibits the expression of that DNA is of low toxicity and capable of inhibiting the *in vivo* activity or function of the protein of the invention or the DNA of the invention (e.g. thyroxin 5'-deiodinase activity, pyrophosphate transport activity, cell differentiation-regulating activity, potassium ion transport activity, the signal transduction activity or ligand-binding activity of Eph receptor, matrix metalloproteinase activity, etc.). Therefore, the antisense polynucleotide may be used as a prophylactic and/or therapeutic agent for bone and/or arthropathy diseases such as chondrodystrophy, osteodystrophy, osteoporosis, osteoarthosis, rheumatoid arthritis, arthritis, synovitis, metabolic arthropathy or arthropathy disorder caused by sports (e.g. tennis elbow).

When the antisense polynucleotide is used as the above-described prophylactic and/or therapeutic agent, the polynucleotide may be formulated and administered according to known methods.

For example, the antisense polynucleotide may be administered, alone or after insertion into an appropriate vector such as a retrovirus vector, adenovirus vector, adeno-associated virus vector, etc., to human or non-human mammals (e.g. rat, rabbit, bird, sheep, pig, bovine, cat, dog, monkey, etc.) orally or parenterally according to conventional means. The antisense polynucleotide may be administered as it is or after formulation with physiologically acceptable carriers such as adjuvants to promote uptake, by means of a gene gun or a catheter such as hydrogel catheter. Alternatively, the antisense polynucleotide may be aerosolized into an inhalant and then administered intratracheally.

Further, the antisense polynucleotide may be formulated (into injections) alone or with a carrier such as liposome in order to improve its internal kinetics, to prolong its half-life or to improve intracellular uptake efficiency, and then administered intravenously, subcutaneously, or into articular cavity or the like.

Dose levels of the antisense polynucleotide may vary depending upon the target disease, patient to be treated, administration route, and so on. When the antisense polynucleotide of the invention is administered for the purpose of treating osteoarthosis, in oral administration, generally, the antisense polynucleotide is administered to adult patients (60 kg in body weight) at a dose of about 0.1-100 mg/day.

Further, the antisense polynucleotide may be used as an oligonucleotide probe for diagnostic purposes to examine the presence or state of expression of the DNA of the invention in tissues or cells.

Like the above-described antisense polynucleotide, double-stranded RNAs comprising a part of an RNA encoding the protein of the invention, ribozymes comprising comprising a part of an RNA encoding the protein of the invention, and decoy oligonucleotide for the DNA sequence to which the protein of the invention (preferably, the protein C of the invention) binds are also capable of inhibiting the expression of the gene of the invention and thus capable of inhibiting the *in vivo* function of the protein of the invention or the DNA of the invention. Therefore, they may be used as prophylactic and/or therapeutic agents for bone and/or arthropathy diseases such as chondrodystrophy, osteodystrophy, osteoporosis, osteoarthosis, rheumatoid arthritis, arthritis, synovitis, metabolic arthropathy or arthropathy disorder caused by sports (e.g. tennis elbow).

Double-stranded RNAs may be designed and prepared based on the sequence of the polynucleotide of the invention by modifications of known methods (e.g. Nature 411:494, 2001).

Ribozymes may be designed and prepared based on the sequence of the polynucleotide of the invention by modifications of known methods (e.g. Trends in Molecular Medicine 7:221, 2001). For example, such a ribozyme may be prepared by linking a known ribozyme to a part of an RNA encoding the protein of the invention. As the part of an RNA encoding the protein of the invention, an RNA fragment close to a cleavage site on the RNA which is cleavable by the known ribozyme may be used.

Decoy oligonucleotides may be designed and prepared based on the DNA sequence to which the protein of the invention (preferably, the protein C of the invention) binds, by modifications of known methods (e.g. The Journal of Clinical Investigation, 106:1071, 2000). Specifically, the decoy oligonucleotide may be any oligonucleotide as long as it has a nucleotide sequence hybridizable under high stringency conditions to the DNA sequence to which the protein of the invention binds to, and the protein of the invention is capable of binding thereto. As the nucleotide sequence hybridizable to the DNA sequence to which the protein of the invention binds, a nucleotide sequence having about 70% or more, preferably about 80% or more, more preferably about 90% or more, most preferably about 95% or more homology to the DNA sequence to which the protein of the invention binds may be used, for example.

When the above-described double-stranded RNA, ribozyme or decoy oligonucleotide is used as the abode-described prophylactic and/or therapeutic agent, they may be formulated and administered in the same manner as described for the antisense polynucleotide.

### (5) Medicines Comprising the Antibody of the Invention

The antibody of the invention that has an effect of neutralizing the activity of the protein of the invention may be used as a prophylactic and/or therapeutic agent for bone and/or arthropathy diseases such as chondrodystrophy, osteodystrophy, osteoporosis, osteoarthosis, rheumatoid arthritis, arthritis, synovitis, metabolic arthropathy or arthropathy disorder caused by sports (e.g. tennis elbow).

The above-mentioned prophylactic and/or therapeutic agent comprising the antibody of the invention is of low toxicity and may be administered orally or parenterally (e.g. intra-articular administration) to human or non-human mammals (e.g. rat, rabbit, sheep, pig, bovine, cat, dog, monkey, etc.) in the forms of liquid preparations without any processing or in appropriate forms of pharmaceutical compositions. Dose levels may vary depending upon the patient to be treated, the target disease, symptoms, administration route, and so on. However, when it is administered to adult patients for the purpose of preventing and treating osteoarthosis, it is convenient to administer in the form of a powder inhalant at a dose of about 0.01-20 mg/kg body weight, preferably about 0.1-10 mg/kg body weight, more preferably about 0.1-5 mg/kg body weight per administration about one to five times a day, preferably about one to three times a day. In other parenteral administration and oral administration, similar dose levels may be used. If symptoms are particularly heavy, the dose may be increased accordingly.

The antibody of the invention may be administered alone or in the forms of appropriate pharmaceutical compositions. The pharmaceutical compositions for the above administration comprise the antibody or salt thereof, pharmacologically acceptable carriers, diluents or excipients. Such compositions are provided in forms appropriate for oral or parenteral (e.g. intra-articular) administration. Preferably, such compositions are provided in the form of an inhalant.

The above-described pharmaceutical compositions may contain other active ingredients as long as they do not produce undesirable interaction with the above-described antibody.

### (6) With Respect to the "Prophylactic and/or Therapeutic Agents for Bone and/or Arthropathy Diseases Comprising a Compound or a Salt thereof that has Thyroxin 5'-Deiodinase Activity-Regulating Effect, Pyrophosphate Transport Activity-Regulating Effect, Potassium Ion Transport Activity-Regulating Effect, Eph Receptor's Signal Transduction Activity-Regulating Effect or Eph Receptor's Ligand-Binding Activity-Regulating Effect" and "Prophylactic and/or Therapeutic Agents for Bone and/or Arthropathy Diseases Comprising a Compound or a Salt thereof that has Thyroxin 5'-Deiodinase Expression-Regulating Effect, Pyrophosphate Transporter Expression-Regulating Effect, Potassium Ion Transporter Expression-Regulating Effect or Eph Receptor Expression-Regulating Effect" of the Invention

The "compound that has thyroxin 5'-deiodinase activity-regulating effect, pyrophosphate transport activity-regulating effect, potassium ion transport activity-regulating effect, Eph receptor's signal transduction activity-regulating effect or Eph receptor's ligand-binding activity-regulating effect" may be any compound as long as it has thyroxin 5'-deiodinase activity-regulating effect, pyrophosphate transport activity-regulating effect, potassium ion transport activity-regulating effect, Eph receptor's signal transduction activity-regulating effect or Eph receptor's ligand-binding activity-regulating effect. Such a compound may be use as a prophylactic and/pr therapeutic agent for bone and/or arthropathy diseases such as chondrodystrophy, osteodystrophy, osteoporosis, osteoarthosis, rheumatoid arthritis, arthritis, synovitis, metabolic arthropathy or arthropathy disorder caused by sports (e.g. tennis elbow).

The "compound that has thyroxin 5'-deiodinase expression-regulating effect, pyrophosphate transporter expression-regulating effect, potassium ion transporter expression-regulating effect or Eph receptor expression-regulating effect" may be any compound as long as it has thyroxin 5'-deiodinase expression-regulating effect, pyrophosphate transporter expression-regulating effect, potassium ion transporter expression-regulating effect or Eph receptor expression-regulating effect. Such a compound may be use as a prophylactic and/pr therapeutic agent for bone and/or arthropathy diseases such as chondrodystrophy, osteodystrophy, osteoporosis, osteoarthosis, rheumatoid arthritis, arthritis, synovitis, metabolic arthropathy or arthropathy disorder caused by sports (e.g. tennis elbow).

Specific examples of the "Eph receptor" include, but are not limited to, EphA1, EphA2, EphA3, EphA4, EphA5, EphA6, EphA7, EphA8, EphB1, EphB2, EphB3, EphB4, EphB5 and EphB6.

The prophylactic and/or therapeutic agents may be prepared in the same manner as described above.

### (7) DNA-Transferred Animals

The present invention further provides non-human mammals harboring a foreign DNA encoding the protein of the invention (hereinafter referred to briefly as the "foreign DNA of the invention") or a mutant thereof (optionally referred to briefly as the "foreign mutant DNA of the invention").

Thus, the present invention provides:
(1) A non-human mammal harboring the foreign DNA of the invention or a mutant DNA thereof:
(2) The non-human mammal according to (1) which is a rodent:
(3) The non-human mammal according to (2) wherein the rodent is mouse or rat; and
(4) A recombinant vector containing the foreign DNA of the invention or a mutant DNA thereof and capable of expressing the DNA in a mammal.

The non-human mammal harboring the foreign DNA of the invention or a mutant DNA thereof (hereinafter referred to briefly as the "DNA-transferred animal of the invention") can be created by transferring the DNA of interest to a germinal cell such as unfertilized egg cells, fertilized egg cells, or sperm cells or primordial cells thereof, preferably during the period of embryogenesis in the ontogenesis of the non-human mammal (more preferably, in the stage of a single cell or a fertilized egg cell and generally at the 8-cell stage or earlier), by the calcium phosphate method, electric pulse method, lipofection method, agglutination method, microinjection method, particle gun method, or DEAE-dextran method. It is also possible to transfer the foreign DNA of the invention of interest into somatic cells, organs in the living body, tissue cells, or the like by such DNA transfer methods to use the resultant cells or tissues in cell culture or tissue culture. Further, by fusing the resultant cells with the above-mentioned germinal cell by known cell fusion methods, it is also possible to create the DNA-transferred animal of the invention.

The non-human mammal useful in the invention includes bovine, pig, sheep, goat, rabbit, dog, cat, guinea pig, hamster, mouse, rat, and so on. From the viewpoint of construction of diseased animal models, rodents which have comparatively short ontogenesis and life cycles and can be easily bred, particularly mouse (e.g. pure strains such as C57BL/6, DBA2, etc. and hybrid strains such as B6C3F₁, BDF₁, B6D2F₁, BALB/c, ICR, etc.) or rat (e.g. Wistar, SD, etc.), are preferred.

Examples of the "mammal" in the expression "a recombinant vector ... capable of expressing the DNA in a mammal" include human in addition to the above-mentioned non-human mammals.

The foreign DNA of the invention is not a DNA of the invention which is inherently possessed by the non-human mammal, but a DNA of the invention that has been once isolated/extracted from a mammal.

Examples of the mutant DNAs of the invention include not only the DNAs that have variations (e.g. mutations) in the nucleotide sequence of the original DNA of the invention as a result of, for example, addition or deletion of nucleotides or substitution with other nucleotides, but also abnormal DNAs.

The term "abnormal DNA" as used herein means any DNA that causes expression of an abnormal protein of the invention. For example, a DNA that allows expression of a protein that inhibits the function of the normal protein of the invention may be used.

The foreign DNA of the invention may be derived from a mammal that is of the same species as that of the host animal or of different species. For transfer of the DNA of the invention to the host animal, it is generally advantageous to use a DNA construct in which the DNA is ligated downstream of a promoter capable of expressing the DNA in animal cells. For example, in transferring the human DNA of the invention, this human DNA of the invention may be ligated downstream of a promoter capable of directing expression of DNAs derived from various animals (e.g. rabbit, dog, cat, guinea pig, hamster, rat, mouse, etc.) harboring the DNA of the invention having high homology to the human DNA to thereby prepare a DNA construct (e.g. vector), which can then be microinjected into fertilized egg cells of a host mammal such as fertilized mouse egg cells. Thus, a DNA-transferred mammal showing high expression of the DNA of the invention can be created.

Examples of the expression vector for the protein of the invention include plasmids derived from *E. coli,* plasmids derived from *B. subtilis,* plasmids derived from yeast, λ phage and other bacteriophages, retroviruses such as Molony leukemia virus, and animal viruses such as vaccinia virus and baculovirus. Preferable examples are *E. coli*-derived plasmids, B. subtilis-derived plasmids and yeast-derived plasmids.

Examples of promoters that regulate the expression of the DNA include (1) promoters for DNAs derived from viruses (e.g. simian virus, cytomegalovirus, Molony leukemia virus, JC virus, papilloma virus, poliovirus, etc.), (2) promoters derived from mammals (e.g. human, rabbit, dog, cat, guinea pig, hamster, rat, mouse, etc.), for example, promoters of albumin, insulin II, uroprakin II, elastase, erythropoietin, endothelin, muscle creatine kinase, glial fibrillary acidic protein, glutathione S-transferase, platelet-derived growth factor β , keratin K1, K10, and K14, collagen type I and type II, cyclic AMP-dependent protein kinase β I subunit, dystrophin, tartaric acid-resistant alkaline phosphatase, atrial natriuretic factor, endothelial receptor tyrosine kinase. (generally abbreviated to Tie2), sodium/potassium-dependent adenosinetriphosphatase (Na, K-ATPase), neurofilament light chain, metallothionein I and IIA, metalloproteinase I tissue inhibitor, MHC Class I antigen (H-2L), H-ras, renin, dopamine β-hydroxylase, thyroid peroxidase (TPO), protein chain elongation factor 1α (EF-1*α),* β-actin, α- and β-myosin heavy chain, myosin light chains 1 and 2, myelin basic protein, thyroglobulin, Thy-1, immunoglobulin H chain variable region (VNP), serum amyloid P component, myoglobin, troponin C, smooth muscle α-actin, preproenkephalin A, vasopressin, and so on. Preferable are those promoters which can direct high expression of the DNA in the whole body, e.g. cytomegalovirus promoter, human protein chain elongation factor 1α (EF-1α) promoter, and human and chicken β-actin promoters.

It is preferable that the vector has a sequence for terminating the transcription of the messenger RNA of interest (generally called terminator) in the DNA-transferred mammal. For example, sequences derived from viruses or various mammals may be used. Preferably, SV40 terminator derived from simian virus or the like is used.

In addition, for enhancing the expression of the DNA of interest further, it is possible, depending on the specific purpose, to ligate a splicing signal, an enhancer domain, a portion of an eukaryotic DNA intron, etc. upstream of the 5'-end of the promoter region, between the promoter region and the translated region, or downstream of the 3'-end of the translated region.

The translated region for the normal protein of the invention may be obtained as the entire genomic DNA or a part thereof from liver-, kidney-, thyroid cell- or fibroblast-derived DNA from human or other mammals (e.g. rabbit, dog, cat, guinea pig, hamster, rat, mouse, etc.) or commercial genomic DNA libraries. Alternatively, the translated region may be obtained using, as a raw material, cDNA prepared from liver-, kidney-, thyroid cell- or fibroblast-derived DNA by conventional methods. The foreign abnormal DNA is capable of creating a mutated translated region from the translated region for the normal protein obtained from the above-mentioned cell or tissue by point mutagenesis.

The translated region can be prepared as a DNA construct which can be expressed in a DNA-transferred animal, by conventional recombinant DNA techniques, i.e. by ligating it downstream of the promoter and, if desired, upstream of the transcription termination site.

The transfer of the foreign DNA of the invention at the fertilized egg cell stage insures that the DNA will be ubiquitous in all the germ cells and somatic cells of the host mammal. The presence of the DNA of the invention in the germ cells of the DNA-transferred animal following DNA transfer means that all the germinal cells and somatic cells of all the subsequent generations of the DNA-transferred animal harbor the DNA of the invention. Thus, the progeny of such DNA-transferred animal which inherited the foreign DNA of the invention have the DNA in all of their germ cells and somatic cells.

The non-human mammal into which the foreign normal DNA of the invention has been transferred can be verified by mating to retain the foreign DNA stably and then bred as a line harboring that DNA from generation to generation under usual breeding conditions.

The transfer of the foreign DNA of the invention at the fertilized egg cell stage insures that the DNA will be present in excess in all the germ cells and somatic cells of the host mammal. The presence of the foreign DNA of the invention in the germ cells of the DNA-transferred animal following the DNA transfer means that all the germinal cells and somatic cells of all the progeny of the DNA-transferred animal harbor the foreign DNA of the invention in excess. Thus, the progeny of such DNA-transferred animal which inherited the foreign DNA of the invention have the DNA in excess in their germ cells and somatic cells.

By preparing homozygous animals having the transferred DNA in both homologous chromosomes and mating male animals with female animals, it is possible to breed through generations so that every progeny harbors the DNA in excess.

The non-human mammal harboring the normal DNA of the invention features a high expression of the normal DNA and may eventually develop a hyperergasia of the protein of the invention through activation of the function of the endogenous normal DNA. Thus, the animal can be utilized as an animal model of that disease. For example, by using the DNA-transferred animal harboring the normal DNA of the invention, it is possible to study the hyperergasia of the protein of the invention, to elucidate the mechanisms of diseases with which the protein of the invention is associated, and to explore therapeutic modalities for the diseases.

Furthermore, the mammal to which the foreign normal DNA of the invention has been transferred presents symptoms due to an increase in the free protein of the invention and, therefore, can also be used in the screening of prophylactic and/or therapeutic agents for diseases with which the protein of the invention is associated, e.g. bone and/or arthropathy diseases such as chondrodystrophy, osteodystrophy, osteoporosis, osteoarthosis, rheumatoid arthritis, arthritis, synovitis, metabolic arthropathy or arthropathy disorder caused by sports (e.g. tennis elbow).

On the other hand, the non-human mammal harboring the foreign abnormal DNA of the invention can be verified by mating to retain the DNA stably and then bred as a line harboring the DNA from generation to generation under usual breeding conditions. Moreover, it is possible to incorporate the foreign DNA of interest in the above-mentioned plasmid and use it as a starting material. The DNA construct with the promoter can be prepared by conventional recombinant DNA techniques. Transfer of the abnormal DNA of the invention in the fertilized egg cell stage insures that the transferred DNA will be ubiquitous in all the germ cells and somatic cells of the host mammal. The presence of the abnormal DNA of the invention in the germ cells of the DNA-transferred animal means that all the progeny of this DNA-transferred animal harbor the abnormal DNA of the invention in all of their germinal cells and somatic cells. The progeny of this animal harbor the abnormal DNA of the invention in all of their germinal cells and somatic cells. By preparing homozygous male and female animals having the introduced DNA in both homologous chromosomes and mating them, it can be insured that every progeny harbors the DNA from generation to generation.

The non-human mammal harboring the abnormal DNA of the invention features a high expression of the abnormal DNA and, therefore, may eventually develop adiaphoria associated with functional inactivation of the protein of the invention through inhibition of the function of the endogenous normal DNA. Thus, the animal can be utilized as an animal model of that disease. For example, by using the DNA-transferred animal harboring the abnormal DNA of the invention, analysis of the mechanism of this functional inactivation adiaphoria attributable to the protein of the invention and therapeutic modalities for the disease can be explored.

As a specific potential use, the DNA-transferred animal with a high expression of the abnormal DNA of the invention can be used as a model for elucidating the functional inhibition of the normal protein by the abnormal protein of the invention (dominant negative effect) in adiaphoria of functional inactivation.

Moreover, the DNA-transferred mammal harboring the foreign abnormal DNA of the invention develops symptoms due to an increase in the free protein of the invention and, therefore, can be utilized in the screening of prophylactic and/or therapeutic agents for adiaphoria attributable to functional inactivation of the protein of the invention, e.g. bone and/or arthropathy diseases such as chondrodystrophy, osteodystrophy, osteoporosis, osteoarthosis, rheumatoid arthritis, arthritis, synovitis, metabolic arthropathy or arthropathy disorder caused by sports (e.g. tennis elbow).

As other potential uses of the two types of DNA-transferred animals harboring the two kinds of DNAs of the invention, the following may be considered:
(1) Use as a cell source for tissue culture;
(2) Analysis of those proteins which are expressed or activated specifically by the protein of the invention, by analyzing the DNA or RNA in tissues of the DNA-transferred animal of the invention or by analyzing the compositions of the peptides expressed by the DNA;
(3) Study of the functions of cells of those tissues which are generally difficult to culture, by using the cells from the tissues containing the DNA as cultured by the standard tissue culture technique;
(4) Screening for drugs capable of enhancing the cell functions by using the cells described in (3); and
(5) Isolation and purification of the mutant protein of the invention and construction of antibodies thereto.

Furthermore, by using the DNA-transferred animal of the invention, clinical symptoms of diseases associated with the protein of the invention, inclusive of above-described adiaphoria associated with functional inactivation of the protein of the invention, can be investigated. In addition, more detailed pathological findings can be obtained in various organs of this model of diseases associated with the protein of the invention, thus contributing to the development of new therapies as well as the study and treatment of secondary diseases arising from such diseases.

Moreover, by removing various organs from the DNA-transferred animal of the invention, mincing them and digesting them with a proteolytic enzyme such as trypsin, free single cells harboring the transferred DNA can be recovered. These cells can be cultured for establishment of a cell line. Furthermore, characterization of cells producing the protein of the invention can be made and their relationship with apoptosis, differentiation, or proliferation, the mechanism of signal transduction in them, and abnormalities involved can be explored to thereby generate information useful for further elucidation of the protein of the invention and its effects.

Moreover, for the development of therapeutic drugs for diseases associated with the protein of the invention, such as adiaphoria resulted from functional inactivation of the protein of the invention by using the DNA-transferred animal of the invention, an effective and rapid screening technology for such therapeutic drugs can be established by using the test and assay methods described hereinbefore. In addition, by using the above DNA-transferred animal or the foreign DNA expression vector of the invention, gene therapies for diseases associated with the protein of the invention can be explored and developed.

### (8) Knockout Animals

The invention further provides non-human mammalian embryonic stem cells wherein the DNA of the invention is inactivated, and non-human mammals deficient in expression of the DNA of the invention wherein the DNA of the invention is deactivated.

The invention, therefore, provides:
(1) A non-human mammalian embryonic stem cell wherein the DNA of the invention is inactivated;
(2) The embryonic stem cell according to in (1) wherein the DNA is inactivated by introduction of a reporter gene (e.g. *E. coli*-derived β -galactosidase gene);
(3) The embryonic stem cell according to (1) which is neomycin-resistant;
(4) The embryonic stem cell according to (1) wherein the non-human mammal is a rodent;
(5) The embryonic stem cell according to (4) wherein the rodent is mouse;
(6) A non-human mammal deficient in expression of the DNA of the invention, wherein the DNA is inactivated;
(7) The non-human mammal according to (6) wherein the DNA is inactivated by introduction of a reporter gene (e.g. *E. coli*-derived β-galactosidase gene) and the reporter gene can be expressed under the control of the promoter for the DNA of the invention;
(8) The non-human mammal according to (6) wherein the non-human mammal is a rodent;
(9) The non-human mammal according to (8) wherein the rodent is mouse; and
(10) A method for screening for compounds, or salts thereof, that enhance or inhibit the promoter activity for the DNA of the invention, which comprises administering a test compound to the non-human mammal according to (7) and detecting expression of the reporter gene.

The expression "non-human mammalian embryonic stem cell wherein the DNA of the invention is inactivated" means the embryonic stem cell (hereinafter referred to briefly as ES cell) of a non-human mammal in which the DNA has been deprived of the capacity to express the protein of the invention (hereinafter, optionally referred to as the "knockout DNA of the invention") through introduction of an artificial mutation to the DNA of the invention possessed by the non-human mammal to thereby inhibit expression of the DNA of the invention or through substantial deprivation of the activity of the protein of the invention encoded by the DNA.

As the non-human mammals, the same animals as mentioned hereinbefore may be used.

Examples of the method for introducing an artificial mutation to the DNA of the invention are a deletion of some or all of the DNA sequence, or an insertion of a different DNA, or substitution with a different DNA by the genetic engineering technology. The knockout DNA of the invention may be created by such a mutation that would shift the reading frame or destroy the function of the promoter or exon.

The non-human mammalian embryonic stem cell wherein' the DNA of the invention is inactivated (hereinafter referred to as the "DNA inactivated ES cell of the invention" or the "knockout ES cell of the invention") can be prepared by, for example, procedures which comprise isolating the DNA of the invention from a non-human mammal of interest, inserting a drug-resistance gene, typically neomycin-resistance gene or hygromycin-resistance gene, or a reporter gene such as *lacZ* (*β*-galactosidase gene) or *cat* (chloramphenicol acetyltransferase gene) into its exon region to disrupt the function of the exon or inserting a DNA sequence for terminating gene transcription (e.g. poly A addition signal) in an intron region between exons to thereby inhibit synthesis of a complete mRNA, introducing the thus-constructed DNA strand having a DNA sequence designed to eventually disrupt the gene (hereinafter, referred to briefly as the "targeting vector") into the chromosomes of the host animal by homologous recombination, subjecting the resulting ES cell to Southern hybridization analysis using a DNA sequence located on the DNA of the invention or in its vicinity as a probe or a PCR procedure using a DNA sequence located on the targeting vector and a DNA sequence in the vicinity but not including the DNA of the invention used in the construction of the targeting vector as primers, and selecting the knockout ES cell of the invention.

The original ES cell to be used for inactivation of the DNA of the invention by the homologous recombination technique or the like may be an already established cell line such as those mentioned hereinbefore or a new cell line established de novo by the known method of Evans and Kaufman. Taking mouse ES cells as an example, ES cells of the 129 line are generally employed but the immunological background of this line is not clear. Therefore, the cell line established by using BDF₁ mice created by the hybridization of C57BL/6 mice and C57BL/6 mice, both yielding few eggs, with DBA/2 mice (BDF₁ =F₁ of C57BL/6 and DBA/2) for preparing pure-line ES cells with an immunologically defined genetic background can be used with advantage. In addition to the advantage of high egg output and sturdiness of the egg, BDF₁ mice have the background of C57BL/6 mice so that in the construction of a disease model with ES cells obtained the genetic background of the model mice can be converted to that of C57BL/6 mice by back-crossing with C57BL/6.

Moreover, in establishing an ES cell line, it is common practice to use blastocytes 3.5 days following fertilization but, aside from them, a large number of early embryos can be prepared with high efficiency by harvesting the embryos at the 8-cell stage and culturing them into blastocytes.

Furthermore, while ES cells from both male and female animals can be used, generally ES cells of male animals are more convenient for the construction of reproduction line chimeras. Moreover, for the purpose of reducing the burden of the complicated cultural procedure, it is preferable to carry out sexing as early as possible.

As a typical method for sexing ES cells, there can be mentioned the method in which the gene in the sex determination region on the Y chromosome is amplified and detected by PCR. Whereas the conventional karyotype analysis requires about 10⁶ cells, the above method requires only about one colony equivalent of ES cells (about 50 cells). Therefore, the primary selection of ES cells in an early stage can be made by this sexing method. Since male cells can thus be selected in the early stage, the trouble in the initial stage of culture can be drastically reduced.

Moreover, the secondary selection can be carried out by G-banding for the number of chromosomes. The number of chromosomes in the resulting ES cell is preferably 100% of the normal number but this goal may not be reached due to the physical and other factors involved in the establishment of the line. In such cases, it is preferable to knockout the gene of the ES cell and re-clone it into the normal cell (taking a mouse as an example, the cell in which the number of chromosomes is 2n=40).

The embryonic stem cell line thus established is generally very satisfactory in proliferation characteristic but since it is liable to lose its ontogenic ability, it must be subcultured with sufficient care. For example, this cell line should be cultured on suitable feeder cells such as STO fibroblasts in the presence of LIF (1-10000 U/ml) in a carbon dioxide incubator (preferably 5% CO₂, 95% air or 5% oxygen, 5% CO₂, 90% air) at about 37°C. and, in subculture, it should be treated with trypsin/EDTA solution (generally 0.001-0.5% trypsin/0.1-5 mM EDTA, preferably about 0.1% trypsin/1 mM EDTA) to provide single cells and seed them on freshly prepared feeder cells. While such subculture is generally performed every 1-3 days, it is good practice to observe the cells on each occasion and, whenever morphologically abnormal cells are discovered, discard the culture.

ES cells can be allowed to differentiate into various types of cells, such as head long muscle cells, visceral muscle cells, heart muscle cells, etc. by conducting monolayer culture to a high density under suitable conditions or suspension culture until a mass of cells is formed (M. J. Evans & M. H. Kaufman, Nature, 292, 154, 1981; G. R. Martin, Proceedings of National Academy of Science USA, 78, 7634, 1981; T. C. Doetschman et al., Journal of Embryology and Experimental Morphology, 87, 27, 1985), and the cell deficient in expression of the DNA of the invention as obtainable by causing the ES cell of the invention to differentiate is useful for the cytobiological *in vitro* study of the protein of the invention.

The non-human mammal deficient in expression of the DNA of the invention can be differentiated from normal animals by assaying the mRNA in the animals by the known method and comparing the amounts of expression indirectly.

As the non-human mammal used for this purpose, the same animals as mentioned hereinbefore may be used.

With respect to the non-human mammal deficient in expression of the DNA of the invention, the DNA of the invention can be knocked out by introducing the targeting vector constructed as described above into, for example, mouse embryonic stem cells or mouse egg cells and thereby allowing the DNA sequence of the targeting vector harboring the inactivated DNA of the invention to undergo homologous recombination with, and accordingly replacing, the DNA of the invention on the mouse embryonic stem cell or egg cell chromosomes.

The cell with the DNA of the invention thus knocked out can be judged by Southern hybridization analysis using a DNA sequence on the DNA of the invention or in its vicinity as a probe or by PCR using a DNA sequence on the targeting vector or a mouse-derived DNA sequence in a region adjacent to but not including the DNA of the invention used in the targeting vector as primers. When a non-human mammalian embryonic stem cell is used, a cell line with the DNA of the invention knocked out by the homologous recombination technique is cloned and injected into the non-human mammalian embryo or blastocyte at a suitable stage of embryogenesis, for example at the 8-cell stage, and the resulting chimera embryo is transplanted in the pseudopregnant uterus of the non-human mammal. The animal thus obtained is a chimera animal constituted by both the cells harboring the normal DNA locus of the invention and the cells harboring the artificially mutated DNA locus of the invention.

When some of the gametes of this chimera animal harbor the mutated DNA locus of the invention, an individual whose entire tissue is constituted by cells harboring the mutated DNA locus of the invention can be screened from the colony of animals obtained by crossing such a chimera animal with a normal animal, for example by coat color discrimination. The individuals thus selected are usually animals hetero-deficient in expression of the protein of the invention and by mating such individuals hetero-deficient in expression of the protein of the invention with each other, animals homo-deficient in expression of the protein of the invention can be acquired.

When egg cells are used, a transgenic non-human mammal with the targeting vector having been introduced into its chromosomes can be prepared by injecting the DNA solution into the egg cell nucleus by the microinjection technique and selecting animals expressing a mutation of the DNA of the invention by homologous recombination.

The individuals with the DNA of the invention thus knocked out are mated to verify that the animals obtained by mating also have the DNA knocked out and they can be sub-bred under the usual breeding conditions.

Preparation and maintenance of the germ line may also be carried out in accordance with conventional methods. Thus, by mating male and female animals harboring the inactivated DNA, homozygotes having the inactivated DNA in both homologous chromosomes can be obtained. The homozygotes thus obtained are bred under such conditions that, with regard to the dam, the number of homozygotes is plural per normal individual. By mating male and female heterozygotes, homozygotes and heterozygotes both harboring the inactivated DNA can be sub-bread.

The non-human mammalian embryonic stem cell harboring the inactivated DNA of the invention is very useful for the construction of non-human mammals deficient in expression of the DNA of the invention.

Moreover, the mammal deficient in expression of the DNA of the invention lacks various biological activities inducible by the protein of the invention and can, therefore, be of use as an animal model of diseases arising from inactivation of the biological activities of the protein of the invention, thus being useful in the etiological studies of such diseases and development of therapeutic methods.

### (8a) Method of Screening for Compounds with Therapeutic/Prophylactic Effect upon Diseases Resulted from Deficiency or Damage of the DNA of the Invention

Non-human mammals deficient in expression of the DNA of the invention may be used for screening for compounds with a therapeutic and/or prophylactic effect upon diseases resulted from deficiency or damage of the DNA of the invention.

The present invention provides a method of screening for compounds, or salts thereof, having a therapeutic and/or prophylactic effect upon diseases resulted from deficiency or damage of the DNA of the invention, which is characterized by administering a test compound to a non-human mammal deficient in expression of the DNA of the invention and observing and measuring the changes in the mammal.

As the non-human mammal deficient in expression of the DNA of the invention used in the above screening method, the same animals as described earlier may be used.

The test compound may be, for example, a peptide, protein, non-peptidic compound, synthetic compound, fermentation product, cell extract, plant extract, animal tissue extract or plasma. These compounds may be either novel compounds or known compounds.

Specifically, a non-human mammal deficient in expression of the DNA of the invention is treated with a test compound and then compared with a control animal not treated with the compound. Subsequently, the therapeutic and/or prophylactic effect of the test compound may be examined using the changes in individual organs, tissues or disease symptoms in the mammal as indicators.

As a method for treating a test animal with a test compound, oral administration, intravenous injection, or the like may be used. The method may be appropriately selected depending on the symptoms of the test animal, the nature of the test compound, and so on. Dose levels of the test compound may be appropriately selected taking into account of the administration method, the nature of the test compound, and so on. Further, dose levels of the test compound may be appropriately selected depending on the administration method, the nature of the relevant test compound, and so on.

When compounds that have a therapeutic and/or prophylactic effect upon bone and/or arthropathy diseases such as chondrodystrophy, osteodystrophy, osteoporosis, osteoarthosis, rheumatoid arthritis, arthritis, synovitis, metabolic arthropathy or arthropathy disorder caused by sports (e.g. tennis elbow) are screened for, test compounds are administered to non-human mammals deficient in the expression of the DNA of the invention, followed by observation of the time course of arthropathy and cartilage of the mammals to observe the state of diseases.

In the above screening method, when a test compound has been administered to a test animal and, as a result, the symptoms of the above-described disease have been improved by about 10% or more, preferably by about 30% or more, more preferably by about 50% or more, the test compound can be selected as a compound that has a therapeutic and/or prophylactic effect upon the above-described disease.

Compounds obtainable by using the screening method of the invention are compounds selected from the above-mentioned test compounds and have a therapeutic and/or prophylactic effect upon diseases resulted from deficiency or damage of the protein of the invention. Therefore, they may be used as medicines that are safe and of low toxicity, such as prophylactic and/or therapeutic agents for those diseases that are safe and of low toxicity. Furthermore, compounds derived from those compounds obtained by the above screening may also be used in the same manner.

The compound obtained by the above screening may be in a salt form. As salts of the compounds, salts formed with physiologically acceptable acids (e.g. organic or inorganic acids) or bases (e.g. alkali metals) may be used. Especially preferable are physiologically acceptable acid addition salts. Examples of such salts include salts formed with inorganic acids (e.g. hydrochloric acid, phosphoric acid, hydrobromic acid or sulfuric acid) and salts formed with organic acids (e.g. acetic acid, formic acid, propionic acid, fumaric acid, maleic acid, succinic acid, tartaric acid, citric acid, malic acid, oxalic acid, benzoic acid, methanesulfonic acid or benzenesulfonic acid).

Medicines comprising the compound, or a salt thereof, obtained by the above screening may be prepared in the same manner as described for medicines comprising the protein of the invention.

Since the thus obtained preparations are safe and of low toxicity, they may be administered to, for example, human or non-human mammals (such as rat, mouse, guinea pig, rabbit, sheep, pig, bovine, horse, cat, dog, monkey, etc.).

Dose levels of the above compound or a salt thereof may vary depending upon the target disease, the patient to be treated, administration route, and so on. When the compound is administered orally, generally the compound is administered to adult patients with osteoarthosis (60 kg in body weight) at a dose of about 0.1-100 mg/day, preferably about 1.0-50 mg/day, more preferably about 1.0-20 mg/day. With respect to parenteral administration, when the compound is administered to adult patients with osteoarthosis (60 kg in body weight), for example, in the form of an injection, it is convenient to intravenously inject the compound at a dose of about 0.01-30 mg/day, preferably about 0.1-20 mg/day, and more preferably about 0.1-10 mg/day, though the dose per administration may vary depending on the patient to be treated, the target disease, etc. For other animals, corresponding doses may be administered after appropriate conversion of the above-mentioned values per 60 kg.

### (8b) Method of Screening for Compounds that Promote or Inhibit the Activity of the Promoter for the DNA of the Invention

The present invention provides a method of screening for compounds, or salts thereof, that promote or inhibit the activity of the promoter for the DNA of the invention, which is characterized by administering a test compound to a non-human mammal deficient in expression of the DNA of the invention and detecting the expression of a reporter gene.

In the above screening method, there is used a non-human mammal deficient in expression of the DNA of the invention wherein the DNA of the invention is inactivated as a result of introduction of a reporter gene, and this reporter gene is capable of being expressed under the control of the promoter for the DNA of the invention.

As the test compound, the compounds as enumerated above may be used.

As the reporter gene, the genes as enumerated above may be used. Among all, β - galactosidase gene (*lacZ*), soluble alkali phosphatase gene or luciferase gene may be preferably used.

In the non-human mammal deficient in expression of the DNA of the invention wherein the DNA of the invention is replaced with a reporter gene, since the reporter gene is present under the control of the promoter for the DNA of the invention, the promoter activity can be detected by tracing the expression of the substance encoded by the reporter gene.

For example, when a part of the DNA region encoding the protein of the invention is replaced with *E. coli*-derived β-galactosidase gene (lacZ), β-galactosidase is expressed instead of the protein of the invention in those tissues where originally the protein of the invention has been expressed. Thus, by staining with a substrate for β-galactosidase such as 5-bromo-4-chloro-3-indolyl-β-D-galactopyrasosidase (X-gal), it is possible to observe the state of *in vivo* expression of the protein of the invention in the mammal simply. Specifically, mice deficient in the protein of the invention or tissue sections thereof may be fixed in glutaraldehyde or the like, washed with phosphate-buffered physiological saline (PBS), and treated with a staining solution containing X-gal at room temperature or around 37°C for about 30 min to 1 hr. Subsequently, the tissue samples are washed with 1 mM EDTA/PBS solution to terminate the β-galactosidase reaction, followed by observation of the resultant color development. Alternatively, mRNA encoding *lacZ* may be detected according to conventional methods.

The compounds or salts thereof obtainable by the above-described screening are compounds that are selected from the above-mentioned test compounds and yet promote or inhibit the promoter activity for the DNA of the invention.

The compound obtained by the above screening may be in a salt form. As salts of the compound, salts formed with physiologically acceptable acids (e.g. organic or inorganic acids) or bases (e.g. alkali metals) may be used. Especially preferable are physiologically acceptable acid addition salts. Examples of such salts include salts formed with inorganic acids (e.g. hydrochloric acid, phosphoric acid, hydrobromic acid or sulfuric acid) and salts formed with organic acids (e.g. acetic acid, formic acid, propionic acid, fumaric acid, maleic acid, succinic' acid, tartaric acid, citric acid, malic acid, oxalic acid, benzoic acid, methanesulfonic acid or benzenesulfonic acid).

Since compounds or salts thereof that promote the promoter activity for the DNA of the invention can promote the expression of the protein of the invention and thereby promote the function thereof, they may be used as prophylactic and/or therapeutic agents for bone and/or arthropathy diseases such as chondrodystrophy, osteodystrophy, osteoporosis, osteoarthosis, rheumatoid arthritis, arthritis, synovitis, metabolic arthropathy or arthropathy disorder caused by sports (e.g. tennis elbow)

Further, compounds derived from those compounds obtained from the above screening may also be used in the same manner.

Medicines comprising the compound or, a salt thereof, obtained by the above screening may be prepared in the same manner as described for medicines comprising the protein of the invention or a salt thereof.

Since the thus obtained preparations are safe and of low toxicity, they may be administered to, for example, human or other non-human mammals (such as rat, mouse, guinea pig, rabbit, sheep, pig, bovine, horse, cat, dog, monkey, etc.).

Dose levels of the above compound or a salt thereof may vary depending upon the target disease, the patient to be treated, administration route, and so on. When a compound that promotes the promoter activity for the DNA of the invention is administered orally, generally the compound is administered to adult patients with osteoarthosis (60 kg in body weight) at a dose of about 0.1-100 mg/day, preferably about 1.0-50 mg/day, more preferably about 1.0-20 mg/day. With respect to parenteral administration, when a compound that promotes the promoter activity for the DNA of the invention is administered to adult patients with osteoarthosis (60 kg in body weight), for example, in the form of an injection, it is convenient to intravenously inject the compound at a dose of about 0.01-30 mg/day, preferably about 0.1-20 mg/day, and more preferably about 0.1-10 mg/day, though the dose per administration may vary depending on the patient to be treated, the target disease, etc. For other animals, corresponding doses may be administered after appropriate conversion of the above-mentioned values per 60 kg.

Thus, the non-human mammal deficient in expression of the DNA of the invention is extremely useful in screening for compounds, or salts thereof, that promote or inhibit the promoter activity for the DNA of the invention, and may contribute greatly to the elucidation of causes of various diseases resulted from deficiency in expression of the DNA of the invention, or to the development of prophylactic and/or therapeutic agents for such diseases.

When the so-called transgenic animal is created by using a DNA comprising the promoter region of the protein of the invention, ligating genes encoding various proteins downstream of the promoter region, and injecting the thus prepared construct into egg cells of an animal, it is possible to allow the animal to synthesize the protein specifically, which will enable the examination of the *in vivo* function of the protein. Further, if a cell line which expresses an appropriate reporter gene ligated to the above promoter region has been established, such a cell line may be used as a search system for those low molecular weight compounds that specifically promote or inhibit *in vivo* production ability for the protein of the invention *per se.*

In the specification and drawings of the present application, the abbreviations used for bases (nucleotides), amino acids and so forth are those recommended by the IUPAC-IUB Commission on Biochemical Nomenclature or those conventionally used in the art. Examples of such abbreviations are given below. Amino acids that may have optical isomers are intended to represent their L-isomer unless otherwise specified.
- DNA:: Deoxyribonucleic acid
- cDNA:: Complementary deoxyribonucleic acid
- A:: Adenine
- T:: Thymine
- G:: Guanine
- C:: Cytosine
- RNA:: Ribonucleic acid
- mRNA:: Messenger ribonucleic acid
- dATP:: Deoxyadenosine triphosphate
- dTTP:: Deoxythymidine triphosphate
- dGTP:: Deoxyguanosine triphosphate
- dCTP:: Deoxycytidine triphosphate
- ATP:: Adenosine triphosphate
- EDTA:: Ethylenediaminetetracetic acid
- SDS:: Sodium dodecyl sulfate
- Gly:: Glycine
- Ala:: Alanine
- Val:: Valine
- Leu:: Leucine
- Ile:: Isoleucine
- Ser:: Serine
- Thr:: Threonine
- Cys:: Cysteine
- Met:: Methionine
- Glu:: Glutamic acid
- Asp:: Aspartic acid
- Lys:: Lysine
- Arg:: Arginine
- His:: Histidine
- Phe:: Phenylalanine
- Tyr:: Tyrosine
- Trp:: Tryptophan
- Pro:: Proline
- Asn :: Asparagine
- Gln:: Glutamine
- pGlu:: Pyroglutamic acid
- Sec:: Selenocysteine

The substituents, protective groups and reagents which are frequently used in the specification are represented by the following abbreviations.
- Me:: Methyl
- Et:: Ethyl
- Bu:: Butyl
- Ph:: Phenyl
- TC:: Thiazolidine-4(R)-carboxamide
- Tos:: p-Toluene sulfonyl
- CHO:: Formyl
- Bzl:: Benzyl
- Cl₂-Bzl:: 2,6-Dichlorobenzyl
- Bom:: Benzyloxymethyl
- Z:: Benzyloxycarbonyl
- Cl-Z:: 2-Chlorobenzyloxycarbonyl
- Br-Z:: 2-Bromobenzyloxycarbonyl
- Boc:: t-Butoxycarbonyl
- DNP:: Dinitrophenol
- Trt:: Trityl
- Bum:: t-Butoxymethyl
- Fmoc:: N-9-Fluorenylmethyloxycarbonyl
- HOBt:: 1-Hydroxybenzotriazole
- HOOBt:: 3,4-Dihydro-3-hydroxy-4-oxo-1,2,3-benzotriazine
- HONB:: 1-Hydroxy-5-norbornene-2,3-dicarboximide
- DCC:: N,N'-Dicyclohexylcarbodiimide

The SEQ ID NOS of the SEQUENCE LISTING of the present specification represent the sequences as indicated below.
[SEQ ID NO: 1]
   This shows the amino acid sequence of DIO2.
[SEQ ID NO: 2]
   This shows the nucleotide sequence of the DNA encoding DIO2 having the amino acid sequence represented by SEQ ID NO: 1.
[SEQ ID NO: 3]
   This shows the amino acid sequence of ANKH.
[SEQ ID NO: 4]
   This shows the nucleotide sequence of the DNA encoding ANKH having the amino acid sequence represented by SEQ ID NO: 3.
[SEQ ID NO: 5]
   This shows the amino acid sequence of SHOX2a.
[SEQ ID NO: 6]
   This shows the nucleotide sequence of the DNA encoding SHOX2a having the amino acid sequence represented by SEQ ID NO: 5.
[SEQ ID NO: 7]
   This shows the amino acid sequence of SHOX2b.
[SEQ ID NO: 8]
   This shows the nucleotide sequence of the DNA encoding SHOX2b having the amino acid sequence represented by SEQ ID NO: 7.
[SEQ ID NO: 9]
   This shows the amino acid sequence of TASK-4.
[SEQ ID NO: 10]
   This shows the nucleotide sequence of the DNA encoding TASK-4 having the amino acid sequence represented by SEQ ID NO: 9.
[SEQ ID NO: 11]
   This shows the amino acid sequence of EphA3.
[SEQ ID NO: 12]
   This shows the nucleotide sequence of the DNA encoding EphA3 having the amino acid sequence represented by SEQ ID NO: 11.
[SEQ ID NO: 13]
   This shows the amino acid sequence of MMP16.
[SEQ ID NO: 14]
   This shows the nucleotide sequence of the DNA encoding MMP16 having the amino acid sequence represented by SEQ ID NO: 13.
[SEQ ID NO: 15]
   This shows the nucleotide sequence of a primer used in Example 9.
[SEQ ID NO: 16]
   This shows the nucleotide sequence of a primer used in Example 9.
[SEQ ID NO: 17]
   This shows the nucleotide sequence of primer 1 used in Example 10.
[SEQ ID NO: 18]
   This shows the nucleotide sequence of primer 2 used in Example 10.
[SEQ ID NO: 19]
   This shows the nucleotide sequence of primer 3 used in Example 10.
[SEQ ID NO: 20]
   This shows the nucleotide sequence of primer 4 used in Example 10.
[SEQ ID NO: 21]
   This shows the nucleotide sequence of primer 5 used in Example 10.
[SEQ ID NO: 22]
   This shows the nucleotide sequence of primer 6 used in Example 10.
[SEQ ID NO: 23]
   This shows the nucleotide sequence of primer 7 used in Example 10.
[SEQ ID NO: 24]
   This shows the nucleotide sequence of primer 8 used in Example 10.

Hereinbelow, the present invention will be described in more detail with reference to the following Examples. However, the present invention is not limited to these Examples.

### EXAMPLES

### EXAMPLE 1

### Gene Expression Analysis

In order to ascertain a group of genes whose expression is increased in cartilage in osteoarthosis (OA cartilage), gene expression analysis was carried out on total RNA extracted from OA cartilage (knee arthropathy: 1 sample; hip arthropathy: 5 samples), normal arthrodial cartilage (knee arthropathy: 1 sample; hip arthropathy: 1 sample) and other 22 normal tissues (1 sample for each tissue) (see Table 1) using oligonucleotide microarrays (Human Genome U95A; Affimetrix).

Experimental procedures were according to the Expression Analysis Technical Manual issued by Affimetrix. Comparison of gene expression profiles between OA cartilage and normal arthrodial cartilage revealed that the expression of *DIO2* gene was increased in OA cartilage (Table 2).

It has been reported that the expression of *DIO2* is controlled tissue-specifically and is expressed in the thyroid at a particularly high level (Salvatore et al., J. Clin. Invest. 98: 962-968, 1996). The expression level of *DIO2* in OA cartilage was almost the same as the expression level in the thyroid.

**Table 1**

| Tissue from which RNA was extracted | Seller |
|---|---|
| Cartilage in knee osteoarthosis | Direct Clinical Access |
| Cartilage in hip osteoarthosis | Direct Clinical Access |
| Normal knee arthrodial cartilage | Direct Clinical Access |
| Normal hip arthropathy | Direct Clinical Access |
| Costal cartilage | Direct Clinical Access |
| Fat | BioChain Institute |
| Skeletal muscle | Clontech |
| Heart | Clontech |
| Kidney | Clontech |
| Adrenal | Clontech |
| Liver | Clontech |
| Pancreas | Clontech |
| Spleen | Clontech |
| Trachea | Clontech |
| Lung | Clontech |
| Total brain | Clontech |
| Cerebellum | Clontech |
| Thyroid | Clontech |
| Thymus | Clontech |
| Mammary gland | Clontech |
| Salivary gland | Clontech |
| Stomach | Clontech |
| Rectum | BioChain Institute |
| Large intestine | BioChain Institute |
| Uterus | Clontech |
| Cervix uteri | BioChain Institute |

**Table 2**

| Tissue | Gene Expression Level^{a} |
|---|---|
| Cartilage in knee osteoarthosis | |
| Cartilage in hip osteoarthosis | |
| Normal knee arthrodial cartilage | |
| Normal hip arthropathy | |
| Costal cartilage | |
| Fat | |
| Skeletal muscle | |
| Heart | |
| Kidney | |
| Adrenal | |
| Liver | |
| Pancreas | |
| Spleen | |
| Trachea | |
| Lung | |
| Total brain | |
| Cerebellum | |
| Thyroid | |
| Thymus | |
| Mammary gland | |
| Salivary gland | |
| Stomach | |
| Rectum | |
| Large intestine | |
| Uterus | |
| Cervix uteri | |

| | |
|---|---|
| ^{a} Gene expression levels were normalized by taking the median of the expression levels of all the genes whose expression was detected with oligonucleotide microarrays as 1. ^{b} The mean is shown (n=5). ND: not detected | |

### EXAMPLE 2

### Gene Expression Analysis

In order to ascertain a group of genes whose expression is increased in cartilage in osteoarthosis (OA cartilage), gene expression analysis was carried out on total RNA extracted from OA cartilage (knee arthropathy: 1 sample; hip arthropathy: 5 samples), normal arthrodial cartilage (knee arthropathy: 1 sample; hip arthropathy: 1 sample) and other 22 normal tissues (1 sample for each tissue) (see Table 3) using oligonucleotide microarrays (Human Genome U95A; U95B, U95C, U95D and U95E; Affimetrix).

Experimental procedures were according to the Expression Analysis Technical Manual issued by Affimetrix. Comparison of gene expression profiles between OA cartilage and normal arthrodial cartilage revealed that the expression of *ANKH* gene was increased in OA cartilage and that the expression level of *ANKH* was the highest in OA cartilage among all of the tissues analyzed (Table 4).

**Table 3**

| Tissue from which RNA was extracted | Seller |
|---|---|
| Cartilage in knee osteoarthosis | Direct Clinical Access |
| Cartilage in hip osteoarthosis | Direct Clinical Access |
| Normal knee arthrodial cartilage | Direct Clinical Access |
| Normal hip arthropathy | Direct Clinical Access |
| Costal cartilage | BioChain Institute |
| Fat | BioChain Institute |
| Skeletal muscle | Clontech |
| Heart | Clontech |
| Kidney | Clontech |
| Adrenal | Clontech |
| Liver | Clontech |
| Pancreas | Clontech |
| Spleen | Clontech |
| Trachea | Clontech |
| Lung | Clontech |
| Total brain | Clontech |
| Cerebellum | Clontech |
| Thyroid | Clontech |
| Thymus | Clontech |
| Mammary gland | Clontech |
| Salivary gland | Clontech |
| Stomach | Clontech |
| Rectum | BioChain Institute |
| Large intestine | BioChain Institute |
| Uterus | Clontech |
| Cervix uteri | BioChain Institute |

**Table 4**

| Tissue | Gene Expression Level^{a} |
|---|---|
| Cartilage in knee osteoarthosis | |
| Cartilage in hip osteoarthosis | |
| Normal knee arthrodial cartilage | |
| Normal hip arthropathy | |
| Costal cartilage | |
| Fat | |
| Skeletal muscle | |
| Heart | |
| Kidney | |
| Adrenal | |
| Liver | |
| Pancreas | |
| Spleen | |
| Trachea | |
| Lung | |
| Total brain | |
| Cerebellum | |
| Thyroid | |
| Thymus | |
| Mammary gland | |
| Salivary gland | |
| Stomach | |
| Rectum | |
| Large intestine | |
| Uterus | |
| Cervix uteri | |

| | |
|---|---|
| ^{a} Gene expression levels were normalized by taking the median of the expression levels of all the genes whose expression was detected with oligonucleotide microarrays as 1. ^{b} The mean is shown (n=5). ND: not detected | |

### EXAMPLE 3

### Gene Expression Analysis

In order to ascertain a group of genes whose expression is increased in cartilage in osteoarthosis (OA cartilage), gene expression analysis was carried out on total RNA extracted from OA cartilage (knee arthropathy: 1 sample; hip arthropathy: 5 samples), normal arthrodial cartilage (knee arthropathy: 1 sample; hip arthropathy: 1 sample) and other 23 normal tissues (1 sample for each tissue) (see Table 5) using oligonucleotide microarrays (Human Genome U95A; U95B, U95C, U95D and U95E; Affimetrix).

Experimental procedures were according to the Expression Analysis Technical Manual issued by Affimetrix. As a result, the expression of *SHOX2* gene was detected only in OA cartilage (Table 6).

**Table 5**

| Tissue from which RNA was extracted | Seller |
|---|---|
| Cartilage in knee osteoarthosis | Direct Clinical Access |
| Cartilage in hip osteoarthosis | Direct Clinical Access |
| Normal knee arthrodial cartilage | Direct Clinical Access |
| Normal hip arthropathy | Direct Clinical Access |
| Costal cartilage | BioChain Institute |
| Fat | BioChain Institute |
| Skeletal muscle | Clontech |
| Heart | Clontech |
| Kidney | Clontech |
| Adrenal | Clontech |
| Liver | Clontech |
| Pancreas | Clontech |
| Spleen | Clontech |
| Trachea | Clontech |
| Lung | Clontech |
| Total brain | Clontech |
| Cerebellum | Clontech |
| Thyroid | Clontech |
| Thymus | Clontech |
| Mammary gland | Clontech |
| Salivary gland | Clontech |
| Stomach | Clontech |
| Rectum | BioChain Institute |
| Large intestine | BioChain Institute |
| Uterus | Clontech |
| Cervix uteri | BioChain Institute |
| Placenta | Clontech |

**Table 6**

| Tissue | Gene Expression Level^{a} |
|---|---|
| Cartilage in knee osteoarthosis | |
| Cartilage in hip osteoarthosis | |
| Normal knee arthrodial cartilage | |
| Normal hip arthropathy | |
| Costal cartilage | |
| Fat | |
| Skeletal muscle | |
| Heart | |
| Kidney | |
| Adrenal | |
| Liver | |
| Pancreas | |
| Spleen | |
| Trachea | |
| Lung | |
| Total brain | |
| Cerebellum | |
| Thyroid | |
| Thymus | |
| Mammary gland | |
| Salivary gland | |
| Stomach | |
| Rectum | |
| Large intestine | |
| Uterus | |
| Cervix uteri | |
| Placenta | |

| | |
|---|---|
| ^{a} Gene expression levels were normalized by taking the median of the expression levels of all the genes whose expression was detected with oligonucleotide microarrays as 1. ^{b} The mean is shown (n=5). ND: not detected | |

### EXAMPLE 4

### Gene Expression Analysis

In order to ascertain a group of genes whose expression is increased in cartilage in osteoarthosis (OA cartilage), gene expression analysis was carried out on total RNA extracted from OA cartilage (knee arthropathy: 1 sample; hip arthropathy: 5 samples), normal arthrodial cartilage (knee arthropathy: 1 sample; hip arthropathy: 1 sample) and other 23 normal tissues (1 sample for each tissue) (see Table 7) using oligonucleotide microarrays (Human Genome U95A; U95B, U95C, U95D and U95E; Affimetrix).

Experimental procedures were according to the Expression Analysis Technical Manual issued by Affimetrix. As a result, the expression of *TASK-4* gene was detected only in OA cartilage (Table 8).

**Table 7**

| Tissue from which RNA was extracted | Seller |
|---|---|
| Cartilage in knee osteoarthosis | Direct Clinical Access |
| Cartilage in hip osteoarthosis | Direct Clinical Access |
| Normal knee arthrodial cartilage | Direct Clinical Access |
| Normal hip arthropathy | Direct Clinical Access |
| Costal cartilage | BioChain Institute |
| Fat | BioChain Institute |
| Skeletal muscle | Clontech |
| Heart | Clontech |
| Kidney | Clontech |
| Adrenal | Clontech |
| Liver | Clontech |
| Pancreas | Clontech |
| Spleen | Clontech |
| Trachea | Clontech |
| Lung | Clontech |
| Total brain | Clontech |
| Cerebellum | Clontech |
| Thyroid | Clontech |
| Thymus | Clontech |
| Mammary gland | Clontech |
| Salivary gland | Clontech |
| Stomach | Clontech |
| Rectum | BioChain Institute |
| Large intestine | BioChain Institute |
| Uterus | Clontech |
| Cervix uteri | BioChain Institute |
| Placenta | Clontech |

**Table 8**

| Tissue | Gene Expression Level^{a} |
|---|---|
| Cartilage in knee osteoarthosis | |
| Cartilage in hip osteoarthosis | |
| Normal knee arthrodial cartilage | |
| Normal hip arthropathy | |
| Costal cartilage | |
| Fat | |
| Skeletal muscle | |
| Heart | |
| Kidney | |
| Adrenal | |
| Liver | |
| Pancreas | |
| Spleen | |
| Trachea | |
| Lung | |
| Total brain | |
| Cerebellum | |
| Thyroid | |
| Thymus | |
| Mammary gland | |
| Salivary gland | |
| Stomach | |
| Rectum | |
| Large intestine | |
| Uterus | |
| Cervix uteri | |
| Placenta | |

| | |
|---|---|
| ^{a} Gene expression levels were normalized by taking the median of the expression levels of all the genes whose expression was detected with oligonucleotide microarrays as 1. ND: not detected | |

### EXAMPLE 5

### Gene Expression Analysis

In order to ascertain a group of genes whose expression is increased in cartilage in osteoarthosis (OA cartilage), gene expression analysis was carried out on total RNA extracted from OA cartilage (knee arthropathy: 1 sample; hip arthropathy: 5 samples), normal arthrodial cartilage (knee arthropathy: 1 sample; hip arthropathy: 1 sample) and other 23 normal tissues (1 sample for each tissue) (see Table 9) using oligonucleotide microarrays (Human Genome U95A; U95B, U95C, U95D and U95E; Affimetrix).

Experimental procedures were according to the Expression Analysis Technical Manual issued by Affimetrix. As a result, the expression of *EphA3* gene was increased specifically in OA cartilage (Table 10).

**Table 9**

| Tissue from which RNA was extracted | Seller |
|---|---|
| Cartilage in knee osteoarthosis | Direct Clinical Access |
| Cartilage in hip osteoarthosis | Direct Clinical Access |
| Normal knee arthrodial cartilage | Direct Clinical Access |
| Normal hip arthropathy | Direct Clinical Access |
| Costal cartilage | BioChain Institute |
| Fat | BioChain Institute |
| Skeletal muscle | Clontech |
| Heart | Clontech |
| Kidney | Clontech |
| Adrenal | Clontech |
| Liver | Clontech |
| Pancreas | Clontech |
| Spleen | Clontech |
| Trachea | Clontech |
| Lung | Clontech |
| Total brain | Clontech |
| Cerebellum | Clontech |
| Thyroid | Clontech |
| Thymus | Clontech |
| Mammary gland | Clontech |
| Salivary gland | Clontech |
| Stomach | Clontech |
| Rectum | BioChain Institute |
| Large intestine | BioChain Institute |
| Uterus | Clontech |
| Cervix uteri | BioChain Institute |
| Placenta | Clontech |

**Table 10**

| Tissue | Gene Expression Level^{a} |
|---|---|
| Cartilage in knee osteoarthosis | |
| Cartilage in hip osteoarthosis | |
| Normal knee arthrodial cartilage | |
| Normal hip arthropathy | |
| Costal cartilage | |
| Fat | |
| Skeletal muscle | |
| Heart | |
| Kidney | |
| Adrenal | |
| Liver | |
| Pancreas | |
| Spleen | |
| Trachea | |
| Lung | |
| Total brain | |
| Cerebellum | |
| Thyroid | |
| Thymus | |
| Mammary gland | |
| Salivary gland | |
| Stomach | |
| Rectum | |
| Large intestine | |
| Uterus | |
| Cervix uteri | |
| Placenta | |

| | |
|---|---|
| ^{a} Gene expression levels were normalized by taking the median of the expression levels of all the genes whose expression was detected with oligonucleotide microarrays as 1. ^{b} The mean is shown (n=5). ND: not detected | |

### EXAMPLE 6

### Gene Expression Analysis

In order to ascertain a group of genes whose expression is increased in cartilage in osteoarthosis (OA cartilage), gene expression analysis was carried out on total RNA extracted from OA cartilage (knee arthropathy: 1 sample; hip arthropathy: 5 samples), normal arthrodial cartilage (knee arthropathy: 1 sample; hip arthropathy: 1 sample) and other 22 normal tissues (1 sample for each tissue) (see Table 11) using oligonucleotide microarrays (Human Genome U95A; U95B, U95C, U95D and U95E; Affimetrix). Experimental procedures were according to the Expression Analysis Technical Manual issued by Affimetrix. As a result, the expression of matrix metalloproteinase 16 gene (*MMP 16*) was increased specifically in OA cartilage (Table 12).

**Table 11**

| Tissue from which RNA was extracted | Seller |
|---|---|
| Cartilage in knee osteoarthosis | Direct Clinical Access |
| Cartilage in hip osteoarthosis | Direct Clinical Access |
| Normal knee arthrodial cartilage | Direct Clinical Access |
| Normal hip arthropathy | Direct Clinical Access |
| Costal cartilage | Direct Clinical Access |
| Fat | BioChain Institute |
| Skeletal muscle | Clontech |
| Heart | Clontech |
| Kidney | Clontech |
| Adrenal | Clontech |
| Liver | Clontech |
| Pancreas | Clontech |
| Spleen | Clontech |
| Trachea | Clontech |
| Lung | Clontech |
| Total brain | Clontech |
| Cerebellum | Clontech |
| Thyroid | Clontech |
| Thymus | Clontech |
| Mammary gland | Clontech |
| Salivary gland | Clontech |
| Stomach | Clontech |
| Rectum | BioChain Institute |
| Large intestine | BioChain Institute |
| Uterus | Clontech |
| Cervix uteri | BioChain Institute |

**Table 12**

| Tissue | Gene Expression Level^{a} |
|---|---|
| Cartilage in knee osteoarthosis | |
| Cartilage in hip osteoarthosis | |
| Normal knee arthrodial cartilage | |
| Normal hip arthropathy | |
| Costal cartilage | |
| Fat | |
| Skeletal muscle | |
| Heart | |
| Kidney | |
| Adrenal | |
| Liver | |
| Pancreas | |
| Spleen | |
| Trachea | |
| Lung | |
| Total brain | |
| Cerebellum | |
| Thyroid | |
| Thymus | |
| Mammary gland | |
| Salivary gland | |
| Stomach | |
| Rectum | |
| Large intestine | |
| Uterus | |
| Cervix uteri | |

| | |
|---|---|
| ^{a} Gene expression levels were normalized by taking the median of the expression levels of all the genes whose expression was detected with oligonucleotide microarrays as 1. ^{b} The mean is shown (n=5). ND: not detected | |

### EXAMPLE 7

### Cartilage Differentiation-Dependent Expression of DIO2

Human mesenchymal stem cells (hMSC; Clontech) were plated on 24-well plates at 10⁵ cells/well and cultured in DMEM high glucose medium (Gibco) containing 6.3 µg/ml insulin, 6.3 µg/ml transferin, 6.3 ng/ml selenious acid, 1.3 mg/ml bovine serum albumin (BSA), 5.4 µg/ml linolenic acid (these components were contained in ITS+ Premix; Becton Dickinson Labware), 40 µg/ml proline, 100 nM dexamethason, 50 µg/ml ascorbic acid phosphate magnesium salt n-hydrate (Wako), 10 ng/ml recombinant human TGF- β3 (Genzyme/Techne) and 200 ng/ml recombinant human BMP-4 (Research & Development Systems) uder oxygen partial pressure of 3%. On day 0, 3, 6 13 and 20 after starting the cultivation, the expression of hMSC was analyzed with oligonucleotide microarrays (Affimetrix). The results revealed that the expression peak of the transcription factor SOX9 which is essential for the differentiation from undifferentiated stem cells to proliferating cartilage cells is from day 3 to day 6 (Fig. 1b); that the expression peak of type II collagen produced by proliferating cartilage cells is from day 6 to day 13 (Fig. 1c); and that the expression peak of osteocalcin produced by hypertrophic cartilage cells is from day 13 to day 20 (Fig. 1d). During this process of hMSC cartilage differentiation, the expression of DIO2 was first recognized on day 3. Then, the expression level gradually increased and reached the highest on day 13. On day 20, the expression level of DIO2 was almost equal to or slightly lower than the level on day 13 (Fig. 1a).

This time course of expression indicated that DIO2 is expressed in cartilage and that the expression level of *DIO2* increases in a differentiation stage-dependent manner. It was suggested that DIO2 functions during the hypertrophy stage or immediately before that.

### EXAMPLE 8

### Induction of Alkaline Phosphatase Activity in Normal Human Articular Chondrocytes of Knee by T3

Normal human articular chondrocytes of knee (NHAC-kn; Clonetics) were plated on gelatin-coated 24-well plates (Iwaki) at 2 x 10⁵ cells/well and cultured in DMEM high glucose medium (Gibco) containing 1 µg/ml insulin, 1 µg/ml transferin, 1 ng/ml selenious acid (these components were contained in ITS; BD Bioscience), 1 mg/ml BSA, 50 µg/ml ascorbic acid phosphate magnesium salt n-hydrate (Wako) and 5 mM *β* - glycerophosphate in a CO₂ incubator under 5% CO₂ at 37°C with addition of 0.05-50 nM T3 (triiodothyronine) (Sigma) if necessary. After 6 days, the culture broth was removed from cells and 200 *µ*l of 10 mM Tris-HCl (pH 7.4) containing 0.1% Triton X-100 was added to each well. Then, cells were shaken at 4°C for 1 hour on a horizontal shaker to thereby extract proteins from the cell layer. The alkaline phosphatase (AP) activity of the extract was determined from the yield of p-nitrophenol using p-nitrophenylphosphate as a substrate (Sigma). The protein content in the extract was measured with BCA protein assay reagent (Pierce) to thereby calculate the AP activity per a specific amount of protein.

The results are shown in Fig. 2.

These results demonstrated that AP activity, which is a cartilage calcification factor in OA, is induced in a T3 concentration-dependent manner.

### EXAMPLE 9

### Induction of MMP13 gene expression in Normal Human Articular Chondrocytes of Knee by T3

Normal human articular chondrocytes of knee (NHAC-kn; Clonetics) were plated on gelatin-coated 96-well plates (Iwaki) at 2 x 10⁵ cells/well and cultured in DMEM high glucose medium (Gibco) containing 1 µg/ml insulin, 1 µg/ml transferin, 1 ng/ml selenious acid (these components were contained in ITS; BD Bioscience) and 1 mg/ml BSA, and supplemented with 5 mM β-glycerophosphate (Sigma), 50 µg/ml ascorbic acid phosphate magnesium salt n-hydrate (Wako) and, if necessary, 0.1-10 nM T3 (triiodothyronine) (Sigma), in a CO₂ incubator under 5% CO₂ at 37°C. After 6 days, RNA was extracted from cells using RNeasy (Qiagen) according to the attached protocol. Using the total RNA as a template, cDNA was synthesized from random primers with TaqManreverse transcription reagent (Applied Biosystems). Subsequently, 2 *µ*l of the reaction solution containing cDNA was mixed with a 2 *µ*l reaction solution containing 0.34 *µ*M of two primers (SEQ ID NO: 15 and SEQ ID NO: 16) and 1 x PCR Master Mix (Applied Biosystems) and subjected to PCR. The thermal conditions were as follows: at 50°C for 2 min, at 95°C for 10 min and 35 cycles of (at 95°C for 15 sec and at 60°C for 1 min).

The PCR was performed with ABI7700 sequence detector (Applied Biosystems). The number of copies of MMP 13 cDNA was measured by real-time monitoring the amount of the PCR product. The number of copies of β-actin cDNA was also measured in the same manner using TaqMan β-actin control reagent (Applied Biosystems) and used as internal standard.

The results are shown in Fig. 3. The number of copies of MMP13 cDNA normalized with β-actin is shown as the average of three wells with the standard deviation.

These results demonstrated that mRNA of MMP13, which is a cartilage degradation factor in OA, is induced in a T3 concentration-dependent manner.

### EXAMPLE 10

### Construction of DIO Expression Plasmid and Measurement of Thyroid Hormone Deiodinase Activity

Using human thyroid-derived cDNA (Marathon Ready cDNA; Clontech) as a template, the following sequences were amplified by PCR: the amino acid coding sequence (cds) of human DIO1 (type 1 thyroid hormone deiodinase) using primer 1 (SEQ ID NO: 17) and primer 2 (SEQ ID NO: 18); DIO1 selenocystein insertion sequence (SECIS) using primer 3 (SEQ ID NO: 19) and primer 4 (SEQ ID NO: 20); human DIO2 cds using primer 5 (SEQ ID NO: 21) and primer 6 (SEQ ID NO: 22); and DIO2 SECIS (Buettner et al., 1998) using primer 7 (SEQ ID NO: 23) and primer 8 (SEQ ID NO: 24).

The resultant DNAs for human DIO1 cds and human DIO2 cds were inserted into pcDNA3.1 (Invitrogen) individually to thereby obtain plasmids pTB2240 and pTB2242. Further, the resultant DNAs for human DIO1 SECIS and human DIO2 SECIS were inserted into pCR2.1 (Invitrogen) individually to thereby obtain plasmids pTB2241 and pTB2244. DIO1 SECIS and DIO2 SECIS were cut out with restriction enzymes Sca I and Not I (Takara Shuzo) and introduced into DIO1 or DIO2 coding sequence-inserted plasmid pretreated with restriction enzymes Eco RV and Not I (Takara Shuzo) to thereby construct DIO1 expression plasmid pTB2248 and DIO2 expression plasmid pTB2249.

COS-7 cells (3 x 10⁵) were plated in 6 cm petri dishes. After one day, 2 *µ*g each of DIO1 expression plasmid pTB2248, DIO2 expression plasmid pTB2249 or DIO2 cds-inserted plasmid pTB2242 was added to the cells together with 6 *µ*l of transfection reagent (FuGENE6; Roche) according to the attached manual. After two days, cells were recovered by trypsin-EDTA (Gibco/BRL) treatment and sonicated in a buffer [0.1 M potassium phosphate-1 mM EDTA (pH 6.9), 10 mM DTT, 0.25 M sucrose] to prepare disrupted cells. In the presence or absence of 1 mM PTU (propylthiouracil; Sigma) or 5 *µ* M rT3 (riverse triiodothyronine; Sigma), 0.05-5 *µ*M T4 (thyroxin; Sigma) was added to the above-disrupted cells, which were then incubated at 37°C for one hour. For the quantitative determination of the produced T3, total T3 RIA (radio immunoassay) kit (Diagnostic Products Corporation) was used. First, 100 *µ*l of the sample and ¹²⁵I-labeled T3 reagent were mixed in a T3 antibody-immobilized tube and incubated at 37°C for 2 hours. Then, the reaction solution was removed from the tube, and the radioactivity remaining in the tube was measured with a γ counter.

As a result, as shown in Fig. 4a, T3 production was recognized in the disrupted cells into which DIO2 expression plasmid pTB2249 was introduced. Since T3 production ability was not recognized in the disrupted cells into which pTB2242 having DIO2 cds alone was introduced, the necessity of SECIS was confirmed. On the other hand, definite T3 production was not recognized under the above-described reaction conditions in the disrupted cells into which DIO1 expression plasmid pTB2248 (having an enzyme activity similar to that of DIO2 expression plasmid) was introduced. However, by increasing the T4 concentration to 5 *µ*M, T3 production was clearly recognized as shown in Fig. 4b. Further, by adding a DIO1-specific inhibitor PTU (Sigma), the activity was completely inhibited. The DIO2 activity of pTB2249 was partially inhibited by the addition of rT3 (5 *µ*M) that was expected to inhibit DIO2 through competition with T4, but the addition of PTU influenced little on the DIO2 activity (Fig. 4c).

### EXAMPLE 11

### Screening

COS-7 cells (3 x 10⁵) are plated in 6 cm petri dishes. After one day, 2 *µ*g each of DIO1 expression plasmid pTB2248 or DIO2 expression plasmid pTB2249 is added to the cells together with 6 *µ*l of transfection reagent (FuGENE6; Roche) according to the attached manual. After two days, cells are recovered by trypsin-EDTA (Gibco/BRL) treatment and sonicated in a buffer [0.1 M potassium phosphate-1 mM EDTA (pH 6.9), 10 mM DTT, 0.25 M sucrose] to prepare disrupted cells. In the presence or absence of a test compound, 0.05-5 µM T4 (thyroxin; Sigma) is added to the above-disrupted cells, which are then incubated at 37°C for one hour. The produced T3 is quantitatively determined using total T3 RIA (radio immunoassay) kit (Diagnostic Products Corporation). Briefly, first, 100 *µ*l of the sample and ¹²⁵I-labeled T3 reagent are mixed in a T3 antibody-immobilized tube and incubated at 37°C for 2 hours. Then, the reaction solution is removed from the tube, and the radioactivity remaining in the tube is measured with a γ counter. It is possible to screen for those compounds having DIO2-selective inhibition activity by comparing T3 yields.

### INDUSTRIAL APPLICABILITY

The protein used in the invention is a diagnostic marker for bone and/or arthropathy diseases. Therefore, compounds or salts thereof that regulate the activity of the protein and neutralizing antibodies that regulate the activity of the protein may be used as prophylactic and/or therapeutic agents for bone and/or arthropathy diseases such as chondrodystrophy, osteodystrophy, osteoporosis, osteoarthosis, rheumatoid arthritis, arthritis, synovitis, metabolic arthropathy or arthropathy disorder caused by sports (e.g. tennis elbow). Further, the antisense polynucleotide of the invention is capable of regulating the expression of the protein used in the invention and thus may be used as a prophylactic and/or therapeutic agent for bone and/or arthropathy diseases such as chondrodystrophy, osteodystrophy, osteoporosis, osteoarthosis, rheumatoid arthritis, arthritis, synovitis, metabolic arthropathy or arthropathy disorder caused by sports (e.g. tennis elbow).

## Claims

1. A prophylactic and/or therapeutic agent for bone and/or arthropathy diseases, comprising a compound or a salt thereof that regulates the activity of a protein comprising an amino acid sequence identical or substantially identical to the amino acid sequence represented by SEQ ID NO: 1, SEQ ID NO: 3, SEQ ID NO: 5, SEQ ID NO: 7, SEQ ID NO: 9, SEQ ID NO: 11 or SEQ ID NO: 13, a partial peptide of the protein, or a salt of the protein or the partial peptide.

2. The prophylactic and/or therapeutic agent according to claim 1, comprising a compound or a salt thereof that inhibits the activity of a protein comprising an amino acid sequence identical or substantially identical to the amino acid sequence represented by SEQ ID NO: 1, SEQ ID NO: 3, SEQ ID NO: 5, SEQ ID NO: 7, SEQ ID NO: 9, SEQ ID NO: 11 or SEQ ID NO: 13, a partial peptide of the protein, or a salt of the protein or the partial peptide.

3. The prophylactic and/or therapeutic agent according to claim 2, which is a compound or a salt thereof that inhibits the activity of a protein consisting of the amino acid sequence represented by SEQ ID NO: 1, a partial peptide of the protein, or a salt of the protein or the partial peptide.

4. A prophylactic and/or therapeutic agent for bone and/or arthropathy diseases, comprising a compound or a salt thereof that regulates the expression of the gene of a protein comprising an amino acid sequence identical or substantially identical to the amino acid sequence represented by SEQ ID NO: 1, SEQ ID NO: 3, SEQ ID NO: 5, SEQ ID NO: 7, SEQ ID NO: 9, SEQ ID NO: 11 or SEQ ID NO: 13, a partial peptide of the protein, or a salt of the protein or the partial peptide.

5. A prophylactic and/or therapeutic agent for bone and/or arthropathy diseases, comprising a compound or a salt thereof that inhibits the expression of the gene of a protein comprising an amino acid sequence identical or substantially identical to the amino acid sequence represented by SEQ ID NO: 1, SEQ ID NO: 3, SEQ ID NO: 5, SEQ ID NO: 7, SEQ ID NO: 9, SEQ ID NO: 11 or SEQ ID NO: 13, a partial peptide of the protein, or a salt of the protein or the partial peptide.

6. The prophylactic and/or therapeutic agent according to claim 5, which is a compound or a salt thereof that regulates the expression of the gene of a protein consisting of the amino acid sequence represented by SEQ ID NO: 1, a partial peptide of the protein, or a salt of the protein or the partial peptide.

7. A prophylactic and/or therapeutic agent for bone and/or arthropathy diseases, comprising an antisense polynucleotide comprising a nucleotide sequence, or a part thereof, complementary or substantially complementary to the nucleotide sequence of a DNA encoding a protein, or a partial peptide thereof, comprising an amino acid sequence identical or substantially identical to the amino acid sequence represented by SEQ ID NO: 1, SEQ ID NO: 3, SEQ ID NO: 5, SEQ ID NO: 7, SEQ ID NO: 9, SEQ ID NO: 11 or SEQ ID NO: 13.

8. A prophylactic and/or therapeutic agent for bone and/or arthropathy diseases, comprising an antibody against a protein comprising an amino acid sequence identical or substantially identical to the amino acid sequence represented by SEQ ID NO: 1, SEQ ID NO: 3, SEQ ID NO: 5, SEQ ID NO: 7, SEQ ID NO: 9, SEQ ID NO: 11 or SEQ ID NO: 13, a partial peptide of the protein, or a salt of the protein or the partial peptide.

9. The prophylactic and/or therapeutic agent according to claim 1, 4, 7 or 8, wherein the bone and/or arthropathy disease is chondrodystrophy, osteodystrophy, osteoporosis, osteoarthosis, rheumatoid arthritis, arthritis, synovitis, metabolic arthropathy or arthropathy disorder caused by sports.

10. A diagnostic agent for bone and/or arthropathy diseases, comprising an antibody against a protein comprising an amino acid sequence identical or substantially identical to the amino acid sequence represented by SEQ ID NO: 1, SEQ ID NO: 3, SEQ ID NO: 5, SEQ ID NO: 7, SEQ ID NO: 9, SEQ ID NO: 11 or SEQ ID NO: 13, a partial peptide of the protein, or a salt of the protein or the partial peptide.

11. A diagnostic agent for bone and/or arthropathy diseases, comprising a DNA encoding a protein, or a partial peptide thereof, comprising an amino acid sequence identical or substantially identical to the amino acid sequence represented by SEQ ID NO: 1, SEQ ID NO: 3, SEQ ID NO: 5, SEQ ID NO: 7, SEQ ID NO: 9, SEQ ID NO: 11 or SEQ ID NO: 13.

12. The diagnostic agent according to claim 10 or 11, wherein the bone and/or arthropathy disease is chondrodystrophy, osteodystrophy, osteoporosis, osteoarthosis, rheumatoid arthritis, arthritis, synovitis, metabolic arthropathy or arthropathy disorder caused by sports.

13. A method of screening for a prophylactic and/or therapeutic agent for bone and/or arthropathy diseases, comprising using a protein comprising an amino acid sequence identical or substantially identical to the amino acid sequence represented by SEQ ID NO: 1, SEQ ID NO: 3, SEQ ID NO: 5, SEQ ID NO: 7, SEQ ID NO: 9, SEQ ID NO: 11 or SEQ ID NO: 13, a partial peptide of the protein, or a salt of the protein or the partial peptide.

14. The screening method according to claim 13, wherein a protein consisting of the amino acid sequence represented by SEQ ID NO: 1, a partial peptide of the protein, or a salt of the protein or the partial peptide is used.

15. A kit for screening for a prophylactic and/or therapeutic agent for bone and/or arthropathy diseases, containing a protein comprising an amino acid sequence identical or substantially identical to the amino acid sequence represented by SEQ ID NO: 1, SEQ ID NO: 3, SEQ ID NO: 5, SEQ ID NO: 7, SEQ ID NO: 9, SEQ ID NO: 11 or SEQ ID NO: 13, a partial peptide of the protein, or a salt of the protein or the partial peptide.

16. A prophylactic and/or therapeutic agent for bone and/or arthropathy diseases, obtainable by using the screening method according to claim 13 or the screening kit according to claim 15.

17. A method of screening for a prophylactic and/or therapeutic agent for bone and/or arthropathy diseases, comprising using a DNA encoding a protein, or a partial peptide thereof, comprising an amino acid sequence identical or substantially identical to the amino acid sequence represented by SEQ ID NO: 1, SEQ ID NO: 3, SEQ ID NO: 5, SEQ ID NO: 7, SEQ ID NO: 9, SEQ ID NO: 11 or SEQ ID NO: 13.

18. The screening method according to claim 17, wherein a DNA encoding a protein, or a partial peptide thereof, consisting of the amino acid sequence represented by SEQ ID NO: 1 is used.

19. A kit for screening for a prophylactic and/or therapeutic agent for bone and/or arthropathy diseases, containing a DNA encoding a protein, or a partial peptide thereof, comprising an amino acid sequence identical or substantially identical to the amino acid sequence represented by SEQ ID NO: 1, SEQ ID NO: 3, SEQ ID NO: 5, SEQ ID NO: 7, SEQ ID NO: 9, SEQ ID NO: 11 or SEQ ID NO: 13.

20. A prophylactic and/or therapeutic agent for bone and/or arthropathy diseases, obtainable by using the screening method according to claim 17 or the screening kit according to claim 19.

21. A method of preventing and/or treating bone and/or arthropathy diseases, comprising administering to a mammal an effective amount of a compound or a salt thereof that regulates the activity of a protein comprising an amino acid sequence identical or substantially identical to the amino acid sequence represented by SEQ ID NO: 1, SEQ ID NO: 3, SEQ ID NO: 5, SEQ ID NO: 7, SEQ ID NO: 9, SEQ ID NO: 11 or SEQ ID NO: 13, a partial peptide of the protein, or a salt of the protein or the partial peptide.

22. A method of preventing and/or treating bone and/or arthropathy diseases, comprising administering to a mammal an effective amount of a compound or a salt thereof that regulates the expression of the gene of a protein comprising an amino acid sequence identical or substantially identical to the amino acid sequence represented by SEQ ID NO: 1, SEQ ID NO: 3, SEQ ID NO: 5, SEQ ID NO: 7, SEQ ID NO: 9, SEQ ID NO: 11 or SEQ ID NO: 13, a partial peptide of the protein, or a salt of the protein or the partial peptide.

23. Use of a compound or a salt thereof that regulates the activity of a protein comprising an amino acid sequence identical or substantially identical to the amino acid sequence represented by SEQ ID NO: 1, SEQ ID NO: 3, SEQ ID NO: 5, SEQ ID NO: 7, SEQ ID NO: 9, SEQ ID NO: 11 or SEQ ID NO: 13, a partial peptide of the protein, or a salt of the protein or the partial peptide, for manufacturing a prophylactic and/or therapeutic agent for bone and/or arthropathy diseases.

24. Use of a compound or a salt thereof that regulates the expression of the gene of a protein comprising an amino acid sequence identical or substantially identical to the amino acid sequence represented by SEQ ID NO: 1, SEQ ID NO: 3, SEQ ID NO: 5, SEQ ID NO: 7, SEQ ID NO: 9, SEQ ID NO: 11 or SEQ ID NO: 13, a partial peptide of the protein, or a salt of the protein or the partial peptide, for manufacturing a prophylactic and/or therapeutic agent for bone and/or arthropathy diseases.

25. A prophylactic and/or therapeutic agent for bone and/or arthropathy diseases, comprising a compound or a salt thereof that has a regulating effect on thyroxin 5'-deiodinase activity, pyrophosphate transport activity, potassium ion transport activity, or the signal transduction activity or ligand-binding activity of Eph receptor.

26. A prophylactic and/or therapeutic agent for bone and/or arthropathy diseases, comprising a compound or a salt thereof that has a regulating effect on the expression of thyroxin 5'-deiodinase, pyrophosphate transporter, potassium ion transporter or Eph receptor.


SEQUENCE LISTING

<110> Takeda Chemical Industries, Ltd.

<120> Preventing and treating agent for bone or joint diseases

<130> P02-0099PCT

<150> JP2001-273914
<151> 2001-09-10

<150> JP2001-281472
<151> 2001-09-17

<150> JP2001-300417
<151> 2001-09-28

<150> JP2001-300289
<151> 2001-09-28

<150> JP2001-300347
<151> 2001-09-28

<150> JP2001-303390
<151> 2001-09-28

<160> 24

<210> 1
<211> 273
<212> PRT
<213> Human

<220>
<221> act site
<222> (133)..(133)
<223> Xaa is selenocysteine

<220>
<221> act site
<222> (266)..(266)
<223> Xaa is selenocysteine

<400> 1
Met Gly Ile Leu Ser Val Asp Leu Leu Ile Thr Leu Gln Ile Leu Pro
                5                   10                  15
Val Phe Phe Ser Asn Cys Leu Phe Leu Ala Leu Tyr Asp Ser Val Ile
            20                  25                  30
Leu Leu Lys His Val Val Leu Leu Leu Ser Arg Ser Lys Ser Thr Arg
        35                  40                  45
Gly Glu Trp Arg Arg Met Leu Thr Ser Glu Gly Leu Arg Cys Val Trp
    50                  55                  60
Lys Ser Phe Leu Leu Asp Ala Tyr Lys Gln Val Lys Leu Gly Glu Asp
65                  70                  75                  80
Ala Pro Asn Ser Ser Val Val His Val Ser Ser Thr Glu Gly Gly Asp
                85                  90                  95

Asn Ser Gly Asn Gly Thr Gln Glu Lys Ile Ala Glu Gly Ala Thr Cys
            100                 105                 110
His Leu Leu Asp Phe Ala Ser Pro Glu Arg Pro Leu Val Val Asn Phe
        115                 120                 125
Gly Ser Ala Thr Xaa Pro Pro Phe Thr Ser Gln Leu Pro Ala Phe Arg
    130                 135                 140
Lys Leu Val Glu Glu Phe Ser Ser Val Ala Asp Phe Leu Leu Val Tyr
145                 150                 155                 160
Ile Asp Glu Ala His Pro Ser Asp Gly Trp Ala Ile Pro Gly Asp Ser
                165                 170                 175
Ser Leu Ser Phe Glu Val Lys Lys His Gln Asn Gln Glu Asp Arg Cys
            180                 185                 190
Ala Ala Ala Gln Gln Leu Leu Glu Arg Phe Ser Leu Pro Pro Gln Cys
        195                 200                 205
Arg Val Val Ala Asp Arg Met Asp Asn Asn Ala Asn Ile Ala Tyr Gly
    210                 215                 220
Val Ala Phe Glu Arg Val Cys Ile Val Gln Arg Gln Lys Ile Ala Tyr
225                 230                 235                 240
Leu Gly Gly Lys Gly Pro Phe Ser Tyr Asn Leu Gln Glu Val Arg His
                245                 250                 255
Trp Leu Glu Lys Asn Phe Xaa Lys Arg Sec Lys Lys Thr Arg Leu Ala
            260                 265                 270
Gly

<210> 2
<211> 819
<212> DNA
<213> Human

<400> 2
atgggcatcc tcagcgtaga cttgctgatc acactgcaaa ttctgccagt ttttttctcc   60
aactgcctct tcctggctct ctatgactcg gtcattctgc tcaagcacgt ggtgctgctg   120
ttgagccgct ccaagtccac tcgcggagag tggcggcgca tgctgacctc agagggactg   180
cgctgcgtct ggaagagctt cctcctcgat gcctacaaac aggtgaaatt gggtgaggat   240
gcccccaatt ccagtgtggt gcatgtctcc agtacagaag gaggtgacaa cagtggcaat   300
ggtacccagg agaagatagc tgagggagcc acatgccacc ttcttgactt tgccagccct   360
gagcgcccac tagtggtcaa ctttggctca gccacttgac ctcctttcac gagccagctg   420
ccagccttcc gcaaactggt ggaagagttc tcctcagtgg ctgacttcct gctggtctac   480
attgatgagg ctcatccatc agatggctgg gcgataccgg gggactcctc tttgtctttt   540
gaggtgaaga agcaccagaa ccaggaagat cgatgtgcag cagcccagca gcttctggag   600
cgtttctcct tgccgcccca gtgccgagtt gtggctgacc gcatggacaa taacgccaac   660
atagcttacg gggtagcctt tgaacgtgtg tgcattgtgc agagacagaa aattgcttat   720
ctgggaggaa agggcccctt ctcctacaac cttcaagaag tccggcattg gctggagaag   780
aatttcagca agagatgaaa gaaaactaga ttagctggt                          819

<210> 3
<211> 492
<212> PRT
<213> Human

<400> 3
Met Val Lys Phe Pro Ala Leu Thr His Tyr Trp Pro Leu Ile Arg Phe
                5                   10                  15
Leu Val Pro Leu Gly Ile Thr Asn Ile Ala Ile Asp Phe Gly Glu Gln
            20                  25                  30
Ala Leu Asn Arg Gly Ile Ala Ala Val Lys Glu Asp Ala Val Glu Met

        35                  40                  45
Leu Ala Ser Tyr Gly Leu Ala Tyr Ser Leu Met Lys Phe Phe Thr Gly
    50                  55                  60
Pro Met Ser Asp Phe Lys Asn Val Gly Leu Val Phe Val Ser Ser Lys
65                  70                  75                  80
Arg Asp Arg Thr Lys Ala Val Leu Cys Met Val Val Ala Gly Ala Ile
                85                  90                  95
Ala Ala Val Phe His Thr Leu Ile Ala Tyr Ser Asp Leu Gly Tyr Tyr
            100                 105                 110
Ile Ile Asn Lys Leu His His Val Asp Glu Ser Val Gly Ser Lys Thr
        115                 120                 125
Arg Arg Ala Phe Leu Tyr Leu Ala Ala Phe Pro Phe Met Asp Ala Met
    130                 135                 140
Ala Trp Thr His Ala Gly Ile Leu Leu Lys His Lys Tyr Ser Phe Leu
145                 150                 155                 160
Val Gly Cys Ala Ser Ile Ser Asp Val Ile Ala Gln Val Val Phe Val
                165                 170                 175
Ala Ile Leu Leu His Ser His Leu Glu Cys Arg Glu Pro Leu Leu Ile
            180                 185                 190
Pro Ile Leu Ser Leu Tyr Met Gly Ala Leu Val Arg Cys Thr Thr Leu
        195                 200                 205
Cys Leu Gly Tyr Tyr Lys Asn Ile His Asp Ile Ile Pro Asp Arg Ser
    210                 215                 220
Gly Pro Glu Leu Gly Gly Asp Ala Thr Ile Arg Lys Met Leu Ser Phe
225                 230                 235                 240
Trp Trp Pro Leu Ala Leu Ile Leu Ala Thr Gln Arg Ile Ser Arg Pro
                245                 250                 255
Ile Val Asn Leu Phe Val Ser Arg Asp Leu Gly Gly Ser Ser Ala Ala
            260                 265                 270
Thr Glu Ala Val Ala Ile Leu Thr Ala Thr Tyr Pro Val Gly His Met
        275                 280                 285
Pro Tyr Gly Trp Leu Thr Glu Ile Arg Ala Val Tyr Pro Ala Phe Asp
    290                 295                 300
Lys Asn Asn Pro Ser Asn Lys Leu Val Ser Thr Ser Asn Thr Val Thr
305                 310                 315                 320
Ala Ala His Ile Lys Lys Phe Thr Phe Val Cys Met Ala Leu Ser Leu
                325                 330                 335
Thr Leu Cys Phe Val Met Phe Trp Thr Pro Asn Val Ser Glu Lys Ile
            340                 345                 350
Leu Ile Asp Ile Ile Gly Val Asp Phe Ala Phe Ala Glu Leu Cys Val
        355                 360                 365
Val Pro Leu Arg Ile Phe Ser Phe Phe Pro Val Pro Val Thr Val Arg
    370                 375                 380
Ala His Leu Thr Gly Trp Leu Met Thr Leu Lys Lys Thr Phe Val Leu
385                 390                 395                 400
Ala Pro Ser Ser Val Leu Arg Ile Ile Val Leu Ile Ala Ser Leu Val
                405                 410                 415
Val Leu Pro Tyr Leu Gly Val His Gly Ala Thr Leu Gly Val Gly Ser
            420                 425                 430
Leu Leu Ala Gly Phe Val Gly Glu Ser Thr Met Val Ala Ile Ala Ala
        435                 440                 445
Cys Tyr Val Tyr Arg Lys Gln Lys Lys Lys Met Glu Asn Glu Ser Ala
    450                 455                 460
Thr Glu Gly Glu Asp Ser Ala Met Thr Asp Met Pro Pro Thr Glu Glu
465                 470                 475                 480
Val Thr Asp Ile Val Glu Met Arg Glu Glu Asn Glu

                485                 490

<210> 4
<211> 1476
<212> DNA
<213> Human

<220>
<221> unsure
<222> (126)..(126)
<223> n stands any base

<400> 4
atggtgaaat tcccggcgct cacgcactac tggcccctga tccggttctt ggtgcccctg 60
ggcatcacca acatagccat cgacttcggg gagcaggcct tgaaccgggg cattgctgct 120
gtcaaggagg atgcagtcga gatgctggcc agctacgggc tggcgtactc cctcatgaag 180
ttcttcacgg gtcccatgag tgacttcaaa aatgtgggcc tggtgtttgt gagcagcaag 240
agagacagga ccaaagccgt cctgtgtatg gtggtggcag gggccatcgc tgctgtcttt 300
cacacnctga tagcttatag tgatttagga tactacatta tcaataaact gcaccatgtg 360
gacgagtcgg tggggagcaa gacgagaagg gccttcctgt acctcgccgc ctttcctttc 420
atggacgcaa tggcatggac ccatgctggc attctcttaa aacacaaata cagtttcctg 480
gtgggatgtg cctcaatctc agatgtcata gctcaggttg tttttgtagc cattttgctt 540
cacagtcacc tggaatgccg ggagcccctg ctcatcccga tcctctcctt gtacatgggc 600
gcacttgtgc gctgcaccac cctgtgcctg ggctactaca agaacattca cgacatcatc 660
cctgacagaa gtggcccgga gctgggggga gatgcaacaa taagaaagat gctgagcttc 720
tggtggcctt tggctctaat tctggccaca cagagaatca gtcggcctat tgtcaacctc 780
tttgtttccc gggaccttgg tggcagttct gcagccacag aagcagtggc gattttgaca 840
gccacatacc ctgtgggtca catgccatac ggctggttga cggaaatccg tgctgtgtat 900
cctgctttcg acaagaataa ccccagcaac aaactggtga gcacgagcaa cacagtcacg 960
gcagcccaca tcaagaagtt caccttcgtc tgcatggctc tgtcactcac gctctgtttc 1020
gtgatgtttt ggacacccaa cgtgtctgag aaaatcttga tagacatcat cggagtggac 1080
tttgcctttg cagaactctg tgttgttcct ttgcggatct tctccttctt cccagttcca 1140
gtcacagtga gggcgcatct caccgggtgg ctgatgacac tgaagaaaac cttcgtcctt 1200
gcccccagct ctgtgctgcg gatcatcgtc ctcatcgcca gcctcgtggt cctaccctac 1260
ctgggggtgc acggtgcgac cctgggcgtg ggctccctcc tggcgggctt tgtgggagaa 1320
tccaccatgg tcgccatcgc tgcgtgctat gtctaccgga agcagaaaaa gaagatggag 1380
aatgagtcgg ccacggaggg ggaagactct gccatgacag acatgcctcc gacagaggag 1440
gtgacagaca tcgtggaaat gagagaggag aatgaa 1476

<210> 5
<211> 331
<212> PRT
<213> Human

<400> 5
Met Glu Glu Leu Thr Ala Phe Val Ser Lys Ser Phe Asp Gln Lys Val
1               5                   10                  15
Lys Glu Lys Lys Glu Ala Ile Thr Tyr Arg Glu Val Leu Glu Ser Gly
            20                  25                  30
Pro Leu Arg Gly Ala Lys Glu Pro Thr Gly Cys Thr Glu Ala Gly Arg
        35                  40                  45
Asp Asp Arg Ser Ser Pro Ala Val Arg Ala Ala Gly Gly Gly Gly Gly
    50                  55                  60
Gly Gly Gly Gly Gly Gly Gly Gly Gly Gly Gly Gly Gly Val Gly Gly
65                  70                  75                  80
Gly Gly Ala Gly Gly Gly Ala Gly Gly Gly Arg Ser Pro Val Arg Glu

                85                  90                  95
Leu Asp Met Gly Ala Ala Glu Arg Ser Arg Glu Pro Gly Ser Pro Arg
            100                 105                 110
Leu Thr Glu Val Ser Pro Glu Leu Lys Asp Arg Lys Asp Asp Ala Lys
        115                 120                 125
Gly Met Glu Asp Glu Gly Gln Thr Lys Ile Lys Gln Arg Arg Ser Arg
    130                 135                 140
Thr Asn Phe Thr Leu Glu Gln Leu Asn Glu Leu Glu Arg Leu Phe Asp
145                 150                 155                 160
Glu Thr His Tyr Pro Asp Ala Phe Met Arg Glu Glu Leu Ser Gln Arg
                165                 170                 175
Leu Gly Leu Ser Glu Ala Arg Val Gln Val Trp Phe Gln Asn Arg Arg
            180                 185                 190
Ala Lys Cys Arg Lys Gln Glu Asn Gln Leu His Lys Gly Val Leu Ile
        195                 200                 205
Gly Ala Ala Ser Gln Phe Glu Ala Cys Arg Val Ala Pro Tyr Val Asn
    210                 215                 220
Val Gly Ala Leu Arg Met Pro Phe Gln Gln Asp Ser His Cys Asn Val
225                 230                 235                 240
Thr Pro Leu Pro Phe Gln Val Gln Ala Gln Leu Gln Leu Asp Ser Ala
                245                 250                 255
Val Ala His Ala His His His Leu His Pro His Leu Ala Ala His Ala
            260                 265                 270
Pro Tyr Met Met Phe Pro Ala Pro Pro Phe Gly Leu Pro Leu Ala Thr
        275                 280                 285
Leu Ala Ala Asp Ser Ala Ser Ala Ala Ser Val Val Ala Ala Ala Ala
    290                 295                 300
Ala Ala Lys Thr Thr Ser Lys Asp Ser Ser Ile Ala Asp Leu Arg Leu
305                 310                 315                 320
Lys Ala Lys Lys His Ala Ala Ala Leu Gly Leu
                325                 330

<210> 6
<211> 993
<212> DNA
<213> Human

<400> 6
atggaagaac ttacggcgtt cgtctccaag tcttttgacc agaaagtgaa ggagaagaag    60
gaggcgatca cgtaccggga ggtgctggag agcgggccgc tgcgcggggc caaggagccg   120
accggctgca ccgaggcggg ccgcgacgac cgcagcagcc cggcagtccg ggcggccggc   180
ggaggcggcg gcggaggagg cggaggcggc ggcggaggag gcggaggagg tgtaggagga   240
ggtggagcag gcggaggagc tggaggaggg cgctctcccg tccgggagct ggacatgggc   300
gccgccgaga gaagcaggga gcccggcagc ccgcggctga cggaggtgtc cccggagctg   360
aaagatcgca aagacgatgc gaaagggatg gaggacgaag gccagaccaa aatcaagcag   420
aggcgaagtc ggaccaattt caccctggaa caactcaatg agctggagag gctttttgac   480
gagacccact atcccgacgc cttcatgcga gaggaactga gccagcgact gggcctgtcg   540
gaggcccgag tgcaggtttg gtttcaaaat cgaagagcta aatgtagaaa acaagaaaat   600
caactccata aaggtgttct cataggggcc gccagccagt ttgaagcttg tagagtcgca   660
ccttatgtca acgtaggtgc tttaaggatg ccatttcagc aggatagtca ttgcaacgtg   720
acgcccttgc cctttcaggt tcaggcgcag ctgcagctgg acagcgctgt ggcgcacgcg   780
caccaccacc tgcatccgca cctggccgcg cacgcgccct acatgatgtt cccagcaccg   840
cccttcggac tgccgctcgc cacgctggcc gcggattcgg cttccgccgc ctcggtagtg   900
gcggccgcag cagccgccaa gaccaccagc aaggactcca gcatcgccga tctcagactg   960
aaagccaaaa agcacgccgc agccctgggt ctg                                993

<210> 7
<211> 190
<212> PRT
<213> Human

<400> 7
Met Glu Asp Glu Gly Gln Thr Lys Ile Lys Gln Arg Arg Ser Arg Thr
                5                   10                  15
Asn Phe Thr Leu Glu Gln Leu Asn Glu Leu Glu Arg Leu Phe Asp Glu
            20                  25                  30
Thr His Tyr Pro Asp Ala Phe Met Arg Glu Glu Leu Ser Gln Arg Leu
        35                  40                  45
Gly Leu Ser Glu Ala Arg Val Gln Val Trp Phe Gln Asn Arg Arg Ala
    50                  55                  60
Lys Cys Arg Lys Gln Glu Asn Gln Leu His Lys Gly Val Leu Ile Gly
65                  70                  75                  80
Ala Ala Ser Gln Phe Glu Ala Cys Arg Val Ala Pro Tyr Val Asn Val
                85                  90                  95
Gly Ala Leu Arg Met Pro Phe Gln Gln Val Gln Ala Gln Leu Gln Leu
            100                 105                 110
Asp Ser Ala Val Ala His Ala His His His Leu His Pro His Leu Ala
        115                 120                 125
Ala His Ala Pro Tyr Met Met Phe Pro Ala Pro Pro Phe Gly Leu Pro
    130                 135                 140
Leu Ala Thr Leu Ala Ala Asp Ser Ala Ser Ala Ala Ser Val Val Ala
145                 150                 155                 160
Ala Ala Ala Ala Ala Lys Thr Thr Ser Lys Asp Ser Ser Ile Ala Asp
                165                 170                 175
Leu Arg Leu Lys Ala Lys Lys His Ala Ala Ala Leu Gly Leu
            180                 185                 190

<210> 8
<211> 570
<212> DNA
<213> Human

<400> 8
atggaggacg aaggccagac caaaatcaag cagaggcgaa gtcggaccaa tttcaccctg    60
gaacaactca atgagctgga gaggcttttt gacgagaccc actatcccga cgccttcatg    120
cgagaggaac tgagccagcg actgggcctg tcggaggccc gagtgcaggt ttggtttcaa    180
aatcgaagag ctaaatgtag aaaacaagaa aatcaactcc ataaaggtgt tctcataggg    240
gccgccagcc agtttgaagc ttgtagagtc gcaccttatg tcaacgtagg tgctttaagg    300
atgccatttc agcaggttca ggcgcagctg cagctggaca gcgctgtggc gcacgcgcac    360
caccacctgc atccgcacct ggccgcgcac gcgccctaca tgatgttccc agcaccgccc    420
ttcggactgc cgctcgccac gctggccgcg gattcggctt ccgccgcctc ggtagtggcg    480
gccgcagcag ccgccaagac caccagcaag gactccagca tcgccgatct cagactgaaa    540
gccaaaaagc acgccgcagc cctgggtctg                                    570

<210> 9
<211> 343
<212> PRT
<213> Human

<400> 9

<210> 10
<211> 1029
<212> DNA
<213> Human

<220>
<221> unsure
<222> (126)..(126)
<223> n stands any base

<400> 10
atgtaccgac cgcgagcccg ggcggctccc gagggcaggg tccggggctg cgcggtgccc    60
ggcaccgtgc tcctgctgct cgcctacctg gcttacctgg cgctgggcac cggcgtgttc   120

tggacnctgg agggccgcgc ggcgcaggac tccagccgca gcttccagcg cgacaagtgg 180
gagctgttgc agaacttcac gtgtctggac cgcccggcgc tggactcgct gatccgggat 240
gtcgtccaag catacaaaaa cggagccagc ctcctcagca acaccaccag catggggcgc 300
tgggagctcg tgggctcctt cttcttttct gtgtccacca tcaccaccat tggctatggc 360
aacctgagcc ccaacacgat ggctgcccgc ctcttctgca tcttctttgc ccttgtgggg 420
atcccactca acctcgtggt gctcaaccga ctggggcatc tcatgcagca gggagtaaac 480
cactgggcca gcaggctggg gggcacctgg caggatcctg acaaggcgcg gtggctggcg 540
ggctctggcg ccctcctctc gggcctcctg ctcttcctgc tgctgccacc gctgctcttc 600
tcccacatgg agggctggag ctacacagag ggcttctact tcgccttcat caccctcagc 660
accgtgggct tcggcgacta cgtgattgga atgaacccct cccagaggta cccactgtgg 720
tacaagaaca tggtgtccct gtggatcctc tttgggatgg catggctggc cttgatctca 780
aactcatcct ctcccagctg gagacgccag ggagggtatg ttcctgctgc caccacagct 840
ctaaggaaga cttcaagtcc caaagctgga gacagggacc tgaccgggag ccagagtccc 900
actccccaca gcaaggatgc tatccagagg gacccatggg aatcatacag catctggaac 960
cttctgctca cgctgcaggc tgtggcaagg acagctagtt atactccatt ctttggtcgt 1020
cgtcctcgg 1029

<210> 11
<211> 983
<212> PRT
<213> Human

<400> 11
Met Asp Cys Gln Leu Ser Ile Leu Leu Leu Leu Ser Cys Ser Val Leu
                5                   10                  15
Asp Ser Phe Gly Glu Leu Ile Pro Gln Pro Ser Asn Glu Val Asn Leu
            20                  25                  30
Leu Asp Ser Lys Thr Ile Gln Gly Glu Leu Gly Trp Ile Ser Tyr Pro
        35                  40                  45
Ser His Gly Trp Glu Glu Ile Ser Gly Val Asp Glu His Tyr Thr Pro
    50                  55                  60
Ile Arg Thr Tyr Gln Val Cys Asn Val Met Asp His Ser Gln Asn Asn
65                  70                  75                  80
Trp Leu Arg Thr Asn Trp Val Pro Arg Asn Ser Ala Gln Lys Ile Tyr
                85                  90                  95
Val Glu Leu Lys Phe Thr Leu Arg Asp Cys Asn Ser Ile Pro Leu Val
            100                 105                 110
Leu Gly Thr Cys Lys Glu Thr Phe Asn Leu Tyr Tyr Met Glu Ser Asp
        115                 120                 125
Asp Asp His Gly Val Lys Phe Arg Glu His Gln Phe Thr Lys Ile Asp
    130                 135                 140
Thr Ile Ala Ala Asp Glu Ser Phe Thr Gln Met Asp Leu Gly Asp Arg
145                 150                 155                 160
Ile Leu Lys Leu Asn Thr Glu Ile Arg Glu Val Gly Pro Val Asn Lys
                165                 170                 175
Lys Gly Phe Tyr Leu Ala Phe Gln Asp Val Gly Ala Cys Val Ala Leu
            180                 185                 190
Val Ser Val Arg Val Tyr Phe Lys Lys Cys Pro Phe Thr Val Lys Asn
        195                 200                 205
Leu Ala Met Phe Pro Asp Thr Val Pro Met Asp Ser Gln Ser Leu Val
    210                 215                 220
Glu Val Arg Gly Ser Cys Val Asn Asn Ser Lys Glu Glu Asp Pro Pro
225                 230                 235                 240
Arg Met Tyr Cys Ser Thr Glu Gly Glu Trp Leu Val Pro Ile Gly Lys
                245                 250                 255
Cys Ser Cys Asn Ala Gly Tyr Glu Glu Arg Gly Phe Met Cys Gln Ala

            260                 265                 270
Cys Arg Pro Gly Phe Tyr Lys Ala Leu Asp Gly Asn Met Lys Cys Ala
        275                 280                 285
Lys Cys Pro Pro His Ser Ser Thr Gln Glu Asp Gly Ser Met Asn Cys
    290                 295                 300
Arg Cys Glu Asn Asn Tyr Phe Arg Ala Asp Lys Asp Pro Pro Ser Met
305                 310                 315                 320
Ala Cys Thr Arg Pro Pro Ser Ser Pro Arg Asn Val Ile Ser Asn Ile
                325                 330                 335
Asn Glu Thr Ser Val Ile Leu Asp Trp Ser Trp Pro Leu Asp Thr Gly
            340                 345                 350
Gly Arg Lys Asp Val Thr Phe Asn Ile Ile Cys Lys Lys Cys Gly Trp
        355                 360                 365
Asn Ile Lys Gln Cys Glu Pro Cys Ser Pro Asn Val Arg Phe Leu Pro
    370                 375                 380
Arg Gln Phe Gly Leu Thr Asn Thr Thr Val Thr Val Thr Asp Leu Leu
385                 390                 395                 400
Ala His Thr Asn Tyr Thr Phe Glu Ile Asp Ala Val Asn Gly Val Ser
                405                 410                 415
Glu Leu Ser Ser Pro Pro Arg Gln Phe Ala Ala Val Ser Ile Thr Thr
            420                 425                 430
Asn Gln Ala Ala Pro Ser Pro Val Leu Thr Ile Lys Lys Asp Arg Thr
        435                 440                 445
Ser Arg Asn Ser Ile Ser Leu Ser Trp Gln Glu Pro Glu His Pro Asn
    450                 455                 460
Gly Ile Ile Leu Asp Tyr Glu Val Lys Tyr Tyr Glu Lys Gln Glu Gln
465                 470                 475                 480
Glu Thr Ser Tyr Thr Ile Leu Arg Ala Arg Gly Thr Asn Val Thr Ile
                485                 490                 495
Ser Ser Leu Lys Pro Asp Thr Ile Tyr Val Phe Gln Ile Arg Ala Arg
            500                 505                 510
Thr Ala Ala Gly Tyr Gly Thr Asn Ser Arg Lys Phe Glu Phe Glu Thr
        515                 520                 525
Ser Pro Asp Ser Phe Ser Ile Ser Gly Glu Ser Ser Gln Val Val Met
    530                 535                 540
Ile Ala Ile Ser Ala Ala Val Ala Ile Ile Leu Leu Thr Val Val Ile
545                 550                 555                 560
Tyr Val Leu Ile Gly Arg Phe Cys Gly Tyr Lys Ser Lys His Gly Ala
                565                 570                 575
Asp Glu Lys Arg Leu His Phe Gly Asn Gly His Leu Lys Leu Pro Gly
            580                 585                 590
Leu Arg Thr Tyr Val Asp Pro His Thr Tyr Glu Asp Pro Thr Gln Ala
        595                 600                 605
Val His Glu Phe Ala Lys Glu Leu Asp Ala Thr Asn Ile Ser Ile Asp
    610                 615                 620
Lys Val Val Gly Ala Gly Glu Phe Gly Glu Val Cys Ser Gly Arg Leu
625                 630                 635                 640
Lys Leu Pro Ser Lys Lys Glu Ile Ser Val Ala Ile Lys Thr Leu Lys
                645                 650                 655
Val Gly Tyr Thr Glu Lys Gln Arg Arg Asp Phe Leu Gly Glu Ala Ser
            660                 665                 670
Ile Met Gly Gln Phe Asp His Pro Asn Ile Ile Arg Leu Glu Gly Val
        675                 680                 685
Val Thr Lys Ser Lys Pro Val Met Ile Val Thr Glu Tyr Met Glu Asn
    690                 695                 700
Gly Ser Leu Asp Ser Phe Leu Arg Lys His Asp Ala Gln Phe Thr Val

705                 710                 715                 720
Ile Gln Leu Val Gly Met Leu Arg Gly Ile Ala Ser Gly Met Lys Tyr
                725                 730                 735
Leu Ser Asp Met Gly Tyr Val His Arg Asp Leu Ala Ala Arg Asn Ile
            740                 745                 750
Leu Ile Asn Ser Asn Leu Val Cys Lys Val Ser Asp Phe Gly Leu Ser
        755                 760                 765
Arg Val Leu Glu Asp Asp Pro Glu Ala Ala Tyr Thr Thr Arg Gly Gly
    770                 775                 780
Lys Ile Pro Ile Arg Trp Thr Ser Pro Glu Ala Ile Ala Tyr Arg Lys
785                 790                 795                 800
Phe Thr Ser Ala Ser Asp Val Trp Ser Tyr Gly Ile Val Leu Trp Glu
                805                 810                 815
Val Met Ser Tyr Gly Glu Arg Pro Tyr Trp Glu Met Ser Asn Gln Asp
            820                 825                 830
Val Ile Lys Ala Val Asp Glu Gly Tyr Arg Leu Pro Pro Pro Met Asp
        835                 840                 845
Cys Pro Ala Ala Leu Tyr Gln Leu Met Leu Asp Cys Trp Gln Lys Asp
    850                 855                 860
Arg Asn Asn Arg Pro Lys Phe Glu Gln Ile Val Ser Ile Leu Asp Lys
865                 870                 875                 880
Leu Ile Arg Asn Pro Gly Ser Leu Lys Ile Ile Thr Ser Ala Ala Ala
                885                 890                 895
Arg Pro Ser Asn Leu Leu Leu Asp Gln Ser Asn Val Asp Ile Thr Thr
            900                 905                 910
Phe Arg Thr Thr Gly Asp Trp Leu Asn Gly Val Trp Thr Ala His Cys
        915                 920                 925
Lys Glu Ile Phe Thr Gly Val Glu Tyr Ser Ser Cys Asp Thr Ile Ala
    930                 935                 940
Lys Ile Ser Thr Asp Asp Met Lys Lys Val Gly Val Thr Val Val Gly
945                 950                 955                 960
Pro Gln Lys Lys Ile Ile Ser Ser Ile Lys Ala Leu Glu Thr Gln Ser
                965                 970                 975
Lys Asn Gly Pro Val Pro Val
            980

<210> 12
<211> 2949
<212> DNA
<213> Human

<400> 12
atggattgtc agctctccat cctcctcctt ctcagctgct ctgttctcga cagcttcggg    60
gaactgattc cgcagccttc caatgaagtc aatctactgg attcaaaaac aattcaaggg   120
gagctgggct ggatctctta tccatcacat gggtgggaag agatcagtgg tgtggatgaa   180
cattacacac ccatcaggac ttaccaggtg tgcaatgtca tggaccacag tcaaaacaat   240
tggctgagaa caaactgggt ccccaggaac tcagctcaga agatttatgt ggagctcaag   300
ttcactctac gagactgcaa tagcattcca ttggttttag gaacttgcaa ggagacattc   360
aacctgtact acatggagtc tgatgatgat catggggtga aatttcgaga gcatcagttt   420
acaaagattg acaccattgc agctgatgaa agtttcactc aaatggatct tggggaccgt   480
attctgaagc tcaacactga gattagagaa gtaggtcctg tcaacaagaa gggattttat   540
ttggcatttc aagatgttgg tgcttgtgtt gccttggtgt ctgtgagagt atacttcaaa   600
aagtgcccat ttacagtgaa gaatctggct atgtttccag acacggtacc catggactcc   660
cagtccctgg tggaggttag agggtcttgt gtcaacaatt ctaaggagga agatcctcca   720
aggatgtact gcagtacaga aggcgaatgg cttgtaccca ttggcaagtg ttcctgcaat   780
gctggctatg aagaaagagg ttttatgtgc caagcttgtc gaccaggttt ctacaaggca   840

ttggatggta atatgaagtg tgctaagtgc ccgcctcaca gttctactca ggaagatggt 900
tcaatgaact gcaggtgtga gaataattac ttccgggcag acaaagaccc tccatccatg 960
gcttgtaccc gacctccatc ttcaccaaga aatgttatct ctaatataaa cgagacctca 1020
gttatcctgg actggagttg gcccctggac acaggaggcc ggaaagatgt taccttcaac 1080
atcatatgta aaaaatgtgg gtggaatata aaacagtgtg agccatgcag cccaaatgtc 1140
cgcttcctcc ctcgacagtt tggactcacc aacaccacgg tgacagtgac agaccttctg 1200
gcacatacta actacacctt tgagattgat gccgttaatg gggtgtcaga gctgagctcc 1260
ccaccaagac agtttgctgc ggtcagcatc acaactaatc aggctgctcc atcacctgtc 1320
ctgacgatta agaaagatcg gacctccaga aatagcatct ctttgtcctg gcaagaacct 1380
gaacatccta atgggatcat attggactac gaggtcaaat actatgaaaa gcaggaacaa 1440
gaaacaagtt ataccattct gagggcaaga ggcacaaatg ttaccatcag tagcctcaag 1500
cctgacacta tatacgtatt ccaaatccga gcccgaacag ccgctggata tgggacgaac 1560
agccgcaagt ttgagtttga aactagtcca gactctttct ccatctctgg tgaaagtagc 1620
caagtggtca tgatcgccat ttcagcggca gtagcaatta ttctcctcac tgttgtcatc 1680
tatgttttga ttgggaggtt ctgtggctat aagtcaaaac atggggcaga tgaaaaaaga 1740
cttcattttg gcaatgggca tttaaaactt ccaggtctca ggacttatgt tgacccacat 1800
acatatgaag accctaccca agctgttcat gagtttgcca aggaattgga tgccaccaac 1860
atatccattg ataaagttgt tggagcaggt gaatttggag aggtgtgcag tggtcgctta 1920
aaacttcctt caaaaaaaga gatttcagtg gccattaaga ccctgaaagt tggctacaca 1980
gaaaagcaga ggagagactt cctgggagaa gcaagcatta tgggacagtt tgaccacccc 2040
aatatcattc gactggaagg agttgttacc aaaagtaagc cagttatgat tgtcacagaa 2100
tacatggaga atggttcctt ggatagtttc ctacgtaaac acgatgccca gtttactgtc 2160
attcagctag tggggatgct tcgagggata gcatctggca tgaagtacct gtcagacatg 2220
ggctatgttc accgagacct cgctgctcgg aacatcttga tcaacagtaa cttggtgtgt 2280
aaggtttctg atttcggact ttcgcgtgtc ctggaggatg acccagaagc tgcttataca 2340
acaagaggag ggaagatccc aatcaggtgg acatcaccag aagctatagc ctaccgcaag 2400
ttcacgtcag ccagcgatgt atggagttat gggattgttc tctgggaggt gatgtcttat 2460
ggagagagac catactggga gatgtccaat caggatgtaa ttaaagctgt agatgagggc 2520
tatcgactgc caccccccat ggactgccca gctgccttgt atcagctgat gctggactgc 2580
tggcagaaag acaggaacaa cagacccaag tttgagcaga ttgttagtat tctggacaag 2640
cttatccgga atcccggcag cctgaagatc atcaccagtg cagccgcaag gccatcaaac 2700
cttcttctgg accaaagcaa tgtggatatc actaccttcc gcacaacagg tgactggctt 2760
aatggtgtct ggacagcaca ctgcaaggaa atcttcacgg gtgtggagta cagttcttgt 2820
gacacaatag ccaagatttc cacagatgac atgaaaaagg ttggtgtcac cgtggttggg 2880
ccacagaaga agatcatcag tagcattaaa gctctagaaa cgcaatcaaa gaatggccca 2940
gttcccgtg 2949

<210> 13
<211> 457
<212> PRT
<213> Human

<400> 13
Met Ile Leu Leu Thr Phe Ser Thr Gly Arg Arg Leu Asp Phe Val His
                5                   10                  15
His Ser Gly Val Phe Phe Leu Gln Thr Leu Leu Trp Ile Leu Cys Ala
            20                  25                  30
Thr Val Cys Gly Thr Glu Gln Tyr Phe Asn Val Glu Val Trp Leu Gln
        35                  40                  45
Lys Tyr Gly Tyr Leu Pro Pro Thr Asp Pro Arg Met Ser Val Leu Arg
    50                  55                  60
Ser Ala Glu Thr Met Gln Ser Ala Leu Ala Ala Met Gln Gln Phe Tyr
65                  70                  75                  80
Gly Ile Asn Met Thr Gly Lys Val Asp Arg Asn Thr Ile Asp Trp Met
                85                  90                  95
Lys Lys Pro Arg Cys Gly Val Pro Asp Gln Thr Arg Gly Ser Ser Lys

                100                 105                 110
Phe His Ile Arg Arg Lys Arg Tyr Ala Leu Thr Gly Gln Lys Trp Gln
        115                 120                 125
His Lys His Ile Thr Tyr Ser Ile Lys Asn Val Thr Pro Lys Val Gly
    130                 135                 140
Asp Pro Glu Thr Arg Lys Ala Ile Arg Arg Ala Phe Asp Val Trp Gln
145                 150                 155                 160
Asn Val Thr Pro Leu Thr Phe Glu Glu Val Pro Tyr Ser Glu Leu Glu
                165                 170                 175
Asn Gly Lys Arg Asp Val Asp Ile Thr Ile Ile Phe Ala Ser Gly Phe
            180                 185                 190
His Gly Asp Ser Ser Pro Phe Asp Gly Glu Gly Gly Phe Leu Ala His
        195                 200                 205
Ala Tyr Phe Pro Gly Pro Gly Ile Gly Gly Asp Thr His Phe Asp Ser
    210                 215                 220
Asp Glu Pro Trp Thr Leu Gly Asn Pro Asn His Asp Gly Asn Asp Leu
225                 230                 235                 240
Phe Leu Val Ala Val His Glu Leu Gly His Ala Leu Gly Leu Glu His
                245                 250                 255
Ser Asn Asp Pro Thr Ala Ile Met Ala Pro Phe Tyr Gln Tyr Met Glu
            260                 265                 270
Thr Asp Asn Phe Lys Leu Pro Asn Asp Asp Leu Gln Gly Ile Gln Lys
        275                 280                 285
Ile Tyr Gly Pro Pro Asp Lys Ile Pro Pro Pro Thr Arg Pro Leu Pro
    290                 295                 300
Thr Val Pro Pro His Arg Ser Ile Pro Pro Ala Asp Pro Arg Lys Asn
305                 310                 315                 320
Asp Arg Pro Lys Pro Pro Arg Pro Pro Thr Gly Arg Pro Ser Tyr Pro
                325                 330                 335
Gly Ala Lys Pro Asn Ile Cys Asp Gly Asn Phe Asn Thr Leu Ala Ile
            340                 345                 350
Leu Arg Arg Glu Met Phe Val Phe Lys Asp Gln Trp Phe Trp Arg Val
        355                 360                 365
Arg Asn Asn Arg Val Met Asp Gly Tyr Pro Met Gln Ile Thr Tyr Phe
    370                 375                 380
Trp Arg Gly Leu Pro Pro Ser Ile Asp Ala Val Tyr Glu Asn Ser Asp
385                 390                 395                 400
Gly Asn Phe Val Phe Phe Lys Val Lys Gly Asp Thr Leu Ser Val Ile
                405                 410                 415
Gln Asp Gly Trp Leu Tyr Lys Tyr His Trp Lys Trp Ile Leu Glu Gln
            420                 425                 430
Arg Gln Ser Val Pro Val Leu Ser Arg Gln Thr Glu Lys His Lys Thr
        435                 440                 445
Tyr Glu Glu Leu Ser Ser Ile Thr Tyr
    450                 455

<210> 14
<211> 1371
<212> DNA
<213> Human

<400> 14
atgatcttac tcacattcag cactggaaga cggttggatt tcgtgcatca ttcgggggtg    60
tttttcttgc aaaccttgct ttggatttta tgtgctacag tctgcggaac ggagcagtat   120
ttcaatgtgg aggtttggtt acaaaagtac ggctaccttc caccgactga ccccagaatg   180
tcagtgctgc gctctgcaga gaccatgcag tctgccctag ctgccatgca gcagttctat   240

ggcattaaca tgacaggaaa agtggacaga aacacaattg actggatgaa gaagccccga 300
tgcggtgtac ctgaccagac aagaggtagc tccaaatttc atattcgtcg aaagcgatat 360
gcattgacag gacagaaatg gcagcacaag cacatcactt acagtataaa gaacgtaact 420
ccaaaagtag gagaccctga gactcgtaaa gctattcgcc gtgcctttga tgtgtggcag 480
aatgtaactc ctctgacatt tgaagaagtt ccctacagtg aattagaaaa tggcaaacgt 540
gatgtggata taaccattat ttttgcatct ggtttccatg gggacagctc tccctttgat 600
ggagagggag gatttttggc acatgcctac ttccctggac caggaattgg aggagatacc 660
cattttgact cagatgagcc atggacacta ggaaatccta atcatgatgg aaatgactta 720
tttcttgtag cagtccatga actgggacat gctctgggat tggagcattc caatgacccc 780
actgccatca tggctccatt ttaccagtac atggaaacag acaacttcaa actacctaat 840
gatgatttac agggcatcca gaagatatat ggtccacctg acaagattcc tccacctaca 900
agacctctac cgacagtgcc cccacaccgc tctattcctc cggctgaccc aaggaaaaat 960
gacaggccaa aacctcctcg gcctccaacc ggcagaccct cctatcccgg agccaaaccc 1020
aacatctgtg atgggaactt taacactcta gctattcttc gtcgtgagat gtttgttttc 1080
aaggaccagt ggttttggcg agtgagaaac aacagggtga tggatggata cccaatgcaa 1140
attacttact tctggcgggg cttgcctcct agtatcgatg cagtttatga aaatagcgac 1200
gggaattttg tgttctttaa agtgaaggga gacactctat ctgtaatcca agatggttgg 1260
ctctacaaat accattggaa atggattcta gaacaaaggc agtcagtgcc tgtgctctca 1320
agacaaactg aaaagcacaa gacctatgaa gaattatctt ccatcacata c 1371

<210> 15
<211> 20
<212> DNA
<213> Artificial Sequence

<220>
<223> Primer

<400> 15
gaccctggag cactcatgtt 20

<210> 16
<211> 20
<212> DNA
<213> Artificial Sequence

<220>
<223> Primer

<400> 16
gtctggcgtt tttggatgtt 20

<210> 17
<211> 18
<212> DNA
<213> Artificial Sequence

<220>
<223> Primer

<400> 17
gagatggggc tgccccag 18

<210> 18
<211> 25
<212> DNA

<213> Artificial Sequence

<220>
<223> Primer

<400> 18
gccttgagaa gccctcccgt tggtc                                    25

<210> 19
<211> 22
<212> DNA
<213> Artificial Sequence

<220>
<223> Primer

<400> 19
ggagagtact cactttacca tc                                       22

<210> 20
<211> 21
<212> DNA
<213> Artificial Sequence

<220>
<223> Primer

<400> 20
gaatcaagtc acagccgtgt g                                        21

<210> 21
<211> 21
<212> DNA
<213> Artificial Sequence

<220>
<223> Primer

<400> 21
ctgactcagg aggcagagaa g                                        21

<210> 22
<211> 20
<212> DNA
<213> Artificial Sequence

<220>
<223> Primer

<400> 22
gggctctgtt gaaatatgga                                          20

<210> 23
<211> 21
<212> DNA
<213> Artificial Sequence

<220>
<223> Primer

<400> 23
ccagtacttt ataaccaaag c                                                21

<210> 24
<211> 20
<212> DNA
<213> Artificial Sequence

<220>
<223> Primer

<400> 24
aagatgggga gaggcaaact                                                  20